# EUROPEAN PATENT SPECIFICATION

(11) **EP 3 825 695 B1**
(45) Date of publication and mention of the grant of the patent: **06.12.2023**
(21) Application number: 20200807.4
(22) Date of filing: 08.10.2020
(51) Int. Cl.: G01N 33/68

(54) **BIOMARKERS FOR RESPONDERS TO AIT**
BIOMARKER FÜR RESPONDER AUF AIT
BIOMARQUEURS POUR LES RÉPONDANTS À UN AIT

(30) Priority: 25.11.2019 EP 19211257
(43) Date of publication of application: 26.05.2021
(73) Proprietor: Allergopharma GmbH & Co. KG, 21465 Reinbek (DE)
(72) Inventor: NANDY, Andreas, 22175 Hamburg (DE); KAHLERT, Helga, 22041 Hamburg (DE); BEREK, Nadine, 22085 Hamburg (DE); MERTENS-BEER, Melanie, 21465 Wentorf (DE); WILLERS, Christoph Paul Joseph, 22393 Hamburg (DE); SOKOLOWSKA, Milena Grazyna, 7270 Davos (CH); AKDIS, Ali Cezmi, 7265 Davos (CH)
(74) Representative: Becker, Eberhard

(56) References cited:
- WO-A1-2016/066770
- WO-A1-2017/121883
- WO-A2-2012/137180
- BRANCO-FERREIRA ET AL: "Can Nasal ECP Help to Predict Clinical Outcome of Specific Immunotherapy in Mite-allergic Rhinitis Patients", JOURNAL OF ALLERGY AND CLINICAL IMMUNOLOGY, ELSEVIER, AMSTERDAM, NL, vol. 117, no. 2, 1 February 2006 (2006-02-01), page S160, XP005360024, ISSN: 0091-6749, DOI: 10.1016/J.JACI.2005.12.637
- VERSTRAELEN S ET AL: "Cell types involved in allergic asthma and their use in in vitro models to assess respiratory sensitization", TOXICOLOGY IN VITRO, ELSEVIER SCIENCE, GB, vol. 22, no. 6, 1 September 2008 (2008-09-01), pages 1419-1431, XP023904599, ISSN: 0887-2333, DOI: 10.1016/J.TIV.2008.05.008 [retrieved on 2008-07-07]
- SAVOLAINEN J. ET AL: "Allergen-induced in vitro expression of IL-18, SLAM and GATA-3 mRNA in PBMC during sublingual immunotherapy", ALLERGY, vol. 62, no. 8, 1 August 2007 (2007-08-01) , pages 949-953, XP055809954, United Kingdom ISSN: 0105-4538, DOI: 10.1111/j.1398-9995.2007.01426.x
- HUOMAN JOHANNA ET AL: "Sublingual immunotherapy alters salivary IgA and systemic immune mediators in timothy allergic children", PEDIATRIC ALLERGY AND IMMUNOLOGY, 14 April 2019 (2019-04-14), XP055809989, GB ISSN: 0905-6157, DOI: 10.1111/pai.13047 Retrieved from the Internet: URL:https://onlinelibrary.wiley.com/doi/fu ll-xml/10.1111/pai.13047>
- THOMPSON ET AL: "Integrin @b3 genotype influences asthma and allergy phenotypes in the first 6 years of life", JOURNAL OF ALLERGY AND CLINICAL IMMUNOLOGY, ELSEVIER, AMSTERDAM, NL, vol. 119, no. 6, 6 June 2007 (2007-06-06), pages 1423-1429, XP022096897, ISSN: 0091-6749, DOI: 10.1016/J.JACI.2007.03.029
- MAKINO Y ET AL: "Apolipoprotein A-IV is a candidate target molecule for the treatment of seasonal allergic rhinitis", JOURNAL OF ALLERGY AND CLINICAL IMMUNOLOGY, ELSEVIER, AMSTERDAM, NL, vol. 126, no. 6, 1 December 2010 (2010-12-01), pages 1163-1169.e5, XP027541435, ISSN: 0091-6749 [retrieved on 2010-12-01]
- SALMIVESI SUSANNA ET AL: "Changes in biomarkers during a six-month oral immunotherapy intervention for cow's milk allergy", ACTA PAEDIATRICA, vol. 105, no. 11, 10 October 2016 (2016-10-10), pages 1349-1354, XP055810664, GB ISSN: 0803-5253, DOI: 10.1111/apa.13550 Retrieved from the Internet: URL:https://api.wiley.com/onlinelibrary/td m/v1/articles/10.1111%2Fapa.13550>
- Lichtenstein P S ET AL: "activity in nasal secretions. formation and contribution to TAME-esterase induced allergic rhinitis: role in kinin Plasma kallikrein during experimentally PLASMA KALLIKREIN DURING EXPERIMENTALLY INDUCED ALLERGIC ESTERASE ACTIVITY IN NASAL SECRETIONS' RHINITIS: ROLE IN KININ F O ~ T ~ O N AND CONT~BU", , 1 January 1986 (1986-01-01), pages 977-982, XP055810673, Retrieved from the Internet: URL:https://www.jimmunol.org/content/jimmu nol/137/3/977.full.pdf [retrieved on 2021-06-04]
- HOWARTH P H ET AL: "Objective monitoring of nasal airway inflammation in rhinitis", JOURNAL OF ALLERGY AND CLINICAL IMMUNOLOGY, ELSEVIER, AMSTERDAM, NL, vol. 115, no. 3, 1 March 2005 (2005-03-01) , pages S414-S441, XP004775722, ISSN: 0091-6749, DOI: 10.1016/J.JACI.2004.12.1134
- ORIHARA KANAMI ET AL: "Plasma Cytokine/Chemokine Profiles in Non-IgE-Mediated Gastrointestinal Food Allergy", JOURNAL OF ALLERGY AND CLINICAL IMMUNOLOGY, vol. 137, no. 2, February 2016 (2016-02), XP029414985, ISSN: 0091-6749, DOI: 10.1016/J.JACI.2015.12.973
- LISA GIOVANNINI-CHAMI ET AL: "Distinct epithelial gene expression phenotypes in childhood respiratory allergy", EUROPEAN RESPIRATORY JOURNAL, vol. 39, no. 5, 17 October 2011 (2011-10-17), pages 1197-1205, XP055400761, GB ISSN: 0903-1936, DOI: 10.1183/09031936.00070511
- LEVCHENKO A S ET AL: "Genetic Aspects of Chronic Rhinosinusitis", RUSSIAN JOURNAL OF GENETICS, MOSCOW, RU, vol. 54, no. 8, 23 August 2018 (2018-08-23), pages 910-918, XP036576605, ISSN: 1022-7954, DOI: 10.1134/S1022795418080082 [retrieved on 2018-08-23]

## Description

The invention relates to the use of marker proteins or biomarkers in the identification of patients with grass and/or birch pollen allergy which are responders to allergen immunotherapy (AIT).

### Background of the invention

Type 1 allergies have worldwide importance. Up to 20% of the population in industrialized countries suffer from complaints such as allergic rhinitis, conjunctivitis or bronchial asthma.

These allergies are caused by sources of various origin, such as trees and grasses (pollen), fungi (spores), mites (excrement), cats or dogs. The allergen sources are released directly into the air (pollen, spores) or can reach the air bonded to diesel soot particles (pollen) or house dust (mite excrement, skin particles, hair). Since the allergy-triggering substances are located in the air, the term 'aeroallergens' is also used.

The type 1 allergy-triggering substances are proteins, glycoproteins or polypeptides. After uptake via mucous membranes, these allergens react with the IgE molecules bound to the surface of mast cells in sensitized persons. If these IgE molecules are crosslinked with one another by an allergen, this results in the secretion of mediators (for example histamine, prostaglandins) and cytokines by the effector cell and thus in the corresponding allergic symptoms.

Up to 40% of type 1 allergy sufferers exhibit specific IgE reactivity with pollen extracts of true grasses (Burney et al., 1997, J. Allergy Clin. Immunol. 99:314-322; D'Amato et al., 1998, Allergy 53: 567-578; Freidhoff et al., 1986, J. Allergy Clin Immunoloav. 78, 1190-2002). The familv of the true grasses (Poaceae) encompasses more than 10000 species, many more than 20 of which are hitherto known as triggers of allergic symptoms (Andersson & Lidholm, 2003, Int. Arch. Allergy Immunol. 130:87-107; Esch, 2008, Allergens and Allergen Immunotherapy, Clinical Allergy and Immunology Series, 107-126).

Most of the allergy-triggering true grasses belong to the Pooideae sub-family. Besides the grass species occurring as wild forms, such as, for example, Holcus lanatus (velvet grass), Phalaris aquatica (canary grass), Anthoxanthum odoratum (sweet vernal grass), Dactylis glomerata (orchard grass), Festuca pratensis (meadow fescue), Poa pratensis (Kentucky blue grass) or Lolium perenne (rye grass), cultivated cereals, such as Triticum aestivum (wheat), Secale cereale (rye) and Hordeum vulgare (barley), are also known members of this sub-family.

Allergic immunotherapy (AIT), specific immunotherapy (SIT) or hyposensitization is regarded as an effective approach to the therapeutic treatment of allergies (Fiebig 1995 Allergo J. 4 (6):336-339, Bousquet et al., 1998, J. Allergy Clin. Immunol. 102 (4): 558-562; Cox et al., 2007, J. Allergy Clin. Immunol. 120: S25-85; James & Durham, 2008, Clin. Exp. Allergy 38: 1074-1088).

The classical therapy form of injection therapy (SCIT), in which natural allergen extracts are injected subcutaneously into the patient in increasing doses, has been used successfully for about 100 years. In this therapy, the immune system of the allergy sufferer is repeatedly confronted with allergens, causing reprogramming of the immune system to be achieved together with tolerance of the allergens. After uptake of the antigens from the allergen preparations by antigen-presenting cells, peptides are presented to the antigens on the cell surface. Some particular peptides which contain so-called T-cell epitopes are recognized by antigen-specific T-cells. This binding leads inter alia, in the development of various types of T-cells having a regulatory function. In the course of AIT, the regulatory T-cell response results in tolerance of the allergen, the downregulation of TH2 cytokines, the restoration of the TH1/TH2 equilibrium, the suppression of allergen-specific IgE, the induction of IgG4, IgG1 and IgA antibodies, the suppression of effector cells (mast cells, basophils and eosinophils) and the renewal of inflamed tissue (Akdis et al., 2007, J. Allergy Clin. Immunol. 119 (4):780-789; Larche et al., 2008, Nature Reviews 6:761-771). The T-cell epitopes are thus of crucial importance for the therapeutic action of allergen preparations in the case of hyposensitization.

Owing to the cross-reactivity of the major allergens of the true grasses which is present at IgE and also at T-cell level, successful therapy with an allergen extract of a single representative grass species is usually sufficient (Mailing et al., 1993, EAACI Position Paper: Immunotherapy, Allergy 48: 9 35; Cox et al., 2007, J Allergy Clin Immunol 120: 25-85).

Besides subcutaneous immunotherapy, a sublingual therapy form, in which the allergens or allergen derivatives are taken up via the oral mucous membrane, is an alternative to injection therapy (James & Durham, 2008, Clin. Exp. Allergy 38: 1074-1088).

A further possibility is treatment with expressible DNA which encodes for the relevant allergens (immunotherapeutic vaccination). Experimental evidence of the allergen-specific influencing of the immune response has been furnished in rodents by injection of allergen-encoding DNA (Hsu et al. 1996, Nature Medicine 2 (5):540-544; Weiss et al., 2006, Int. Arch. Allergy Immunol. 139: 332-345).

In all these therapy forms, there is a fundamental risk of allergic reactions or even anaphylactic shock (Kleine-Tebbe, 2006, Allergologie, 4:135-156). In order to minimize these risks, innovative preparations in the form of allergoids are employed. These are chemically modified allergen extracts which have significantly reduced IgE reactivity, but identical T-cell reactivity compared with the untreated extract (Fiebig 1995 Allergo J. 4 (6):336-339; Kahlert et al., 1999, Int. Arch. Allergy Immunol, 120: 146-157).

Therapy optimization is possible with allergens prepared by recombinant methods. Defined cocktails of high-purity allergens prepared by recombinant methods, which are optionally matched to the individual sensitization patterns of the patients, could replace extracts from natural allergen sources, since, apart from the various allergens, the latter contain a relatively large number of immunogenic, but nonallergenic accompanying proteins. Initial clinical studies with recombinant allergens have already been carried out with success (Jutel et al., 2005, J. Allergy Clin. Immunol., 116: 608-613; Valenta & Niederberger, 2007, J. Allergy Clin. Immunol. 119: 826-830).

Realistic prospects which may result in safe hyposensitization with recombinant expression products are offered specifically by mutated recombinant allergens in which IgE epitopes are modified without impairing the T-cell epitopes which are essential for the therapy (Schramm et al. 1999, J. Immunol. 162:2406-2414). These hypoallergenic proteins could be employed in relatively high doses during AIT without increasing the probability of undesired IgE-promoted side effects.

In the past, such "hypoallergenic" variants with reduced IgE binding have been published for many aeroallergens (inter alia pollen and house dust mite allergens) and food allergens. On the basis of the DNA of unmodified allergens, it has been possible to prepare and express a recombinant DNA, inter alia by fragmentation, oligomerization, deletions, point mutations or recombination of individual sections of an allergen (DNA shuffling) (Ferreira et al., 2006, Inflamm. & Allergy - Drug Targets 5: 5 14; Bhalla & Singh, 2008, Trends in Biotechnology 26:153-161; Westritschnig et al., 2007, J. Immunol. 179: 7624-7634).

Eligibility to immunotherapy is based on the determination of IgE reactivity to a specific allergen by means of skin prick or in-vitro testing. Biomarkers or marker proteins predicting the likelihood of clinical improvement during immunotherapy would significantly improve patient selection.

Although AIT is effective, the degree of remission strongly varies depending on the complex interaction between patient, allergy, symptoms and vaccines used for AIT (Bousquet J, Van Cauwenberge P, Khaltaev N, Aria Workshop G, World Health O. J Allergy Clin Immunol 2001;108(5 Suppl.): S147-S334; Bousquet J, Khaltaev N, Cruz AA, Denburg J, Fokkens WJ, Togias A et al. Allergy 2008;63 (Suppl. 86): 8-160; Bousquet J, Lockey R, Mailing HJ. J Allergy Clin Immunol 1998;102: 558-562; Shamji MH, Ljorring C, Francis JN, Calderon MA, Larche M, Kimber I et al. Allergy 2012; 67: 217- 226; Durham SR, Walker SM, Varga EM, Jacobson MR, O'Brien F, Noble W et al. N Engl J Med 1999; 341:468-475; Calderon MA, Alves B, Jacobson M, Hurwitz B, Sheikh A, Durham S. Cochrane Database Syst Rev 2007; CD001936; Radulovic S, Calderon MA, Wilson D, Durham S. Cochrane Database Syst Rev 2010; CD002893).

Clinical management of patients receiving AIT and efficacy in randomized controlled trials for drug development could be significantly enhanced if there were means to identify those who are most likely to respond, when to stop treatment, how to predict relapse and when to perform booster AIT. Furthermore, biomarkers in AIT can play a central role in personalized medicine (Galli SJ. J Allergy Clin Immunol 2016; 137:1289-1300). Although recommendations for the standardization of clinical outcomes used in AIT trials for AR have recently been defined (Pfaar O, Demoly P, Gerth van Wijk R, Bonini S, Bousquet J, Canonica GW et al. Allergy 2014;69:854-867. Burks AW, Calderon MA, Casale T, Cox L, Demoly P, Jutel M et al. J Allergy Clin Immunol 2013;131: 1288-1296. Calderon MA, Casale T, Cox L, Akdis CA, Burks AW, Nelson HS et al. Allergy 2013;68: 825-828. Calderon MA, Bernstein DI, Blaiss M, Andersen JS, Nolte H. Clin Exp Allergy 2014;44: 1228-1239), to date there is no consensus on candidate surrogate biomarkers of efficacy or biomarker combinations that would be prognostic, predictive and/or surrogate of the clinical response to AIT (Food, Drug Administration HHS. International Conference on harmonization; guidance on E15 pharmacogenomics definitions and sample coding; availability. Notice. Fed Regist 2008; 73: 19074-19076.). In the sense of personalized medicine, biomarkers can be utilized to assist patient selection, identification of responders, target intervention at those who will benefit and to exclude those who are less likely to respond to treatment, thus meeting the criteria of personalized medicine.

Additionally, they can be of major importance for the development of novel vaccines and for the optimization of existing therapeutic regimes. According to International Conference on Harmonization (ICH) E15 guidance on `Definitions for Genomic Biomarkers, Pharmacogenomics, Pharmacogenetics, Genomic Data and Sample Coding Categories', biomarkers are 'indicators of normal biologic processes, pathogenic processes and/or response to therapeutic or other interventions' (Food, Drug Administration HHS. International Conference on harmonization; guidance on E15 pharmacogenomics definitions and sample coding; availability. Notice. Fed Regist 2008; 73: 19074-19076.). Biomarkers can be applied in the context of controlled clinical trials for regulatory approval as well as in a clinical practice.

Criteria for evaluating and selecting candidate biomarkers are provided by ICH E16 guideline 'Biomarkers Related to Drug or Biotechnology Product Developments: Context, structure and format of Qualification Submissions' (Food, Drug Administration HHS. International conference on harmonization; guidance on E16 biomarkers related to drug or biotechnology product development: context, structure, and format of qualification submissions; availability. Notice. Fed Regist 2011; 76: 49773-49774.). Per candidate biomarker, an overview containing the strengths and the limitations, design of the studies supporting its utility should be reported. The 'Guideline on the Clinical Development of Products for Specific Immunotherapy for the Treatment of Allergic Diseases' by the European Medicines Agency (EMA) published in 2008 advises to include immunological changes (e.g. changes in allergen-specific IgG levels, T-cell responses and/or cytokine production) and/or modifications of the end-organ specific response (e.g. provocation tests) in pharmacokinetic and dynamic studies in order to show the effect of AIT on the immune system (CHMP EMA Guideline on the Clinical Development of Products for Specific Immunotherapy for the Treatment of Allergic Diseases. 2008. No separate publisher).

However, since 2008 several novel immunological markers in AIT have become available and may potentially be used as surrogate/predictive biomarkers for AIT. In this context, laboratory techniques should be reproducible, robust, sensitive and specific following the ICH guidelines for validation of analytical procedures 'Validation of Analytical Procedures: Methodology' (International Conference on Harmonization of Technical Requirements for registration of pharmaceuticals for human use (ICH) adopts consolidated guideline on good clinical practice in the conduct of clinical trials on medicinal products for human use. Int Dig Health Legis 1997; 48:231-234.). The European Academy of Allergy and Clinical Immunology (EAACI) Immunotherapy Interest Group (IT IG) has conducted a task force (TF) on 'Biomarkers for monitoring the clinical efficacy of allergen Immunotherapy'.

Thus, there remains a need for biomarkers and for highly effective diagnostic tests using such specific marker proteins which are able to identify responders to AIT and non-responders and healthy individuals where AIT is contraindicated.

### Summary of the invention

The invention is defined in the appended claims.

Surprisingly it was found, that the expression level of specific marker proteins in plasma or nasal fluid (the later not according to the invention) samples is predictive for a successful allergic immunotherapy in an allergic patient, i.e. for the identification of responders to AIT, or for the discrimination between allergic patients and healthy individuals.

Thus, an embodiment of the present invention is a method of identifying a patient with grass and/or birch pollen allergy responsive to treatment with allergic immunotherapy (AIT) comprising determining in-vitro in a plasma sample from said allergic patient the level of expression of the marker protein CCL8 alone or in combination with one or more marker proteins selected from the group consisting of ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 and METAP1 D.

Another embodiment of the present invention is a method of determining the clinical efficacy of a treatment with AIT using the method according to the present invention.

Another embodiment of the present invention is a method for the identification of an allergic patient wherein the treatment with AIT is contraindicated, using the method according to the present invention. Thus, another embodiment of the present invention is a method for the identification of an allergic patient nonresponsive to treatment with AIT, using the method according to the present invention and comparing the expression levels of the marker proteins according to the present invention a control Another embodiment of the present invention is a method for the determination whether a patient with grass and/or birch pollen allerav benefits from treatment with AIT characterized in determining the expression level of the marker proteins according to the present invention in-vitro in a plasma sample from said allergic patient, wherein in case the expression of one or more of the above-mentioned marker proteins is above or below a certain cut-off, the patient is eligible to such treatment with AIT.

Another embodiment not part of the present invention is a method for the selection of an AIT treatment regimen for a patient with grass and/or birch pollen allergy using the method according to the present invention.

The above-mentioned marker proteins are predictive biomarkers for a successful treatment with AIT. Thus, another embodiment of the present invention is use of a marker protein according to the present invention in the identification of a patient with grass and/or birch pollen allergy responsive to treatment with AIT comprising determining in-vitro in a plasma sample from said patient the level of expression of said marker protein.

Another embodiment of the present invention is the use of an assay device for the identification of an allergic patient responsive to treatment with AIT using the method according to the present invention.

Another embodiment of the present invention is the use of an assay device for the identification of a patient as eligible for AIT using the method according to the present invention.

Another embodiment of the present invention is the use of a kit (set) for the identification of an allergic patient responsive to treatment with AIT, which comprises:
a) means for determining the level of expression of one or more marker proteins according to the present invention,
b) a standard control curve or control value showing a relationship between the level of expression of the marker proteins in plasma or nasal fluid samples and the responsiveness to AIT of allergic patients, and
c) a control sample or control value indicative of the level of expression of the marker proteins in a plasma or nasal fluid sample from a non-responder to AIT.

The kit (set) comprises suitable containers, such as boxes or cartons, individual bottles, bags or ampoules.

Another embodiment not part of the present invention is a method for the identification of a patient with grass and/or birch pollen allergy using the method according to the present invention and comparing the expression levels of the marker proteins according to the present invention with a control

Another embodiment not part of the present invention is a method for the identification of a patient with grass and/or birch pollen allergy characterized in determining the expression level of the marker proteins according to the present invention in-vitro in a plasma sample from said patient, wherein in case the expression of one or more of the above-mentioned marker proteins is above or below a certain cut-off, the patient is an allergic patient.

Another embodiment not part of the present invention is use of a marker protein according to the present invention in the identification of a patient with grass and/or birch pollen allergy using the method according to the present invention.

The level of expression of the marker protein may be determined by gel electrophoresis (SDS-PAGE), in particular one- and two-dimensional gel electrophoresis (1D-, 2D-PAGE), carried out on the sample or a protein-containing extract thereof. 2D-PAGE is a well-established technique in which proteins are first separated in one dimension by isoelectric focusing and further separated by SDS-PAGE along a second dimension. Protein expression may be analysed by visualization of labeled proteins, or by western blotting (i.e. using a monoclonal or polyclonal antibody). Protein quantitation by 2D-PAGE is usually carried out by 2D-DiGE, in which proteins from a control sample and the test sample are labeled with different dyes. The dyes are of similar mass and identical charge so the labeled proteins migrate to the same position on the gel, allowing quantitation to be carried out within a single gel. The level of expression of the marker protein may also be determined by mass spectrometry assays (LC-MS or LC-MS/MS). Qualitative and quantitative mass spectrometric techniques are known and used in the art. To this aim, target peptides specific for marker proteins are selected and quantified based on calibration curves established with synthetic peptides labeled with stable isotopes. Enzymatic digests, spiked with a defined amount of isotope labeled

Another embodiment not part of the present invention is a method of identifying an allergic patient comprising determining in-vitro in a plasma sample from said patient the level of expression of
a combination of two or more marker proteins selected from the group consisting of CCL8, VEGFD and WNT9A or of an mRNA thereof.

Another embodiment not part of the present invention is a method of identifying an allergic patient comprising determining in-vitro in a nasal fluid sample from said patient the level of expression of
a combination of two or more marker proteins selected from the group consisting of ALOX15, KIAA0513, SULT1A1, RSPH3, GMPPA, TUBB2A, LRRC46, UBE2N, IFT46, MYCBP, PLIN3, LZTFL1, KRT75, HSPA9, ATP5F1B, FABP5, SIRT2, IRAK4, DAPP1, ICA1, SH2B3, PIK3AP1, ZBTB16, ZBTB17, DCTN1, TANK, BIRC2, IRF9, ARHGEF12, PAK4, PPP1R9B, DGKZ, GCNT1, IL22RA1, CTSO and METRNL or of an mRNA thereof.

Another embodiment not part of the present invention is a method for the identification of an allergic patient using the method according to the present invention and comparing the expression levels of the marker proteins according to the present invention or the mRNAs thereof with a control.

Another embodiment not part of the present invention is a method for the identification of an allergic patient characterized in determining the expression level of the marker proteins according to the present invention or the mRNAs thereof in-vitro in a plasma sample or a nasal fluid sample from said patient, wherein in case the expression of one or more of the above-mentioned marker proteins or the mRNAs thereof is above or below a certain cut-off, the patient is an allergic patient.

Another embodiment not part of the present invention is a marker protein according to the present invention or an mRNA thereof for use in the identification of an allergic patient using the method according to the present invention.

The level of expression of the marker protein may be determined by gel electrophoresis (SDS-PAGE), in particular one- and two-dimensional gel electrophoresis (1D-, 2D-PAGE), carried out on the sample or a protein-containing extract thereof. 2D-PAGE is a well-established technique in which proteins are first separated in one dimension by isoelectric focusing and further separated by SDS-PAGE along a second dimension. Protein expression may be analysed by visualization of labeled proteins, or by western blotting (i.e. using a monoclonal or polyclonal antibody). Protein quantitation by 2D-PAGE is usually carried out by 2D-DiGE, in which proteins from a control sample and the test sample are labeled with different dyes. The dyes are of similar mass and identical charge so the labeled proteins migrate to the same position on the gel, allowing quantitation to be carried out within a single gel. The level of expression of the marker protein may also be determined by mass spectrometry assays (LC-MS or LC-MS/MS). Qualitative and quantitative mass spectrometric techniques are known and used in the art. To this aim, target peptides specific for marker proteins are selected and quantified based on calibration curves established with synthetic peptides labeled with stable isotopes. Enzymatic digests, spiked with a defined amount of isotope labeled target peptides, are analysed by liquid chromatography coupled with mass spectrometry. The ratio between labeled and nonlabelled target peptides is measured to assess target peptide concentrations and therefore protein marker concentration.

The level of expression of the marker protein may also be determined using an antibody which binds to the protein, for example a monoclonal or polyclonal antibody, an antibody variant or fragments such as a single chain antibody, a diabody, a minibody, a single chain Fv fragment (sc(Fv)), a Sc(Fv), antibody, a Fab fragment or a F(ab'), fragment, a VHH antibody or a single domain antibody. The antibody may be mono-, bi-, tri- or multivalent. The antibody may be immobilized on a solid support. Antibodies may be used to determine protein expression in a range of immunological assays including competitive and non-competitive assay systems using techniques such as western blotting, immunohistochemistry / immunofluorescence (i.e. protein detection on fixed cells or tissues), radioimmunoassay such as RIA (radio-linked immunoassay), ELISA (enzyme linked immunosorbent assay), "sandwich" immunoassays, immunoprecipitation assays, immunodiffusion assays, agglutination assays, complement-fixation assays, immunoradiometric assays, fluorescent immunoassays, e.g. FIA (fluorescence-linked immunoassay), chemiluminescence immunoassays, ECLIA (electrochemiluminescence immunoassay) and protein A immunoassays.

Such assays are routine and well known to the person skilled in the art. The level of expression of the marker protein may alternatively be determined using a protein-specific aptamer. An aptamer is a short peptide capable of specifically binding to a specific protein sequence, consisting of a variable peptide loop attached at both ends to a protein scaffold. Methods for making protein aptamers are well known in the art, the most commonly used method being the yeast twohybrid system. Such aptamers may preferably be labeled in order to allow the detection of a protein-ligand interaction. A nanotechnology-based assay could also be used. Accordingly, in particular embodiments, the level of expression of the at least one marker protein as defined above is determined by immunological assay, mass spectrometry assays or gel electrophoresis.

The level of expression of mRNAs of the marker proteins may be determined by real-time quantitative RT-PCR, using primers specific for the marker proteins to be measured. This method allows the detection of mRNA in a biological sample by generating cDNA by reverse transcription using at least one primer; amplifying the cDNA so produced using gene specific polynucleotides as sense and antisense primers and detecting the presence of the amplified cDNA by methods well known to the person skilled in the art. This includes cDNA amplification with specific predesigned primers using SYBR GREEN or Taqman reagents. Other methods', such as high throughput sequencing, are also applicable.

The term "marker protein" or "biomarker" includes all isoforms of said proteins. Thus, for the marker proteins described above, the term "marker protein" or "biomarker" includes the polypeptide having the protein name, the synonyms, the amino acid sequences, the gene ID number, the Pubmed Entrez number, the protein ID or accession number, the Uniprot ID or accession number disclosed herein and all isoforms thereof.

FGCZ Plasma Responders vs. Non-responders (in all of the following tables only marker proteins selected from the group consisting of CCL8, ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 and METAP1D are according to the invention and the remaining markers are present for illustration purpose only).

| **Marker Protein / Gene Name** | **Gene ID** | **Protein ID** |
|---|---|---|
| ACTB | ENSG00000075624 | P60709;P63261.ACTB_HUMAN; ACTG_HUMAN.ACTB;ACTG1 ACTG |
| APOA1 | ENSG00000118137 | P02647.APOA1_HUMAN.APOA1 |
| APOA2 | ENSG00000158874 | P02652.APOA2_HUMAN.APOA2 |
| APOC3 | ENSG00000110245 | P02656.APOC3_HUMAN.APOC3 |
| APOM | ENSG00000235754; ENSG00000204444; ENSG00000227567; ENSG00000224290; ENSG00000226215; ENSG00000231974; | O95445.APOM_HUMAN.APOM G3A NG20 HSPC336 |
| | ENSG00000206409 | |
| C2 | ENSG00000231543; ENSG00000235017; ENSG00000166278; ENSG00000226560; ENSG00000204364; ENSG00000206372; ENSG00000235696 | P06681.CO2_HUMAN.C2 |
| C3 | ENSG00000125730 | P01024.CO3_HUMAN.C3 CPAMD1 |
| C4A | ENSG00000244207; ENSG00000244731; ENSG00000206340; ENSG00000227746 | P0C0L4.CO4A_HUMAN.C4A CO4 CPAMD2 |
| FERMT3 | ENSG00000149781 | Q86UX7.URP2_HUMAN.FERMT3 KIND3 MIG2B URP2 |
| FLNA | ENSG00000196924 | P21333.FLNA_HUMAN.FLNA FLN FLN1 |
| GPLD1 | ENSG00000112293 | P80108.PHLD_HUMAN.GPLD1 PIGPLD1 |
| HPR | ENSG00000261701 | P00739.HPTR HUMAN.HPR |
| HSPA8 | ENSG00000109971 | P11142.HSP7C_HUMAN.HSPA8 HSC70 HSP73 HSPA10 |
| ITGB3 | ENSG00000259207 | P05106.ITB3_HUMAN.ITGB3 GP3A |
| ITIH1 | ENSG00000055957 | P19827.ITIH1_HUMAN.ITIH1 IGHEP1 |
| ITIH2 | ENSG00000151655 | P19823.ITIH2_HUMAN.ITIH2IGHEP2 |
| ITIH3 | ENSG00000162267 | Q06033.ITIH3_HUMAN.ITIH3 |
| KLKB1 | ENSG00000164344 | P03952.KLKB1_HUMAN.KLKB1 KLK3 |
| LTF | ENSG00000012223 | P02788.TRFL_HUMAN.LTF GIG12 LF |
| MYL6 | ENSG00000092841 | P60660.MYL6_HUMAN.MYL6 |
| SERPINA6 | ENSG00000170099; ENSG00000277405 | P08185.CBG_HUMAN.SERPINA6 CBG |
| SERPIND1 | ENSG00000099937 | P05546. HEP2_HUMAN. SERPI ND 1 HCF2 |
| SHBG | ENSG00000129214 | P04278.SHBG_HUMAN.SHBG |
| VCL | ENSG00000035403 | P18206.VINC_HUMAN.VCL |
| VWF | ENSG00000110799 | P04275.VWF_HUMAN.VWF F8VWF |

### OLINK Plasma Responders vs. Non-responders

| **Marker Protein / Gene Name** | **ENTREZ ID** | **Uniprot ID** |
|---|---|---|
| APLP1 | 333 | P51693 |
| AREG | 374 | P15514 |
| ARNT | 405 | P27540 |
| BTN3A2 | 11118 | P78410 |
| CCL11 | 6356 | P51671 |
| CCL8 | 6355 | P80075 |
| CD274 | 29126 | Q9NZQ7 |
| CD6 | 923 | P30203 |
| CHRDL2 | 25884 | Q6WN34 |
| CXCL11 | 6373 | O14625 |
| CXCL5 | 6374 | P42830 |
| GDNF | 2668 | P39905 |
| GFRA2 | 2675 | O00451 |
| GHRL | 51738 | Q9UBU3 |
| GKN1 | 56287 | Q9NS71 |
| GSAP | 54103 | A4D1B5 |
| IL18 | 3606 | Q14116 |
| IL4 | 3565 | P05112 |
| IL6 | 3569 | P05231 |
| KLRD1 | 3824 | Q13241 |
| MEP1B | 4225 | Q16820 |
| METAP1D | 254042 | Q6UB28 |
| MMP1 | 4312 | P03956 |
| PROK1 | 84432 | P58294 |
| REG4 | 83998 | Q9BYZ8 |
| SIGLEC7 | 27036 | Q9Y286 |
| SOST | 50964 | Q9BQB4 |

### FGCZ Nasal Responders vs. Non-responders (not according to the invention)

| **Marker Protein / Gene Name** | **Gene ID** | **Protein ID** |
|---|---|---|
| A2M | ENSG00000175899 | P01023.A2MG_HUMAN.A2M CPAMD5 FWP007 |
| AGT | ENSG00000135744 | P01019.ANGT_HUMAN.AGT SERPINA8 |
| AK2 | ENSG00000004455 | P54819.KAD2_HUMAN.AK2 ADK2 |
| ALOX15 | ENSG00000161905 | P16050. LOX15 _HUMAN. ALOX15 LOG15 |
| APOB | ENSG00000084674 | P04114.APOB_HUMAN.APOB |
| BPIFA1 | ENSG00000198183 | Q9NP55.BPIA1_HUMAN.BPIFA1 LUNX NASG PLUNC SPLUNC1 SPURT UNQ787/PRO1606 |
| BPIFB2 | ENSG00000078898 | Q8N4F0.BPIB2_HUMAN.BPIFB2 BPIL1 C20orf184 LPLUNC2 UNQ2489/PRO5776 |
| C1QBP | ENSG00000108561 | Q07021.C1QBP_HUMAN.C1QBP GC1QBP HABP1 SF2P32 |
| C3 | ENSG00000125730 | P01024.CO3_HUMAN.C3 CPAMD1 |
| C5 | ENSG00000106804 | P01031.CO5_HUMAN.C5 CPAMD4 |
| C6 | ENSG00000039537 | P13671.CO6_HUMAN.C6 |
| CA13 | ENSG00000185015 | Q8N1Q1.CAH13_HUMAN.CA13 |
| CALR | ENSG00000179218 | P27797.CALR_HUMAN.CALR CRTC |
| CFB | ENSG00000241534; ENSG00000204359; ENSG00000243649; ENSG00000242335; ENSG00000243570; ENSG00000241253; ENSG00000239754 | P00751.CFAB_HUMAN.CFB BF BFD |
| CFH | ENSG00000000971 | P08603.CFAH_HUMAN.CFH HF HF1 HF2 |
| CP | ENSG00000047457 | P00450.CERU_HUMAN.CP |
| CP | ENSG00000047457 | P00450.CERU_HUMAN.CP |
| ERICH3 | ENSG00000178965 | Q5RHP9.ERIC3_HUMAN.ERICH3 C1orf173 |
| ERP29 | ENSG00000089248 | P30040.ERP29_HUMAN.ERP29 C12orf8 ERP28 |
| FGA | ENSG00000171560 | P02671.FIBA_HUMAN.FGA |
| FGG | ENSG00000171557 | P02679.FIBG_HUMAN.FGG PRO2061 |
| HLA-B | ENSG00000234745; ENSG00000223532; ENSG00000232126; ENSG00000224608; ENSG00000206450; ENSG00000228964 | P30461.1B13_HUMAN.HLA-B HLAB |
| HRG | ENSG00000113905 | P04196.HRG_HUMAN.HRG |
| HSP90B1 | ENSG00000166598 | P14625.ENPL_HUMAN. HSP90B1 GRP94 TRA1 |
| HSPA5 | ENSG00000044574 | P11021.BIP_HUMAN.HSPA5 GRP78 |
| HSPA9 | ENSG00000113013 | P38646.GRP75_HUMAN.HSPA9 GRP75 HSPA9B mt-HSP70 |
| HSPE1 | ENSG00000115541 | P61604.CH10_HUMAN.HSPE1 |
| IGHG1 | ENSG00000211896; ENSG00000277633 | P01857.IGHG1_HUMAN.IGHG1 |
| ITIH1 | ENSG00000055957 | P19827.ITIH1_HUMAN.ITIH1 IGHEP1 |
| ITIH2 | ENSG00000151655 | P19823.ITIH2_HUMAN.ITIH2 IGHEP2 |
| ITIH4 | ENSG00000055955 | Q14624_ITIH4_HUMAN.ITIH4 IHRP ITIHL1 PK120 PRO1851 |
| KIAA1522 | ENSG00000162522 | Q9P206.K1522_HUMAN.KlAA1522 |
| KLKB1 | ENSG00000164344 | P03952.KLKB1_HUMAN.KLKB1 KLK3 |
| KNG1 | ENSG00000113889 | P01042.KNG1_HUMAN.KNG1 BDK KNG |
| KRT4 | ENSG00000170477 | P19013.K2C4_HUMAN.KRT4 CYK4 |
| KTN1 | ENSG00000126777 | Q86UP2.KTN1_HUMAN.KTN1 CG1 KIAA0004 |
| LEG1 | NA | Q6P5S2.LEG1H_HUMAN.LEG1 C6orf58 |
| LGALS7 | ENSG00000205076; ENSG00000283082 | P47929.LEG7_HUMAN.LGALS7 PIG1; LGALS7B |
| LRG1 | ENSG00000171236 | P02750.A2GL_HUMAN.LRG1 LRG |
| LTF | ENSG00000012223 | P02788.TRFL_HUMAN.LTF GIG12 LF |
| P4HB | ENSG00000185624 | P07237.PDIA1_HUMAN.P4HB ERBA2L PDI PDIA1 PO4DB |
| PDIA3 | ENSG00000167004 | P30101.PDIA3_HUMAN.PDIA3 ERP57 ERP60 GRP58 |
| PDIA4 | ENSG00000155660 | P13667.PDIA4_HUMAN.PDIA4 ERP70 ERP72 |
| PDIA6 | ENSG00000143870 | Q15084.PDIA6_HUMAN.PDIA6 ERP5 P5 TXNDC7 |
| PLG | ENSG00000122194 | P00747.PLMN_HUMAN.PLG |
| POSTN | ENSG00000133110 | Q15063.POSTN_HUMAN.POSTN OSF2 |
| POSTN | ENSG00000133110 | Q15063.POSTN_HUMAN.POSTN OSF2 |
| PPP3R1 | ENSG00000221823 | P63098.CANB1_HUMAN.PPP3R1 CNA2 CNB |
| SAMHD1 | ENSG00000101347 | Q9Y3Z3.SAMH1_HUMAN.SAMHD1 MOP5 |
| SERPINA1 | ENSG00000277377; ENSG00000197249 | P01009.A1AT_HUMAN.SERPINA1 AAT PI PRO0684 PRO2209 |
| SERPIND1 | ENSG00000099937 | P05546.HEP2_HUMAN.SERPIND1 HCF2 |
| TF | ENSG00000091513 | P02787.TRFE_HUMAN.TF PRO1400 |

### OLINK Nasal Responders vs. Non-responders (not according to the invention)

| **Marker Protein / Gene Name** | **ENTREZ ID** | **Uniprot ID** |
|---|---|---|
| CDNF | 441549 | Q49AH0 |
| CKAP4 | 10970 | Q07065 |
| CPXM1 | 56265 | Q96SM3 |
| CRISP2 | 7180 | P16562 |
| CXCL5 | 6374 | P42830 |
| KLK12 | 43849 | Q9UKR0 |
| PRDX3 | 10935 | P30048 |
| PREB | 10113 | Q9HCU5 |
| REG4 | 83998 | Q9BYZ8 |
| STX16 | 8675 | 014662 |
| SUMF2 | 25870 | Q8NBJ7 |
| TXNDC5 | 81567 | Q8NBS9 |

### OLINK Plasma Allergic patient vs. Healthy

| **Marker** | **ENTREZ ID** | **Uniprot ID** |
|---|---|---|
| **Protein / Gene Name** | | |
| CCL28 | 56477 | Q9NRJ3 |
| VEGFD | 2277 | O43915 |
| WNT9A | 7483 | O14904 |

### FGCZ Nasal Allergic patients vs. Healthy (not according to the invention)

| **Marker Protein / Gene Name** | **Gene ID** | **Protein ID** |
|---|---|---|
| ALOX15 | ENSG00000161905 | P16050.LOX15_HUMAN.ALOX15 LOG15 |
| ATP5F1B | NA | P06576.ATPB_HUMAN.ATP5F1B ATPSB ATPMB ATPSB |
| FABP5 | ENSG00000164687 | Q01469.FABP5_HUMAN.FABP5 |
| GMPPA | ENSG00000144591 | Q96IJ6.GMPPA_HUMAN.GMPPA |
| HSPA9 | ENSG00000113013 | P38646.GRP75_HUMAN.HSPA9 GRP75 HSPA9B mt-HSP70 |
| IFT46 | ENSG00000118096 | Q9NQC8.IFT46_HUMAN.IFT46 C11orf2 C11orf60 |
| KIAA0513 | ENSG00000135709 | O60268.K0513_HUMAN.KIAA0513 |
| KRT75 | ENSG00000170454 | O95678.K2C75_HUMAN.KRT75 K6HF KB18 |
| LRRC46 | ENSG00000141294 | Q96FV0.LRC46_HUMAN.LRRC46 |
| LZTFL1 | ENSG00000163818 | Q9NQ48.LZTL1_HUMAN.LZTFL1 |
| MYCBP | ENSG00000214114 | Q99417.MYCBP_HUMAN.MYCBP AMY1 |
| PLIN3 | ENSG00000105355 | O60664.PLIN3_HUMAN.PLIN3 M6PRBP1 TIP47 |
| RSPH3 | ENSG00000130363 | Q86UC2.RSPH3_HUMAN.RSPH3 RSHL2 RSP3 |
| SULT1A1 | ENSG00000196502 | P50225.ST1A1_HUMAN.SULT1A1 STP STP1 OK/SW-cl.88 |
| TUBB2A | ENSG00000137267 | Q13885.TBB2A_HUMAN.TUBB2A TUBB2 |
| UBE2N | ENSG00000177889 | P61088.UBE2N_HUMAN.UBE2N BLU |

### OLINK Nasal Allergic patients vs. Healthy (not according to the invention)

| **Marker Protein / Gene Name** | **ENTREZ ID** | **Uniprot ID** |
|---|---|---|
| ARHGEF12 | 23365 | Q9NZN5 |
| BIRC2 | 329 | Q13490 |
| CTSO | 1519 | P43234 |
| DAPP1 | 27071 | Q9UN19 |
| DCTN1 | 1639 | Q14203 |
| DGKZ | 8525 | Q13574 |
| GCNT1 | 2650 | Q02742 |
| ICA1 | 3382 | Q05084 |
| IL22RA1 | 58985 | Q8N6P7 |
| IRAK4 | 51135 | Q9NWZ3 |
| IRF9 | 10379 | Q00978 |
| METRNL | 284207 | Q641Q3 |
| PAK4 | 10298 | O96013 |
| PIK3AP1 | 118788 | Q6ZUJ8 |
| PPP1R9B | 84687 | Q96SB3 |
| SH2B3 | 10019 | Q9UQQ2 |
| SIRT2 | 22933 | Q8IXJ6 |
| TANK | 10010 | Q92844 |
| ZBTB16 | 7704 | Q05516 |
| ZBTB17 | 7709 | Q13105 |

"Isoform" refers to all alternative forms of a protein, for example amino-acid substituted forms, alternatively spliced versions and post-translationally modified forms such as glycoforms. Post-translationally modified isoforms may include acetylated, formylated, lipoylated, myristoylated, palmitoylated, alkylated, methylated, amidated, glycosylated, hyrdroxylated, nitrosylated, phosphorylated, sulphated, polysialylated and sialylated forms. Isoforms include naturally occurring variants, allelic variants, SNPs (single nucleotide polymorphisms), alternative splice variants and truncated or secreted forms of the protein. Alternatively, spliced and truncated mRNAs encoding the marker proteins may also be detected. Detection of the "level of expression" of a marker protein may refer to the level of expression of any individual isoform of said protein, the collective level of expression of selected isoforms of said protein, or the total level of expression of said protein including the reference sequence and all isoforms.

"Plasma sample" or "nasal fluid sample" as used herein denotes a plasma sample or nasal fluid sample obtained from a patient, i.e. either a healthy individual or an allergic patient according to the present invention. An example of the preparation of a plasma sample and a nasal fluid sample for the in-vitro determination of the expression of the marker proteins according to the present invention is disclosed in example 1 but a plasma sample or a nasal fluid sample can alternatively be obtained by methods well-known by the skilled artesian and depending on the expression determination method.

"In-vitro" as used herein denotes that a blood sample is obtained from a patient and the plasma is isolated from the blood sample or a nasal fluid sample is obtained from a patient and prepared for the in-vitro determination of the expression of the marker proteins according to the present invention as disclosed in example 1. "In-vitro" as used herein includes or is a synonym for the term "ex-vivo".

"Control" or "standard value" as used herein denotes in the case for identification of an allergic patient responsive for the treatment with AIT the mean value or arithmetical mean of the expression level of the individual marker protein obtained from non-responders. "Control" or "standard value" as used herein denotes in the case for identification of an allergic patient the mean value or arithmetical mean of the expression level of the individual marker protein obtained from healthy individuals.

"Sensitivity" as used herein denotes the proportion of actual positives that are correctly identified. It is also called the true positive rate. As an example, it shows the percentage of truly identified responder patients that are identified with a certain biomarker. The sensitivity is calculated by dividing the number of true positives by the number of total positives (total positive = true positive + false negative).

"Specificity" as used herein denotes the proportion of actual negatives that are correctly identified. It is also called the true negative rate. As an example, it shows the percentage of truly identified non-responder patients that are identified with a certain biomarker. The specificity is calculated by dividing the number of true negatives by the number of total negatives (total negative = true negative + false positive).

"Therapy", "therapeutic", "treatment" or "treating" include reducing, alleviating or inhibiting or eliminating the symptoms of allergies, as well as treatment intended to reduce, alleviate, inhibit or eliminate said symptoms. These terms may include preventive treatment which is intended to, or has the effect of, reducing, alleviating, inhibiting or eliminate future symptoms. They may also include treatment of ongoing symptoms.

"Allergy" or "type 1 hypersensitivity", is a condition characterized by production of allergen-specific IgE in response to a specific allergen, usually a protein. Clinical manifestations and symptoms of allergy may include nasal congestion, nasal pruritis, ocular pruritis, tearing, rhinorrhoea, sinusitis, rhinitis, sneezing, wheezing, conjunctivitis, dermal itching, dermatitis, skin irritation and asthma.

An "allergen" is a substance, usually a protein, which elicits the production of IgE antibodies in predisposed individuals. Allergens may include pollen allergens (such as tree, herb, weed and grass pollen allergens), insect allergens (such as inhalant, saliva and venom allergens, e.g. cockroach, midge and house dust mite allergens and hymenoptera venom allergens), animal hair and dander allergens (from e.g. dog, cat, horse, rat, mouse, rabbit) and food allergens (from e.g. tree nuts, peanut, milk, egg). In a preferred embodiment, the patient has grass pollen allergy and the immunotherapy uses grass pollen allergen.

For instance, a protein allergen may be selected from the group consisting of a protein allergen of the genus Dermatophagoides; a protein allergen of the genus Felis; a protein allergen of the genus Ambrosia; a protein allergen of the genus Lolium; a protein allergen of the genus Cryptomeria; a protein allergen of the genus Alternaria; a protein allergen of the genus Alder, a protein allergen of the genus Betula; a protein allergen of the genus of Blomia; a protein allergen of the genus Quercus; a protein allergen of the genus Olea; a protein allergen of the genus Artemisia; a protein allergen of the genus Plantago; a protein allergen of the genus Parietaria; a protein allergen of the genus Canine; a protein allergen of the genus Blattella; a protein allergen of the genus Apis; a protein allergen of the genus Cupressus; a protein allergen of the genus Thuya; a protein allergen of the genus Chamaecyparis; a protein allergen of the genus Periplaneta; a protein allergen of the genus Agropyron; a protein allergen of the genus Secale; a protein allergen of the genus Triticum; a protein allergen of the genus Cynorhodon; a protein allergen of the genus Juniperus; a protein allergen of the genus Dactylis; a protein allergen of the genus Festuca; a protein allergen of the genus Poa; a protein allergen of the genus Lolium; a protein allergen of the genus Avena; a protein allergen of the genus Holcus; a protein allergen of the genus Anthoxanthum; a protein allergen of the genus Arrhenatherum; a protein allergen of the genus Agrostis; a protein allergen of the genus Phleum; a protein allergen of the genus Phalaris; a protein allergen of the genus Paspalum; and a protein allergen of the genus Sorghum. "Officially recognized allergens are listed at the website of the WHO/IUIS Allergen Nomenclature Sub-Committee at www.allergen.org". Examples of various known protein allergens derived from some of the above-identified genus include: Betula (verrucosa) Bet v 1; Bet v 2; Blomia Blo 1 1; Blo t 3; Blo t 5; Blo t 12; Cynorhodon Cyn d 1; Dermatophagoides (pteronyssinus or farinae) Der p 1; Der p 2; Der p 3; Der p 7; Der f 1; Der f 2; Der f 3; Der f 7; Felis (domesticus) Fel d 1; Ambrosia (artemiisfolia) Amb a 1.1; Amb a 1.2; Amb a 1.3; Amb a 1.4; Amb a 2; Lollium (perenne) Lol p 1; Lot p 2; Lol p 3; Lot p 4; Lol p 9 (Lol p 5 or Lol p 1b); Cryptomeria (japonica) Cry j 1; Cry j 2; Canis (familiaris) Can f 1; Can f 2; Juniperus (sabinoides or virginiana) Jun s 1; Jun v 1; Juniperus (ashei) Jun a 1; Jun a 2; Dactylis (glomerata) Dae g 1; Dae g 5; Poa (pratensis) Poa p 1; Phl p 1; Phl p 5; Phl p 6 and Sorghum (halepensis) Sor h 1.

"Allergic immunotherapy" or "AIT" is intended to mean a treatment of an allergic disease by inducing, enhancing, or suppressing an immune response by administration a naturally occurring allergen, an allergoid, a recombinant allergen, a hypoallergenic allergen or a peptide, fragment or multimer therefrom.

"Vaccine" refers to a pharmaceutical composition comprising an allergen as an antigen and optionally an adjuvant to stimulate the immune system of an individual to develop adaptive immunity to said antigen. The antigen may be a naturally occurring allergen, an allergoid, a recombinant allergen, a hypoallergenic allergen or a peptide, fragment or multimer therefrom. Vaccines can be prophylactic (e.g. to prevent or ameliorate the effects of a future contact with the naturally occurring allergens).

The substance used in immunotherapy and the vaccine may be administered via a parenteral route, such as subcutaneously or intravenously, for example via injection, or via alternative routes such as intranasal, skin immunisation e.g. transdermal, epicutaneous, intralymphatic administration or mucosal (administration on mucosal surfaces, e.g. a sublingual, oral, nasal, buccal, ocular, rectal, urinal, vaginal, pulmonary or otolar surface). In relation to allergy, immunotherapy may for example consist of administering an allergen to a patient with the aim of reducing current or future immune response, such as an IgE response, and/or manifestation of clinical symptoms of allergy. Immunotherapy is conventionally carried out by administering repeatedly a monodose or incremental doses of an allergen to a patient in need thereof, thereby resulting in an adaptive immune response of the patient who becomes desensitised to the allergen. Immunotherapy may comprise administration of allergen to a mucosal surface, optionally a sublingual, oral, nasal, buccal, ocular, rectal, urinal, vaginal, pulmonary or otolar surface. In particular, immunotherapy may be sublingual immunotherapy. Alternatively, immunotherapy may comprise administration via a parenteral route, such as subcutaneously or intravenously, for example via injection, or via alternative routes such as intranasal, skin immunisation e.g. transdermal, or intralymphatic administration.

The allergen used for immunotherapy may be a single allergenic substance or a mixture of such substances, for example a mixture of proteins. It may be a partially or fully purified extract, such as a pollen extract, a recombinant protein, a hypoallergen or peptide derived therefrom. For example, where the immunotherapy is used to treat grass pollen allergy, the allergen administered for immunotherapy may be a grass pollen extract from pollen of one or several genera of grasses, such as Dactylis, Poa, Lolium, Anthoxanthum and Phleum genera. The allergen may also be an allergoid, i.e. a chemically modified form of a naturally occurring allergen which has been chemically modified (for example by aldehydation). The allergen may be administered in conjunction with an adjuvant.

"Patient" as used herein includes any individual who is either an "allergic patient" or a "healthy patient", "healthy individuum" or "healthy donor" as defined hereafter.

"Allergic patient" includes any individual who is a candidate for allergic immunotherapy or vaccination including individuals not currently undergoing therapy. Concerning allergy, in most cases, the patient will be an individual who has, or has had at any time in the past, clinical symptoms of allergy and/or sensitization to an allergen and/or an allergen-specific IgE response, or an individual at risk of developing such symptoms. Sensitization to an allergen may be assessed by detecting IgE directed against allergen(s) from this source in the serum of the patient or by skin testing with a preparation containing the corresponding allergen(s). The allergen may without limitation include any of the allergens disclosed herein, in particular a grass pollen allergen.

"Healthy patient", "healthy individual" or "healthy donor" denotes a subject who has not previously had an allergy defined above. A healthy patient, healthy individual or healthy donor also does not otherwise e exhibit symptoms of allergic disease. In other words, such healthy patient, healthy individual or healthy donor, if examined by a medical professional, would be characterized as healthy and/or free of symptoms of allergic disease.

"Three", "six" or "twelve" "month from initiation of AIT" as used herein denotes that a plasma sample or nasal fluid sample was obtained from a patient for the in-vitro determination of the expression of the marker proteins of their mRNAs according to the present invention three, six or twelve month from the start or initiation of the immunotherapy or allergen immunotherapy as disclosed herein or as disclosed in example 1.

"Response" of a patient to treatment indicates that the patient manifests a reduction in the clinical symptoms. Clinical symptoms may be assessed over the course of treatment, i.e. symptoms before treatment may be compared to symptoms during and after treatment. Alternatively, a reduction in symptoms may be determined by comparison to a baseline level established before treatment. Concerning allergy, this approach is particularly useful where, for example, immunotherapy is carried out in patients not currently experiencing symptoms, as may be the case for seasonal grass pollen allergy sufferers, who may be treated before the pollen season. Symptoms may be assessed by standard methods, such as patient self-assessment or record of the amount of medication required. The degree of a patient's response to treatment may be assessed by measuring the degree of reduction of severity in symptoms, for example as described in Example 4 below.

A "responder" subject or an allergic patient "responsive" to treatment with AIT as defined herein is a subject who responds to allergic immunotherapy or vaccine administration with an improvement in clinical symptoms, preferably a statistically significant improvement as compared to patients receiving placebo or no treatment. Preferably, a responder subject will demonstrate an improvement in clinical symptoms which is greater than the average or median improvement seen in a random sample of subjects.

A "non-responder" subject or an allergic patient "non-responsive" to treatment with AIT is a subject who does not manifest any improvement in clinical symptoms following allergic immunotherapy or vaccine administration, or who demonstrates a non-statistically significant improvement in symptoms, or who demonstrates an improvement in clinical symptoms which is less than the average or median improvement seen in a random sample of subjects. For example, where the allergy is grass pollen allergy, improvement in clinical symptoms may be detected by a reduction in the frequency or severity of nasal congestion, nasal pruritis, ocular pruritis, tearing, rhinorrhoea, sinusitis, rhinitis, sneezing, wheezing and/or conjunctivitis and/or lessening in the uptake of known relief medication.

Throughout the instant application, the term "comprising" is to be interpreted as encompassing all specifically mentioned features as well optional, additional, unspecified ones. As used herein, the use of the term "comprising" also encompasses the embodiment wherein no features other than the specifically mentioned features are present (i.e. "consisting of') as well as the embodiment wherein features other than the specifically mentioned feature are present provided that the essential characteristics of the composition are not materially affected by their presence (i.e. "consisting essentially of").

The present invention will be further illustrated by the following figures and examples which illustrate the characterization of markers of dendritic cell subsets, and their role in assessing the clinical response of patients undergoing anti-allergy immunotherapy. It should be understood however that these examples are given only by way of illustration of the invention and do not constitute in any way a limitation thereof.

Even without further embodiments, it is assumed that a person skilled in the art will be able to use the above description in the broadest scope. The preferred embodiments should therefore merely be regarded as descriptive disclosure which is absolutely not limiting in any way.

The following examples are thus intended to explain the invention without limiting it. Unless indicated otherwise, per cent data denote per cent by weight. All temperatures are indicated in degrees Celsius. "Conventional work-up": water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate, filtered and evaporated, and the product is purified by chromatography on silica gel and/or by crystallisation.

### Example 1 - Determination of expression of marker protein in plasma or nasal fluid samples (not according to the invention) of allergic patients and healthy patients as controls

### Allergic patients and healthy patient controls

16 Grass and/or birch pollen allergic subjects were recruited in spring/summer 2015 at Inselspital Bern (Bern, Switzerland). The study was initiated in December 2015/January 2016 when patients received subcutaneous immunotherapy to birch and grass (Allergovit). All subjects were living in the region of Bern (Switzerland), clinically diagnosed with pollen allergic rhinitis (positive skin prick test and the presence of allergen-specific IgE with Immunocap (Phadia AB, Uppsala, Sweden). Participants were assessed according to demography and clinical features and did not show any clinically significant concomitant acute or chronic disease. Further exclusion criteria were pregnancy, non-compliance, inability to follow the study procedures, drug or alcohol abuse, or participation in a study involving investigational drug use one month prior and during the course of this study. 10 of 13 allergic subjects completed all study visits (baseline (November/December), 3 months (March/April), 6 months (June/July), 12 months (November/December) from AIT initiation, while three patients left the study due to non-medical reasons. 12 healthy, non-atopic, non-allergic subjects from the same region as allergic patients were followed in the similar seasonal time points (as the patient population to control for potential seasonal changes in the innate immune cell populations.

### Plasma samples collection

Venous blood was collected from each patient and healthy control into the heparinized 10 ml tubes and transported in ambient temperature from the clinic for immediate further processing within 5 hours from sampling. The tubes were next centrifuged for 10 min at 150g in room temperature. Plasma was carefully collected, aliquoted in several 1ml aliquots in 1.4 mL micronic tubes and frozen in - 80°C. All plasma samples were subjected to further untargeted and targeted proteomics in one time and batch together with nasal samples, after finalization of time points in patients and controls.

### Nasal samples collection (not according to the invention)

Nasal samples were collected from each patient and healthy control by thorough brushing the middle meatus area (15 sec) with the Copan swab (Regular flocked swab, plastic applicator, sterile in dry tub (Copan, Italy) ref nr 552C) and then submerged in the eppendorf tube containing 400 ml of cold PBS with protease inhibitor, Complete-EDTA-free (1 tablet in 10 ml). Samples were transported on ice from the clinic for immediate further processing within 5 hours from sampling. Next, they were shaken overnight on the shaker at 500 rpm in 4°C., which was followed by 30 sec vertexing and removal of the collection brush with the careful squeezing of all remaining liquid through the bottomless sterile eppendorf. The fluid was next centrifuged for 10 min at 400g in 4°C, the supernatant transferred to the new Eppendorf which was followed by the second centrifugation step for 20 min at 12000g in 4°C. The final supernatant was aliquoted in two 100-150 ml aliquots and frozen in -80°C. All nasal samples were subjected to further untargeted and targeted proteomics in one time and batch together with plasma samples, after finalization of time points in patients and controls.

### Proteomics methodology

### Untargeted proteomics (Functional Genomics Center Zurich (FGCZ), Switzerland)

### Sample preparation

Plasma and nasal samples were prepared by using a commercial iST Kit (PreOmics, Germany) with an updated version of the protocol. Briefly, 2 µl of serum and 100 µl of nasal samples were solubilized in 'Lyse' buffer, boiled at 95°C for 10 minutes and processed with HIFU for 30 seconds setting the ultrasonic amplitude to 85%. Afterwards, the samples were transferred to the cartridge and digested by adding 50 µl of the 'Digest' solution. After 60 minutes of incubation at 37 °C the digestion was stopped with 100 µl of Stop solution. The solutions in the cartridge were removed by centrifugation at 3800 g, while the peptides were retained by the iST-filter. Finally, the peptides were washed, eluted, dried and resolubilized in 20 µL of buffer (3% acetonitrile, 0.1% formic acid) buffer for LC-MS-analysis and 1 µl of indexed retention time (iRT)-peptides (Biognosys) was spiked in each sample for future retention time calibration.

### Liquid chromatography-mass spectrometry analysis

Mass spectrometry analysis was performed on a Q Exactive HF-X mass spectrometer (Thermo Scientific) equipped with an Easy Spray source (Thermo Scientific) and coupled to a M-Class UPLC (Waters). Solvent composition at the two channels was 0.1% formic acid for channel A and 0.1% formic acid, 99.9% acetonitrile for channel B. For each sample 2 µL and 4 µL peptides of plasma and nasal, respectively, were loaded on a commercial C18 HSS T3 Column (1.8 µm, 300 µm X 150 mm, Waters). The peptides were eluted at a flow rate of 5 µL/min by a gradient from 8 to 24% B in 50 min, 36% B in 10 min and 64% B in 1 min. Plasma and nasal samples were acquired in 2 separate batches, each one with the samples in a randomized order. The mass spectrometer was operated in dataindependent mode (DIA). DIA scans covered a range from 386 to 1016 m/z in 70 windows of 9 m/z as described by Kelstrup et al (Kelstrup CD, Bekker-Jensen DB, Arrey TN, Hogrebe A, Harder A, Olsen JV. J Proteome Res 2018; 17:727-38). The resolution of the DIA windows was set to 15000, with an AGC target value of 3e6, the maximum injection time set of 22 ms and a fixed normalized collision energy (NCE) of 28. Each instrument cycle (3 s) was completed by a full MS scan monitoring 385 to 1015 m/z at a resolution of 120000 (AGC target: 3e6, maximum injection time: 200 ms).

All samples were also analysed in data-dependent mode (DDA) for the generation of spectral libraries: full MS scans covering 350-1400 m/z were conducted at a resolution of 60 000 (AGC target: 3e6, max injection time: 45 ms) and were followed by HCD (higher-energy collision dissociation) fragmentation on the six most intense signals per cycle. HCD spectra were acquired at a resolution of 30000 using a normalized collision energy of 28 and a maximum injection time of 54 ms. The automatic gain control (AGC) was set to 100000 ions. Charge state screening was enabled and singly, above seven and unassigned charge states were rejected. Only precursors with intensity above 41000 were selected for MS/MS. Precursor masses previously selected for MS/MS measurement were excluded from further selection for 30 s, and the exclusion window was set at 10 ppm. The samples were acquired using internal lock mass calibration on m/z 371.1010 and 445.1200. The mass spectrometry proteomics data were handled using the local laboratory information management system (LIMS) (Turker C, Akal F, Joho D, Panse C, Oesterreicher B, Rehrauer H, et al. B-Fabric: The Swiss Army Knife for Life Sciences. EDBT. Lausanne, Switzerland 2010).

### Spectral library generation and protein quantification

The identification of proteins from DDA data was performed using MaxQuant (version 1.4.1.2) (Cox J, Mann M. Nat Biotechnol 2008; 26:1367-72), followed by protein identification using the integrated Andromeda search engine. Spectra were searched against a canonical forward SwissProt database for human, concatenated to a reversed decoyed fasta database and common protein contaminants (NCBI taxonomy ID9606, release date 20161209). Carbamidomethylation of cysteine was set as fixed modification, while methionine oxidation and N-terminal protein acetylation were set as variable. Enzyme specificity was set to trypsin/P allowing a minimal peptide length of 7 amino acids and a maximum of two missed-cleavages. Precursor and fragment tolerance was set to 10 ppm and 20 ppm, respectively for the initial search. The maximum false discovery rate (FDR) was set to 0.01 for peptides and 0.05 for proteins.

Spectra libraries were generated importing the MaxQuant results and the human protein database into Spectronaut 11.0.15038.20.25720, Biognosys) using the spectral library generation functionality and the default parameter settings. All the protein quantification was performed in Spectronaut using the default settings. Briefly: the type of retention time prediction was set to dynamic iRT with a window correction factor of 1. Decoys were generated using a scrambled, dynamic method and the interference correction on MS2 level was enabled. For protein quantification, a Kernel density estimator was used. The quantitative data were extracted using the BGS Factory Report (default) and used for follow-up analysis. From the Spectronaut export, all samples for patient AITB01, AITG03 and AITG08 were removed from both datasets. Furthermore, the nasal samples AITH01V05, AITH09V03 and AITB0902 where considered as outlier due to the low numbers of identified peptides and proteins (less than 600 peptides).

Stringent filtering of the extracted feature groups by the Spectronaut reported qValue was applied. For precursor fragment groups we required a per sample q-Value at most 0.05 and a per experiment q-Value of at most 0.01. The per experiment qValue is the smallest qValue of a precursor in all the samples and it has to be less than 0.01, ensuring that in at least one of the samples a precursor was identified with high confidence. To perform statistical modelling, fragment intensities were aggregated into precursor and peptide intensities. Only proteins identified with at least 2 peptides and observed within at least 60% of samples were used for quantification. Finally, data were normalized with a modified robust z-score transformation. Normalization is applied to remove systematic differences in protein abundance due to different sample concentrations, or different amount of sample loaded on column. Normalization is important, so that true differentially expressed proteins can be detected. To do this the z-score of the log2 transformed intensities is computed, which is updated by the average of the standard deviation of the log2 transformed intensities in all samples. After normalization all samples have a similar distribution.

### Statistical modelling of untargeted proteomics data

The peptide level measurement data were modeled using mixed effect linear models using the R package *Ime4* (Bates D, Mächler M, Bolker B, Walker S. Journal of Statistical Software 2015; 67:1-48). Two different statistical models were fitted: (i) the first model included the factors "visittime" and "group" (allergy and health) and was fitted using the following formula. "transformed Intensity - 1 + visittime + group + visittime * group + (1 | peptide_Id)". The contrasts between factor levels and factor interactions was computed using the R package *multcomp* (Hothorn T, Bretz F, Westfall P. Biom J 2008; 50:346-63).

The second model (ii) included the factors "visittime" and "response" (responders and nonresponders) and was fitted using the following formula: "transformed Intensity ~ 1 + visittime + response + visittime * response + (1 | peptide_ld)". In both models the multiple testing corrections was disabled and no p-value adjustment was applied.

### Targeted proteomics (PEA, Olink AB, Uppsala, Sweden)

Plasma and nasal protein (not according to the invention) data were generated using proximity extension assays (ProSeek, Olink AB, Uppsala, Sweden), as previously described in detail (Assarsson E, Lundberg M, Holmquist G, Bjorkesten J, Thorsen SB, Ekman D, et al. PLoS One 2014; 9:e95192). Four panels of 92 proteins were used to detect predetermined proteins from Cell Regulation, Inflammation, Immune Response and Metabolism panels (Table S1). In brief, paired complementary-oligonucleotide-coupled antibodies detected each protein in the sample. In situation when paired antibodies are brought in close proximity through binding to their target protein, their complementary oligonucleotide sequence formed a double stranded sequence which is a target for the following amplification with qPCR. The qPCR detects the unique DNA sequence formed when complementary oligonucleotide-tags attached to pairs of analyte-specific antibodies hybridize and extend in the presence of DNA polymerase.

82.25% predicted proteins were detectable in plasma samples, which is higher than the expected detectability of 77.5% (based on EDTA plasma from the test healthy donors). Two plasma samples did not pass quality controls. Nasal samples were first assessed for the total protein concentration using BCA kit (Thermo Fisher, Pierce, UK) and adjusted to 1000 mg/ml prior to performing of the assay for normalization of the procedure. After obtaining the results, each protein was multiplied by the dilution ratio to calculate back to their original concentration to serve as an easily obtainable biomarker. 72.25% predicted proteins were detectable in nasal samples. All nasal samples passed successfully the quality control.

Olink PEA translates the Ct values from the qPCR into the relative quantification units, Normalized Protein expression (NPX), using a series of data normalization and transformation. These operations are designed to minimize technical variation and improve interpretability of the results.

### Statistical Modelling of targeted proteomics data

NPX values were modelled using moderated linear model with Bioconductor package *limma* (Ritchie ME, Phipson B, Wu D, Hu Y, Law CW, Shi W, et al. Nucleic Acids Res 2015; 43:e47) to identify differentially expressed protein. Paired information was used in longitudinal comparison. A protein was considered as expressed only when it's above Olink detection limit and quality control in more than half of the subjects in either of two compared groups. The significance level of p-value was 0.05.

### Results FGCZ and OLINK proteomics

The results below are also disclosed in the figures.

### 1.1. Responders vs. Non-Responders - Plasma

### FGCZ Plasma Responders vs. Non-responders - baseline

| **Marker Protein / Gene Name** | **Threshold** | **Specificity** | **Sensitivity** |
|---|---|---|---|
| ITGB3 | -5,5155714 | 0,75 | **1** |
| MYL6 | -5,2290616 | 0,75 | 0,85714286 |
| FLNA | -5,481400875 | 0,8 | 0,75 |
| ACTB | -0,10205167 | 0,8 | 0,75 |
| KLKB1 | -0,82345058 | 0,8 | 0,75 |
| APOA1 | 4,920768207 | 0,8 | 0,75 |
| ITIH1 | 0,767800384 | 0,8 | 0,875 |
| C2 | -2,099085859 | 0,8 | 0,75 |
| SERPINA6 | 1,04019269 | 1 | 0,875 |
| SHBG | -3,519349 | 0,8 | 0,75 |

### OLINK Plasma Responders vs. Non-responders - baseline

| **Marker Protein / Gene Name** | **Threshold** | **Specificity** | **Sensitivity** |
|---|---|---|---|
| GFRA2 | 1,88957 | 0,75 | 1 |
| GKN1 | -0,7602 | 0,75 | 1 |
| CCL11 | 7,660745 | 0,75 | 0,8 |
| BTN3A2 | 3,616155 | 0,75 | 0,8 |
| SIGLEC7 | 3,41769 | 1 | 0,8 |
| IL18 | 8,074285 | 0,875 | 0,8 |
| CHRDL2 | 2,51453 | 0,875 | 0,8 |
| REG4 | 7,76922 | 0,75 | 0,8 |
| GHRL | 2,64712 | 0,875 | 0,8 |
| CXCL11 | 10,51875 | 0,875 | 0,8 |
| MEP1B | 2,53502 | 0,75 | 0,8 |
| GSAP | 2,142805 | 0,875 | 0,8 |
| **CCL8** | **9,59136** | **1** | **1** |
| CXCL5 | 10,52286 | 0,875 | 0,8 |
| METAP1D | 5,42911 | 0,875 | 0,8 |

### FGCZ Plasma Responders vs. Non-responders - 3 months AIT

| **Marker Protein / Gene Name** | **Threshold** | **Specificity** | **Sensitivity** |
|---|---|---|---|
| **LTF** | **-5,3506793** | **1** | **1** |
| KLKB1 | -0,8525635 | 0,8 | 0,75 |
| SERPIND1 | -0,0394006 | 0,8 | 0,75 |
| HPR | 2,64270242 | 0,8 | 0,75 |
| VWF | -4,0432351 | 1 | 0,75 |
| VCL | -6,478693551 | 0,75 | 0,75 |

### OLINK Plasma Responders vs. Non-responders - 3 months AIT

| **Marker Protein / Gene Name** | **Threshold** | **Specificity** | **Sensitivity** |
|---|---|---|---|
| MMP1 | 7,67648 | 0,75 | 0,8 |
| CCL11 | 7,56568 | 0,875 | 0,8 |
| GDNF | 0,83833 | 0,875 | 0,8 |
| CD6 | 5,106855 | 0,75 | 1 |
| IL18 | 8,018045 | 0,875 | 1 |
| CCL8 | 9,328345 | 0,75 | 0,8 |
| MEP1B | 2,497545 | 0,75 | 0,8 |

### FGCZ Plasma Responders vs. Non-responders - 6 months AIT

| **Marker Protein / Gene Name** | **Threshold** | **Specificity** | **Sensitivity** |
|---|---|---|---|
| C3 | 1,71828964 | 0,8 | 0,85714286 |
| ITIH2 | 0,61177569 | 0,8 | 1 |
| APOA2 | 4,50595723 | 0,8 | 0,85714286 |
| APOM | 1,52799189 | 0,8 | 0,85714286 |
| KLKB1 | -0,580295 | 0,8 | 0,85714286 |
| ITIH1 | 0,86824403 | 0,8 | 1 |
| APOA1 | 4,65660059 | 1 | 0,85714286 |
| APOC3 | 5,727078824 | 0,8 | 1 |
| SERPINA6 | 1,01894254 | 0,8 | 1 |
| FERMT3 | -4,6367944 | 0,75 | 0,83333333 |
| VWF | -3,9438037 | 1 | 0,85714286 |
| **ITGB3** | **-4,281775575** | **1** | **1** |
| **HSPA8** | **-5,53088757** | **1** | **1** |

### OLINK Plasma Responders vs. Non-responders - 6 months AIT

| **Marker Protein / Gene Name** | **Threshold** | **Specificity** | **Sensitivity** |
|---|---|---|---|
| MMP1 | 8,303175 | 1 | 0,8 |
| IL6 | 2,488255 | 0,85714286 | 0,8 |
| AREG | 2,94682 | 1 | 0,8 |
| CD274 | 6,349435 | 0,85714286 | 1 |
| SIGLEC7 | 3,479935 | 1 | 0,8 |
| KLRD1 | 6,16078 | 0,85714286 | 0,8 |
| CHRDL2 | 2,634845 | 1 | 0,8 |
| **GHRL** | **2,258735** | **1** | **1** |
| MEP1B | 2,125105 | 0,85714286 | 0,8 |
| APLP1 | 5,639315 | 1 | 0,8 |

### FGCZ Plasma Responders vs. Non-responders - 12 months AIT

| **Marker Protein / Gene Name** | **Threshold** | **Specificity** | **Sensitivity** |
|---|---|---|---|
| **HSPA8** | **-6,695962565** | **1** | **1** |
| C4A | 1,392413485 | 0,75 | 0,857142857 |
| ITIH3 | -2,389370158 | 0,75 | 1 |
| GPLD1 | -1,206281242 | 0,75 | 1 |
| APOA2 | 4,600113399 | 0,75 | 0,857142857 |
| APOA1 | 4,748096865 | 0,75 | 0,857142857 |
| SERPINA6 | 1,094018791 | 0,75 | 1 |
| VWF | -3,786599201 | 0,75 | 0,857142857 |

### OLINK Plasma Responders vs. Non-responders - 12 months AIT

| **Marker Protein / Gene Name** | **Threshold** | **Specificity** | **Sensitivity** |
|---|---|---|---|
| ARNT | 1,430195 | 1 | 0,75 |
| IL4 | 0,41933 | 0,857142857 | 0,75 |
| CCL11 | 7,64847 | 0,857142857 | 0,75 |
| SIGLEC7 | 3,350205 | 1 | 0,75 |
| GDNF | 0,845935 | 1 | 0,75 |
| GHRL | 2,29721 | 0,857142857 | 0,75 |
| SOST | 5,183595 | 1 | 0,75 |
| CCL8 | 9,397915 | 0,857142857 | 0,75 |
| PROK1 | 2,95491 | 0,857142857 | 0,75 |

### 1.1. Responders vs. Non-Responders - Nasal fluid (not according to the invention)

### FGCZ Nasal Responders vs. Non-responders - baseline

| **Marker Protein / Gene Name** | **Threshold** | **Specificity** | **Sensitivity** |
|---|---|---|---|
| C1QBP | -2,184054971 | 1 | 0,75 |
| **HLA-B** | **-1,597507652** | 1 | **1** |
| PPP3R1 | -2,00841071 | 1 | 0,8 |
| **ERP29** | **-0,272940902** | **1** | **1** |
| CALR | 0,807388306 | 1 | 0,875 |
| **P4HB** | **1,346119436** | **1** | **1** |
| PDIA4 | -1,129633119 | 1 | 0,75 |
| PDIA6 | 0,62348477 | 1 | 0,875 |
| HSP90B1 | -0,220263313 | 1 | 0,75 |
| HSPA5 | 0,750192242 | 1 | 0,875 |
| **PDIA3** | **1,559003258** | **1** | **1** |
| KIAA1522 | -1,427085404 | 1 | 0,857142857 |
| HSPA9 | -1,183013361 | 0,75 | 0,75 |
| HSPE1 | 0,367389371 | 1 | 0,875 |
| AK2 | -1,223579473 | 0,75 | 1 |
| SAMHD1 | 1,478298404 | 1 | 0,875 |
| APOB | -1,72627392 | 1 | 0,75 |
| ALOX15 | 1,905885738 | 0,75 | 1 |
| HRG | 0,602123147 | 0,75 | 0,875 |
| CP | -0,290002179 | 1 | 0,75 |
| CFH | -0,087402248 | 1 | 0,75 |
| C6 | -2,001723067 | 1 | 0,75 |
| LEG1 | 0,561888049 | 1 | 0,75 |
| C3 | 0,132490014 | 1 | 0,75 |
| AGT | -0,934875275 | 0,75 | 0,75 |
| POSTN | -0,681008713 | 0,75 | 1 |
| FGG | 0,556989507 | 1 | 0,75 |
| SERPIND1 | -1,711962162 | 1 | 0,75 |
| KNG1 | 0,84412956 | 1 | 0,75 |
| KLKB1 | -0,566791131 | 0,75 | 1 |
| **ITIH1** | **-0,337602392** | **1** | **1** |
| BPIFB2 | 2,368453212 | 1 | 0,875 |
| ITIH4 | -0,870814333 | 1 | 0,75 |
| IGHG1 | 7,360082123 | 1 | 0,75 |
| PLG | 1,046058916 | 1 | 0,75 |

### OLINK Nasal Responders vs. Non-responders - baseline (not according to the invention)

| **Marker Protein / Gene Name** | **Threshold** | **Specificity** | **Sensitivity** |
|---|---|---|---|
| CRISP2 | 10,59089514 | 0,875 | 0,8 |
| REG4 | 6,411016654 | 0,875 | 1 |
| CDNF | 2,630320985 | 0,875 | 1 |
| PRDX3 | 3,695607196 | 0,75 | 1 |
| STX16 | 3,938874069 | 0,75 | 1 |
| SUMF2 | 7,623459288 | 0,875 | 1 |
| PREB | 3,968952196 | 0,875 | 1 |
| TXNDC5 | 4,902439975 | 0,75 | 1 |
| CKAP4 | 9,0978349 | 0,875 | 1 |
| KLK12 | 7,123289069 | 0,875 | 1 |

### FGCZ Nasal Responders vs. Non-responders - 3 months AIT (not according to the invention)

| **Marker Protein / Gene Name** | **Threshold** | **Specificity** | **Sensitivity** |
|---|---|---|---|
| ERICH3 | -1,177755147 | 0,8 | 0,857142857 |
| KTN1 | -0,784883375 | 0,8 | 0,75 |
| KRT4 | -0,279369038 | 1 | 0,75 |
| TF | 1,949223827 | 0,8 | 1 |
| LRG1 | 0,602897882 | 0,8 | 0,75 |
| HRG | 0,452008131 | 0,8 | 1 |
| LGALS7 | -0,411661573 | 0,8 | 0,75 |
| BPIFB2 | 2,978380539 | 0,8 | 0,875 |
| LEG1 | 0,776575514 | 1 | 0,75 |
| LTF | 3,19418273 | 0,8 | 0,75 |
| CFB | 1,231618562 | 0,8 | 0,875 |
| C3 | 0,652712706 | 0,8 | 1 |
| ITIH4 | -1,093676304 | 0,8 | 0,875 |
| ITIH1 | -0,415739821 | 0,8 | 0,875 |
| IGHG1 | 7,968570497 | 0,8 | 1 |
| CP | -0,146365962 | 0,8 | 0,875 |
| ITIH2 | -0,930089155 | 0,8 | 1 |
| KNG1 | 0,947915383 | 0,8 | 0,875 |
| CFH | -0,329308899 | 0,8 | 1 |
| AGT | -0,87677141 | 0,6 | 1 |
| POSTN | -1,193169998 | 10,8 | 1 |
| C5 | -1,909272746 | 0,8 | 1 |
| FGG | 0,615305569 | 0,8 | 0,875 |
| SERPIND1 | -0,857984862 | 0,6 | 1 |
| BPIFA1 | -0,206920181 | 0,8 | 0,75 |
| C6 | -1,549731193 | 0,8 | 1 |
| PLG | 0,882997953 | 0,8 | 1 |
| KLKB1 | -1,594323598 | 1 | 0,833333333 |

### OLINK Nasal Responders vs. Non-responders - 3 months AIT (not according to the invention)

| **Marker Protein / Gene Name** | **Threshold** | **Specificity** | **Sensitivity** |
|---|---|---|---|
| CXCL5 | 12,5572522 | 0,875 | 1 |
| KLK12 | 6,28375046 | 0,875 | 0,8 |

### FGCZ Nasal Responders vs. Non-responders - 6 months AIT (not according to the invention)

| **Marker Protein / Gene Name** | **Threshold** | **Specificity** | **Sensitivity** |
|---|---|---|---|
| PDIA4 | -1,177011422 | 0,8 | 0,857142857 |
| CALR | 1,541281439 | 1 | 0,857142857 |
| TF | 2,144268668 | 0,8 | 0,857142857 |
| ITIH4 | -1,2220291 | 0,8 | 0,857142857 |
| ITIH2 | -1,996334033 | 0,8 | 0,857142857 |
| CFH | 0,147641534 | 0,8 | 0,857142857 |
| C5 | -2,50282428 | 0,8 | 0,857142857 |
| FGG | 0,914601217 | 0,8 | 0,857142857 |
| IGHG1 | 7,261994634 | 0,8 | 0,857142857 |
| PLG | 1,014569089 | 0,8 | 0,857142857 |
| FGA | 0,726185032 | 0,8 | 1 |
| A2M | -0,051700606 | 0,8 | 0,857142857 |

### OLINK Nasal Responders vs. Non-responders - 6 months AIT (not according to the invention)

| **Marker Protein / Gene Name** | **Threshold** | **Specificity** | **Sensitivity** |
|---|---|---|---|
| CPXM1 | 6,504807875 | 0,857142857 | 0,8 |
| KLK12 | 5,74482 | 0,857142857 | 0,8 |

### FGCZ Nasal Responders vs. Non-responders - 12 months AIT (not according to the invention)

| **Marker Protein / Gene Name** | **Threshold** | **Specificity** | **Sensitivity** |
|---|---|---|---|
| CA13 | -0,595472998 | 0,75 | 0,8 |
| SERPINA1 | 1,188602096 | 0,75 | 0,857142857 |
| KNG1 | 1,137204803 | 0,75 | 0,857142857 |
| ITIH2 | -1,323512406 | 0,75 | 0,857142857 |

### OLINK Nasal Responders vs. Non-responders - 12 months AIT (not according to the invention)

| **Marker Protein / Gene Name** | **Threshold** | **Specificity** | **Sensitivity** |
|---|---|---|---|
| CPXM1 | 7,523368976 | 1 | 0,75 |
| CRISP2 | 10,01221405 | 1 | 0,75 |
| KLK12 | 5,614194865 | 0,857142857 | 1 |

### 1.2. Allergic patient vs. Healthy - Plasma (not according to the invention)

### OLlNK Plasma Allergic patient vs. Healthy - baseline

| **Marker Protein / Gene Name** | **Threshold** | **Specificity** | **Sensitivity** |
|---|---|---|---|
| CCL28 | 1,223785 | 0,916666667 | 0,923076923 |
| VEGFD | 8,526435 | 0,75 | 0,846153846 |
| WNT9A | 1,639805 | 0,916666667 | 0,846153846 |

### 1.3. Allergic patient vs. Healthy - Nasal fluid (not according to the invention)

### FGCZ Nasal Allergic patient vs. Healthy - baseline

| **Marker Protein / Gene Name** | **Threshold** | **Specificity** | **Sensitivity** |
|---|---|---|---|
| ALOX15 | 0,977939714 | 0,916666667 | 0,75 |
| KIAA0513 | -2,204812317 | 0,833333333 | 0,818181818 |
| SULT1A1 | -1,575705514 | 0,8 | 0,916666667 |
| RSPH3 | -3,183945544 | 0,75 | 0, 909090909 |
| GMPPA | -1,842804757 | 0,9 | 0,916666667 |
| TUBB2A | 2,58003022 | 0,75 | 1 |
| LRRC46 | -2,001144581 | 0,818181818 | 0,909090909 |
| UBE2N | 0,851922695 | 0,75 | 1 |
| IFT46 | -0,99177675 | 0,875 | 0,818181818 |
| MYCBP | 0,108030925 | 0,833333333 | 0,916666667 |
| PLIN3 | 0,941416176 | 0,75 | 0,75 |
| LZTFL1 | 0,868566248 | 0,833333333 | 0,75 |
| KRT75 | 0,233355944 | 0,916666667 | 0,833333333 |
| HSPA9 | -0,750618424 | 0,75 | 0,833333333 |
| ATP5F1B | -0,857689194 | 0,75 | 0,75 |
| FABP5 | 1,801293754 | 0,916666667 | 0,833333333 |

### OLINK Nasal Allergic patient vs. Healthy - baseline (not according to the invention)

| **Marker Protein / Gene Name** | **Threshold** | **Specificity** | **Sensitivity** |
|---|---|---|---|
| SIRT2 | 10,18929849 | 0,833333333 | 0,846153846 |
| IRAK4 | 7,864668492 | 0,833333333 | 0,846153846 |
| DAPP1 | 10,19927349 | 0,833333333 | 0,846153846 |
| ICA1 | 10,23322875 | 0,833333333 | 0,769230769 |
| SH2B3 | 5,018939426 | 0,833333333 | 0,846153846 |
| PIK3AP1 | 8,393013222 | 0,833333333 | 0,769230769 |
| ZBTB16 | 6,3419575 | 0,833333333 | 0,769230769 |
| ZBTB17 | 6,041091941 | 0,75 | 0,846153846 |
| DCTN1 | 9,257664148 | 0,833333333 | 0,846153846 |
| TANK | 9,421788222 | 0,833333333 | 0,846153846 |
| BIRC2 | 4,413808492 | 0,833333333 | 0,846153846 |
| IRF9 | 10,20290571 | 0,833333333 | 0,769230769 |
| ARHGEF12 | 8,749263781 | 0,833333333 | 0,846153846 |
| PAK4 | 7,103735707 | 0,833333333 | 0,769230769 |
| PPP1R9B | 6,7072075 | 0,75 | 0,769230769 |
| DGKZ | 2,180722726 | 0,75 | 0,846153846 |
| GCNT1 | 1,645389295 | 0,75 | 1 |
| IL22RA1 | 3,225334543 | 0,75 | 0,769230769 |
| CTSO | 3,232848547 | 0,75 | 0,769230769 |
| METRNL | 2,548098492 | 0,75 | 0,846153846 |

### Example 2 - Examples for Immunologic Biomarker Tests

### Sandwich-ELISA, Two-Side-Binding Assay, Quantification-ELISA

An antibody against the biomarker is used as a capture antibody and coated to a microtiter plate (MTP). Free binding space on the MTP is blocked by an appropriate reagent and in the next step the sample consisting of serum/plasma or the supernatant from nasal swabs is applied. When the desired biomarker is present in the fluid it is captured by the antibody attached to the solid phase. In the next step an antibody with specificity against another epitope of the biomarker then the capture antibody is applied. This antibody is labeled with an enzyme like Alkaline phosphatase (AP) or Horse-Reddish-Peroxidase (HRP) or with biotin. In case of biotin-labeling another step is necessary using AP- or HRP-conjugated Streptavidin. After the enzyme labeled components the appropriate substrate is followed. The enzymes catalyze a substrate reaction which is associated with the formation of a color which can be measured at a defined wave length using a photometer (microplate-reader).

### Inhibition-Assay

Purified biomarker protein is attached to the solid phase of an MTP. The fluid of unknown biomarker presence/ concentration is incubated in serial dilutions with an enzyme-labeled antibody or antiserum against this biomarker. In a standard curve, serial dilutions of pure biomarker are incubated with the same enzyme-labeled antibody or antiserum, followed by the reaction leading to a colored substrate which can be measured by a photometer. The unlabeled antigen/biomarker in solution is competing with the solid phase bound antigen/ biomarker and the degree of inhibition is a measure for the concentration of the antigen/ biomarker.

### Dipstick! immune-stick/ biomarker-stick

Biomarker-specific antibody is bound to a stick of special paper. Such a stick can be prepared by using several antibodies against different biomarkers in sequence and can be offered as a prepared stick ready to use. The stick will be incubated in a small tube containing plasma or nasal swab supernatant followed by incubation with an enzyme-labeled soluble antibody against the biomarker with different epitope specificity then the one attached to the solid phase. After a washing step the stick will be incubated with the substrate of the conjugated enzyme. It might be possible to incubate the biomarker containing fluid, enzyme-labeled antibody and substrate in one step to limit the performance time of the assay. After stopping the reaction, the intensity of the color is compared visually with a standard card of the biomarker. Such an assay allows a rather rough calculation of the biomarker concentration but might be done without the need to purchase expensive laboratory equipment.

### Evanescent Technology (EVA-Technology)

The evanescence technology is a fluorescence-based biosensor technology exploiting the optical phenomenon of total internal reflection to create an evanescent field which is used to selectively excite bound fluorophores localized within the penetration depth at the bottom of each well of the sensor chip. Fluorescent molecules present in the sample liquid do not receive light and hence do not emit fluorescence. As a result, the binding can be detected directly without the need to remove the sample liquid (no washing). The assays are based on the biochemistry of an ELISA, but unlike ELISA can be completed within 10 minutes. In the format of a two-site binding assay for the quantitative determination of the biomarker, one antibody type is immobilized on the sensor surface of a well, which also contains a fluorophore-conjugated antibody of different epitope specificity to the biomarker than the bound one. The biomarker in the patient sample binds to both, the antibodies on the surface of the well and the fluorescently labeled detection antibody-conjugate. The test result is time sensitive and must be immediately measured after the application of the sample to the wells. Only Biomarker-positive samples bind to both the well surface and to the conjugated detection antibody and give a positive fluorescent signal. Advantage of the EVA-test is small sample volume (~20 µL), results in few minutes (~10 min), easy-to use - just one pipetting step, high sensitivity.

The assay can be also set up as an inhibition assay, where recombinant biomarker is immobilized on the sensor surface and a conjugated antibody against the biomarker is also already in the wells in dried form. The biomarker, present in the fluids, inhibit the binding of the conjugated antibody to the immobilized biomarker. There is an inverse correlation of the conjugate binding to the concentration of the biomarker in the sample fluid: The more biomarker in the sample the less binding of the conjugate occurs and allows conclusions about the concentration of the biomarker.

### Chip-technology

Other technology like EVA-test. Working under a similar principle like the sandwich-ELISA but with minimal volume of the sample and perhaps detection antibody and substrate reaction added at the same time to reduce the duration of the test leading to an immediate result.

### Fluorimetric Bead-Array

The assay uses 2 sets of beads (microspheres) of different size internally labeled with different intensities of a fluorescent dye. The two different bead sizes make it possible to distinguish multiple (e.g. 20) bead sets in one fluorescence channel, so the different bead sets distinguished by internal dye intensity and bead size allow the simultaneous quantification of e.g. 20 analytes (biomarkers) in a single small volume sample using the same principle as an ELISA.

The beads with different intensities of the fluorescent dye are coated with specific antibodies to the desired different biomarkers to be detected in the multiplex system. A mixture of coated beads for each analyte to be measured is incubated with the samples or standard mixtures. The analytes present in the sample bind to the antibodies linked to the fluorescent beads. Simultaneously, a fluorescentconjugated antibody mixture is added, the specific antibodies bind to the analytes captured by the first antibodies. The fluorescent labeled antibodies which bound to the analyte emit a fluorescent signal which is detected by a flow cytometer.

### Lateral flow assay

The sample containing biomarker will be applied on the sample pad of a test stripe of porous paper or sintered polymer. After addition of a special fluid the sample diffuses through the test stripe due to capillary force (thin-layer chromatography). The sample is migrating with the fluid into a part of the stripe where dried immuneconjugates is located (conjugate pad). The fluid dissolves the immune-conjugate, which enables the binding to the antigen (biomarker) in the sample, when present. The fluid continues to migrate further to a capture pad, which contains immobilized antibody specific for another part of the antigen/ biomarker, which binds to the antibody and is thus immobilized, while the fluid continues to migrate. In this step the immobilized antigen/ biomarker is enriched and by conjugation of the immobilized antibody with an enzyme or fluorescens dye, it can be detected.

### RT-qPCR

Quantitative reverse transcription PCR (RT-qPCR) is used when the starting material is RNA. In this method, RNA is first transcribed into complementary DNA (cDNA) by reverse transcriptase from total RNA or messenger RNA (mRNA). The cDNA is then used as the template for the qPCR reaction. RT-qPCR is used in a variety of applications including gene expression analysis, RNAi validation, microarray validation, pathogen detection, genetic testing, and disease research.

### Luminex

Immunoassays based on the Luminex xMAP Technology (Multianalyte profiling) enable the simultaneous detection and quantification of multiple secreted proteins (cytokines, chemokines, growth factors etc.). This high-through-put technology leads to results comparable with an ELISA, but with higher efficacy, speed and a larger dynamic range.

The Open-architecture xMAP Technology enables multiplexing of biological tests (assays), reducing time, labor, and costs over traditional methods such as ELISA, western blotting, PCR, and traditional arrays. Systems using xMAP Technology perform discrete assays on the surface of color-coded beads known as microspheres, which are then read in a compact analyzer. Using multiple lasers or LEDs and high-speed digital-signal processors, the analyzer reads multiplex assay results by reporting the reactions occurring on each individual microsphere.

Due to robust multiplexing, xMAP Technology potentially delivers more data in less time than other bioassay products, with comparable results to ELISA and microarray and is thus less expensive than ELISA. The technology offers several other distinct advantages over traditional methods:

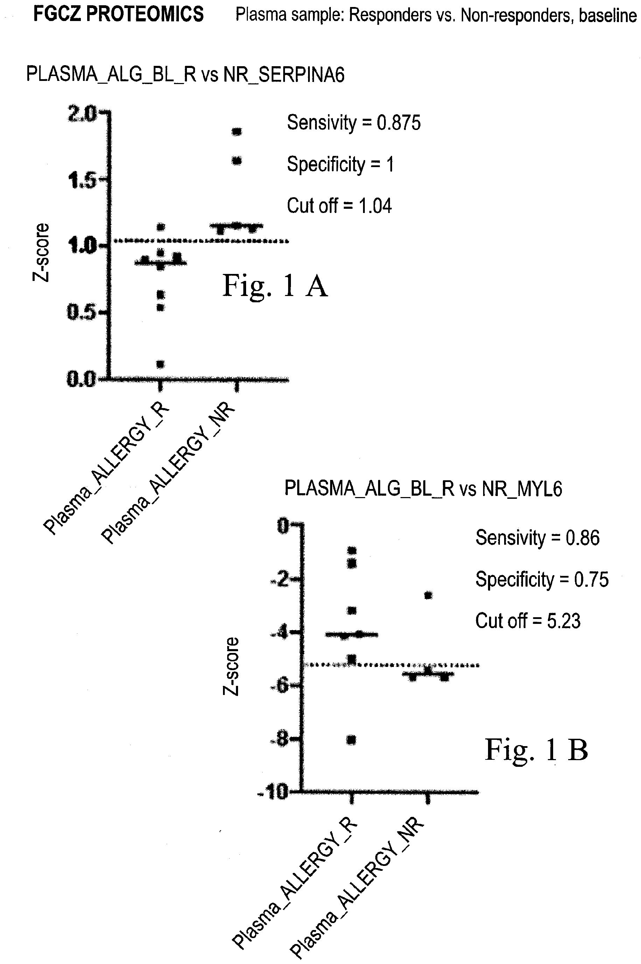

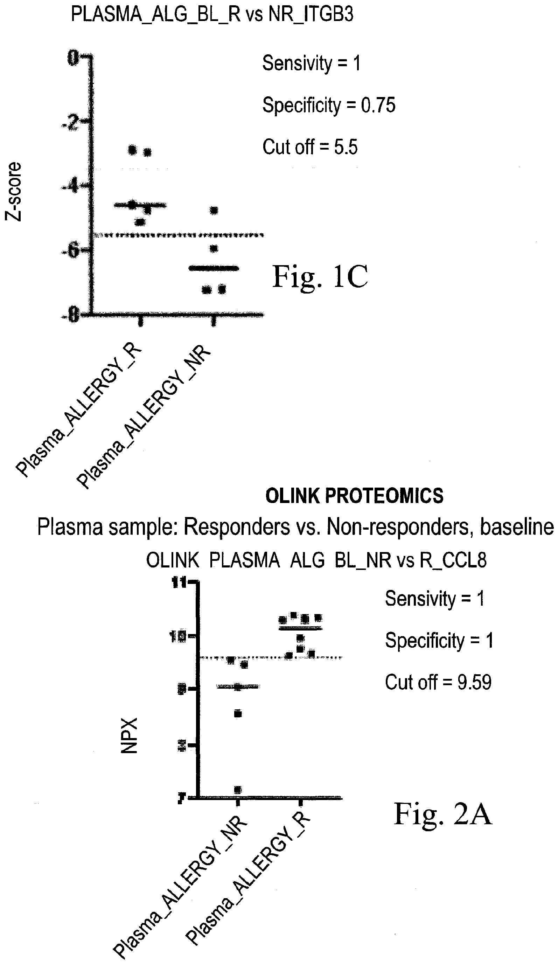

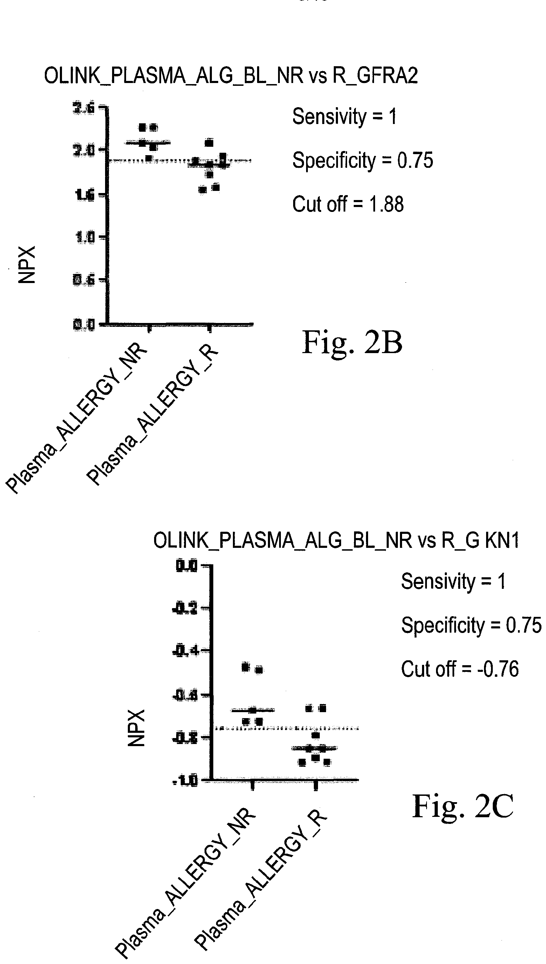

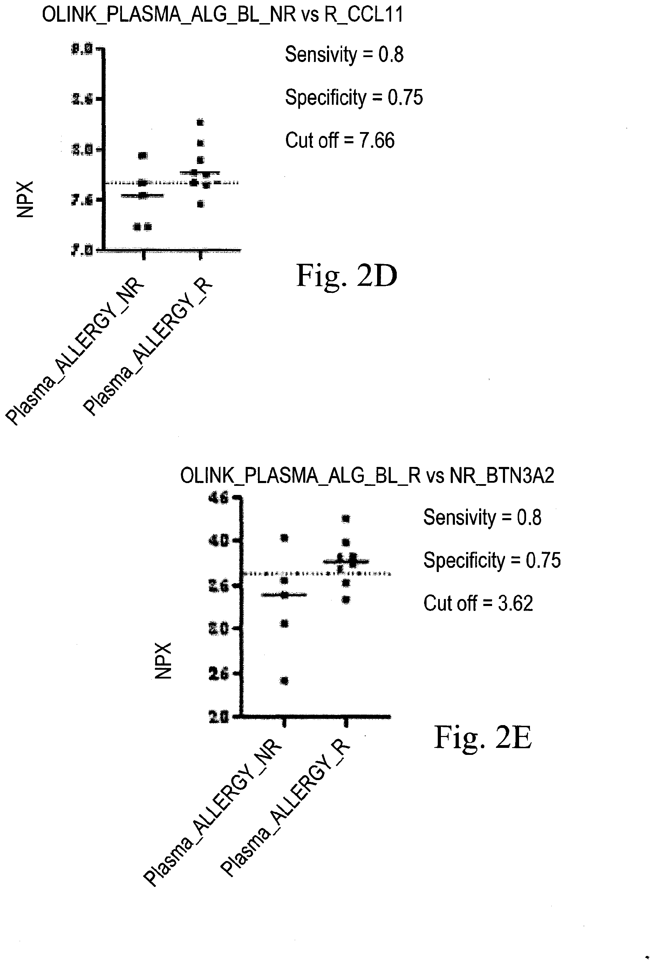

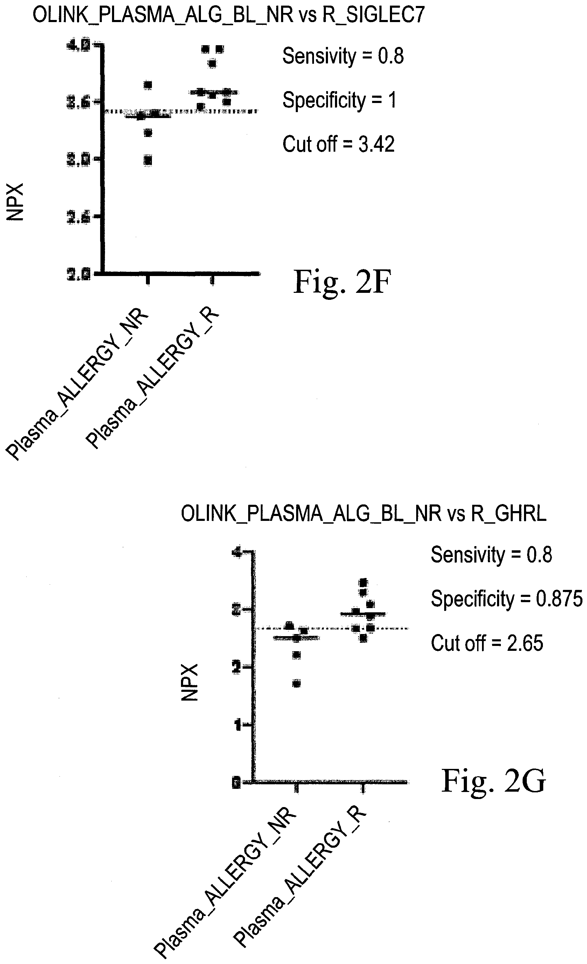

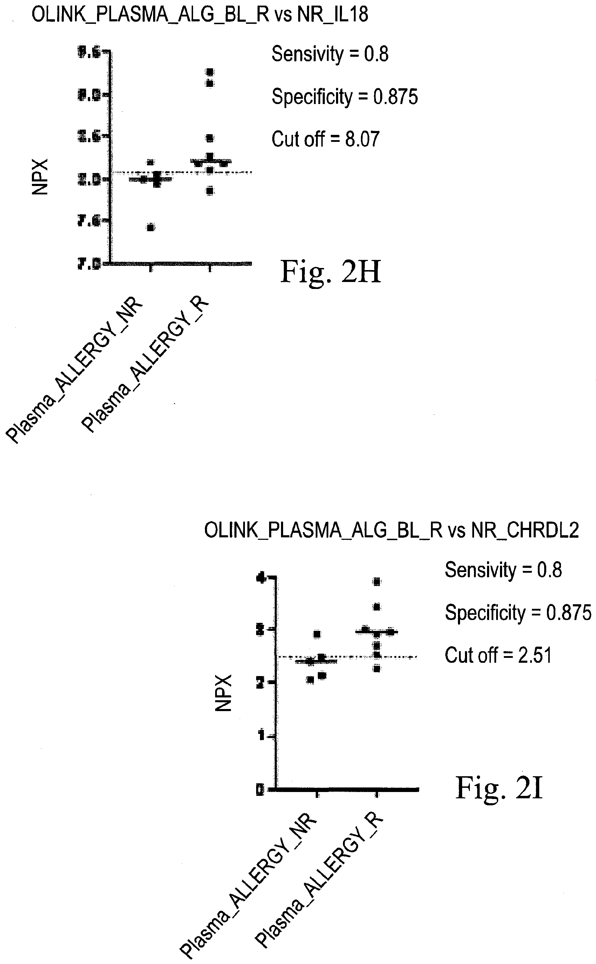

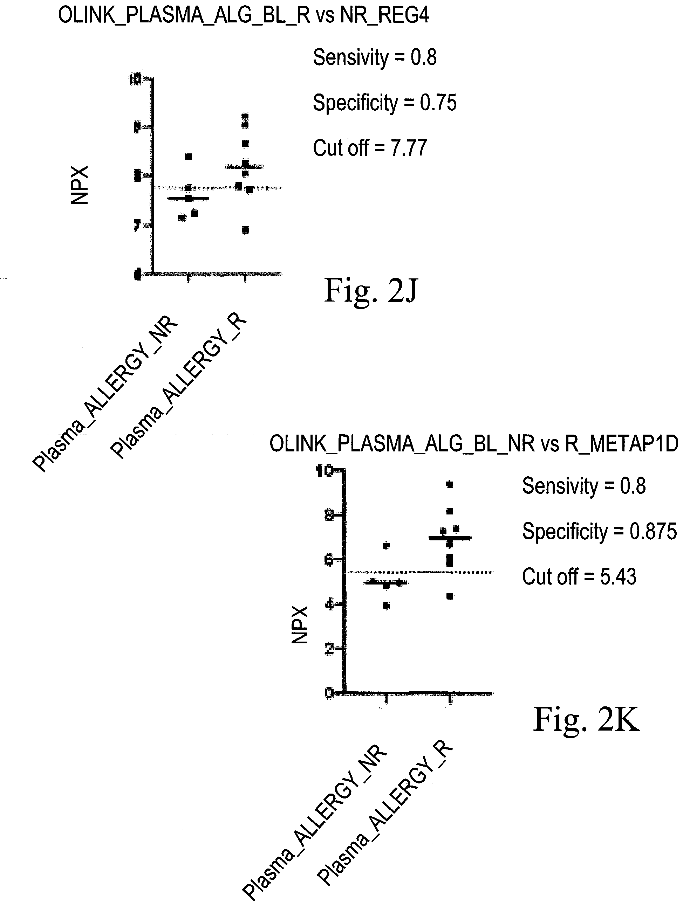

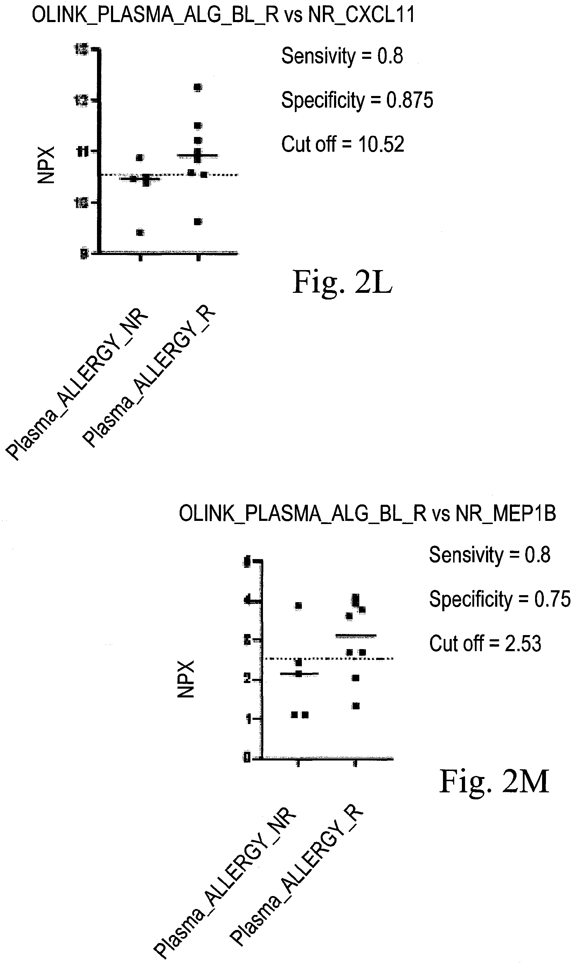

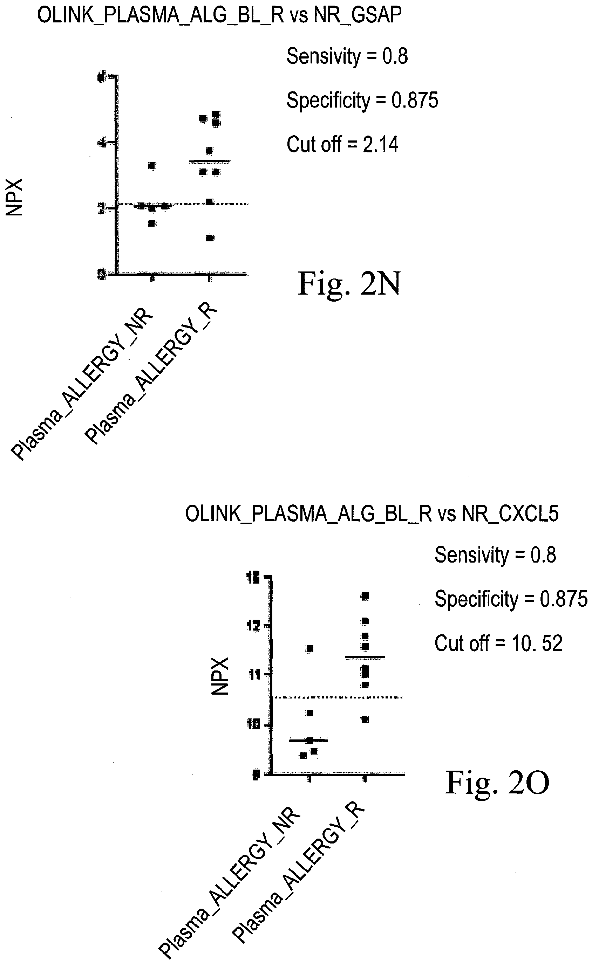

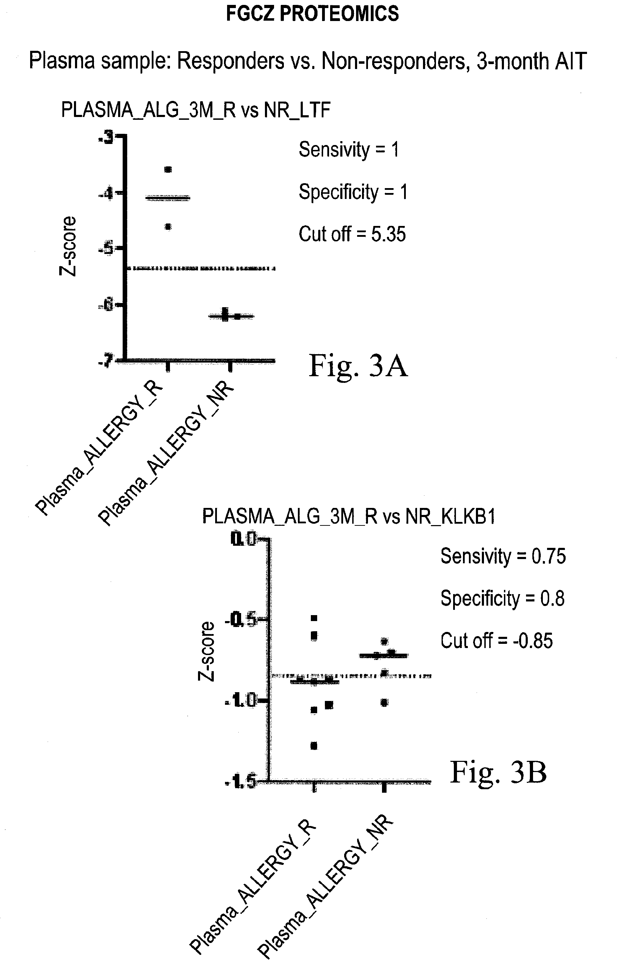

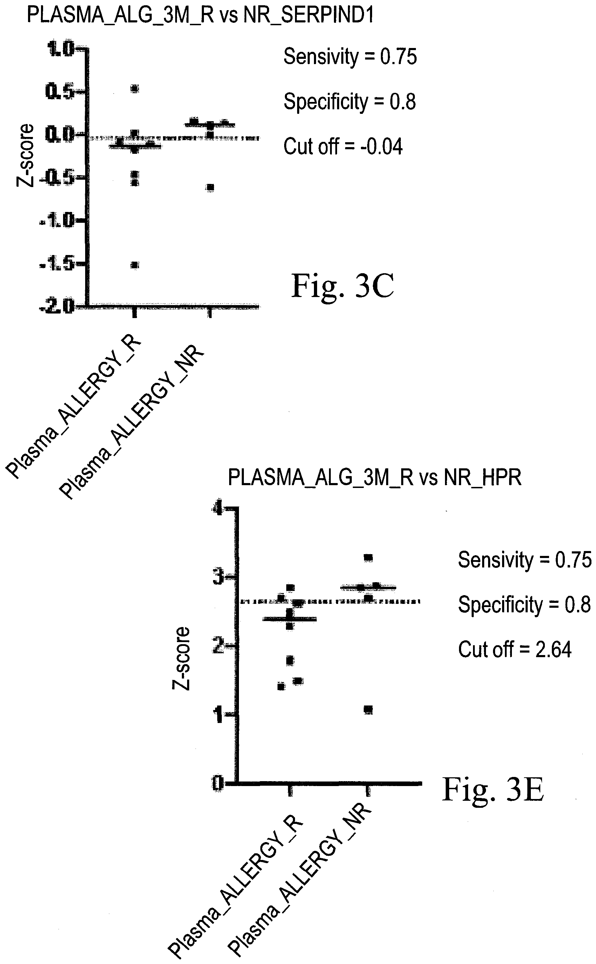

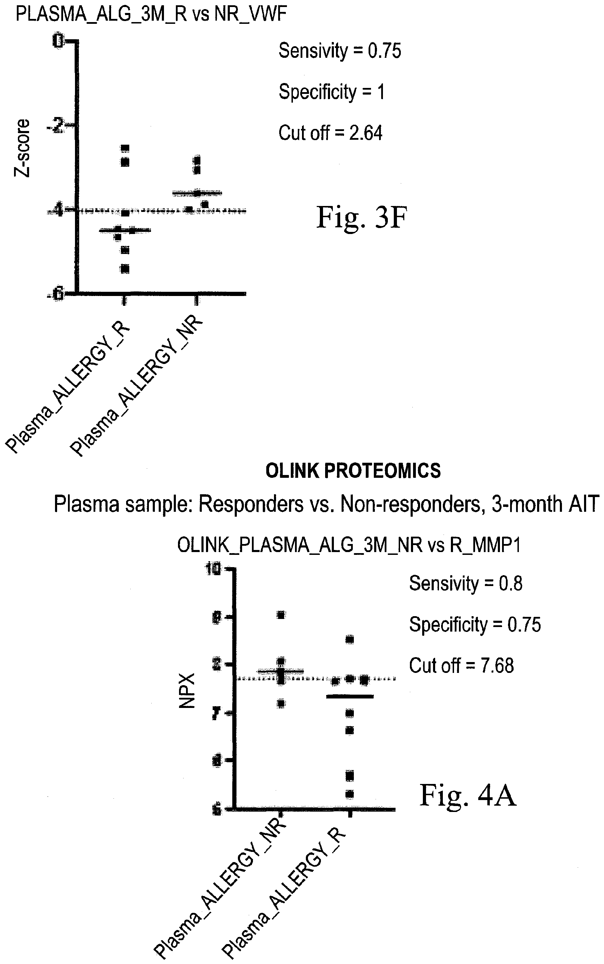

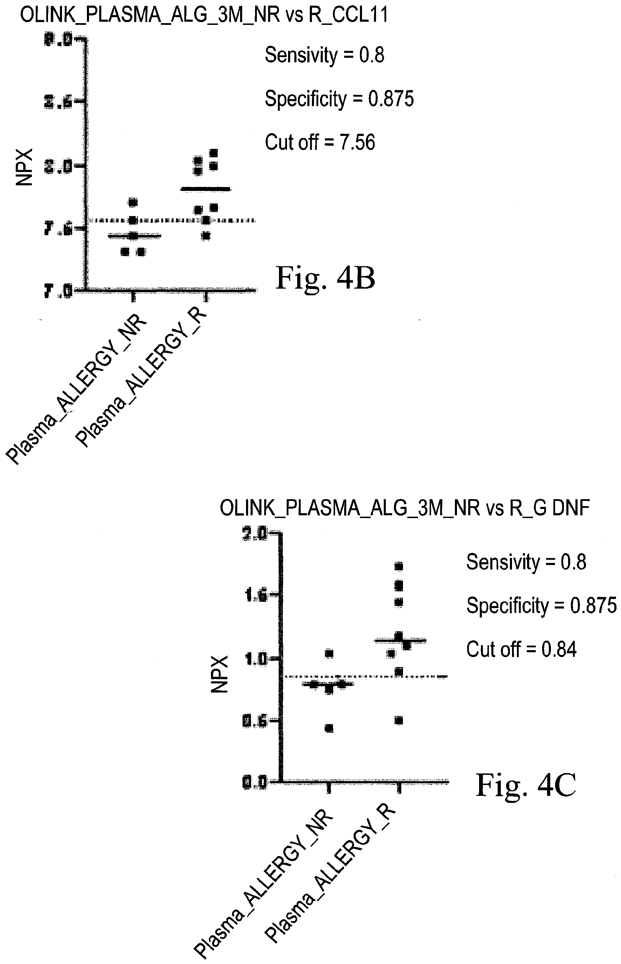

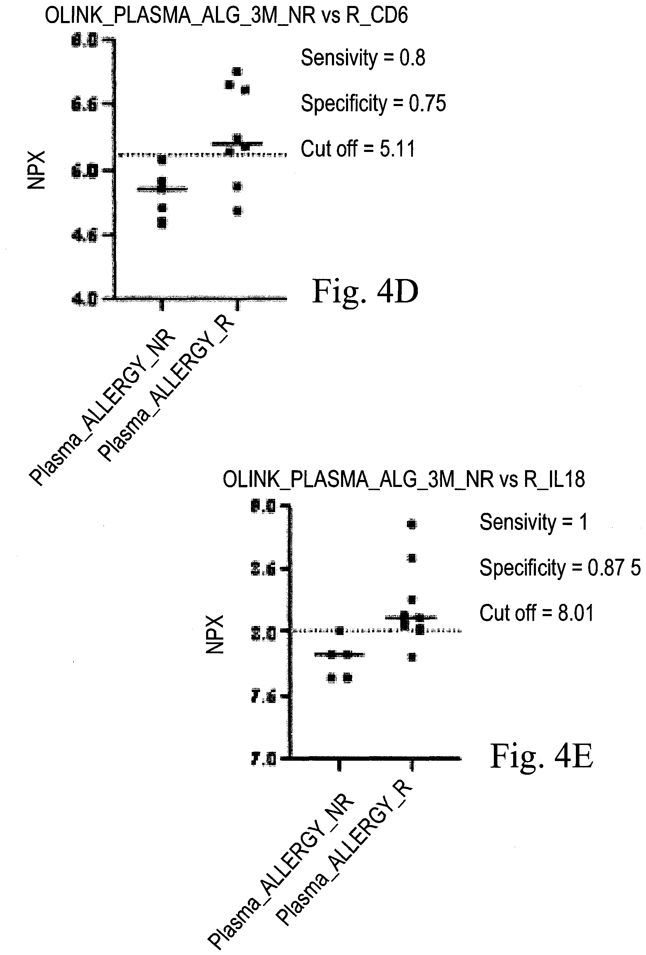

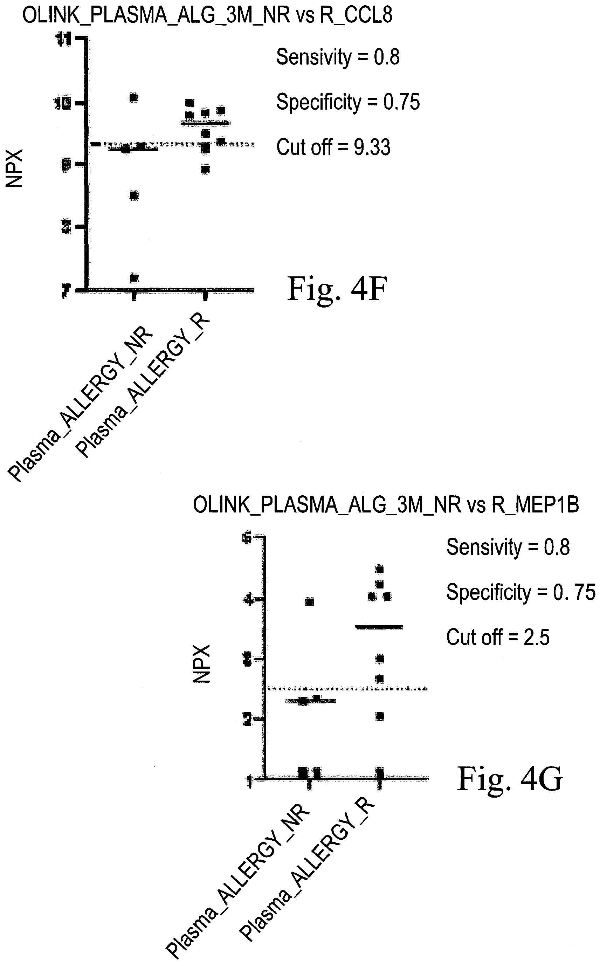

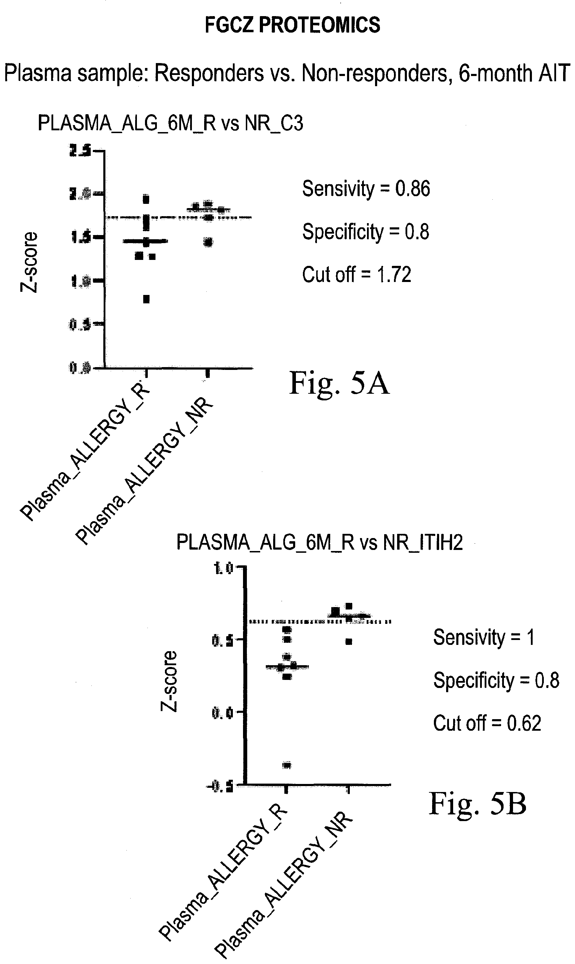

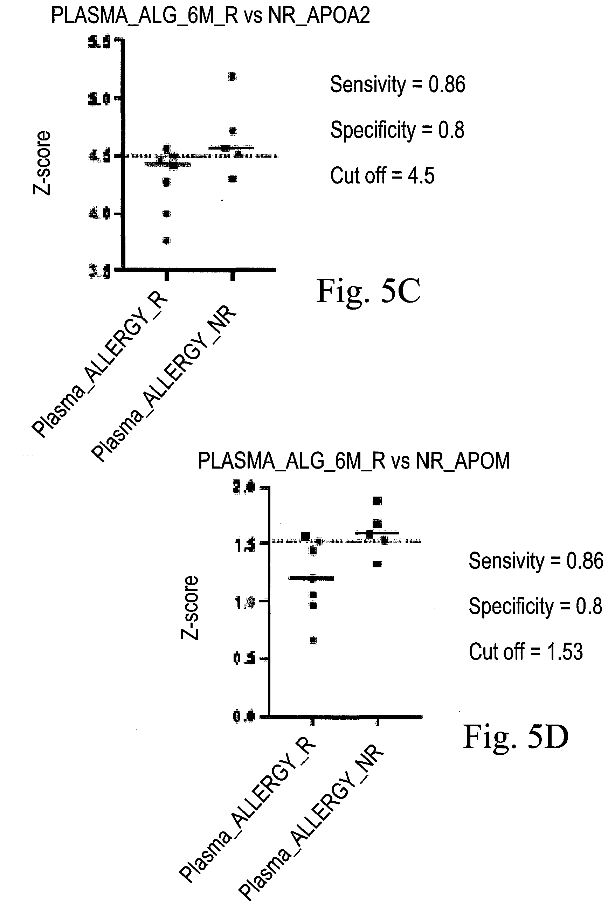

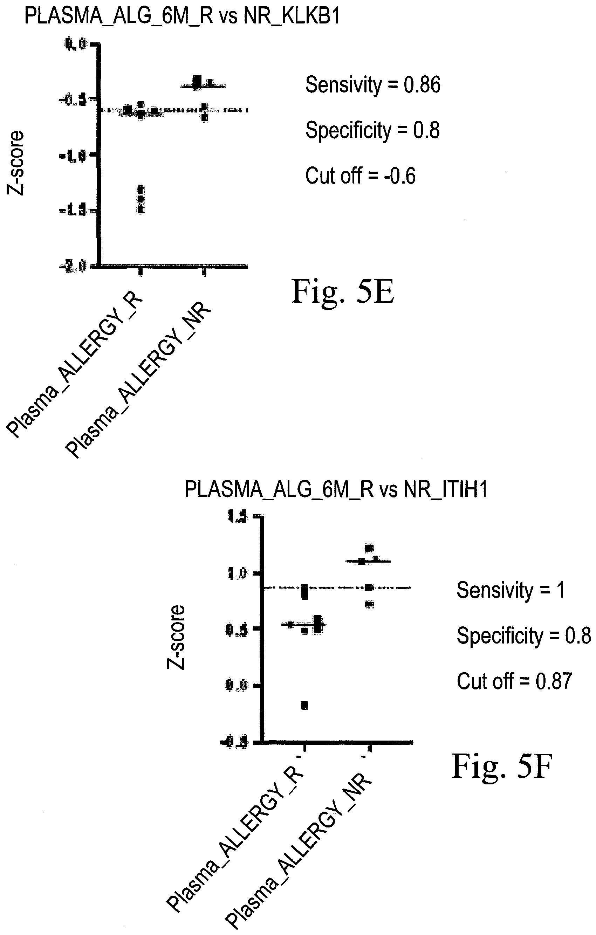

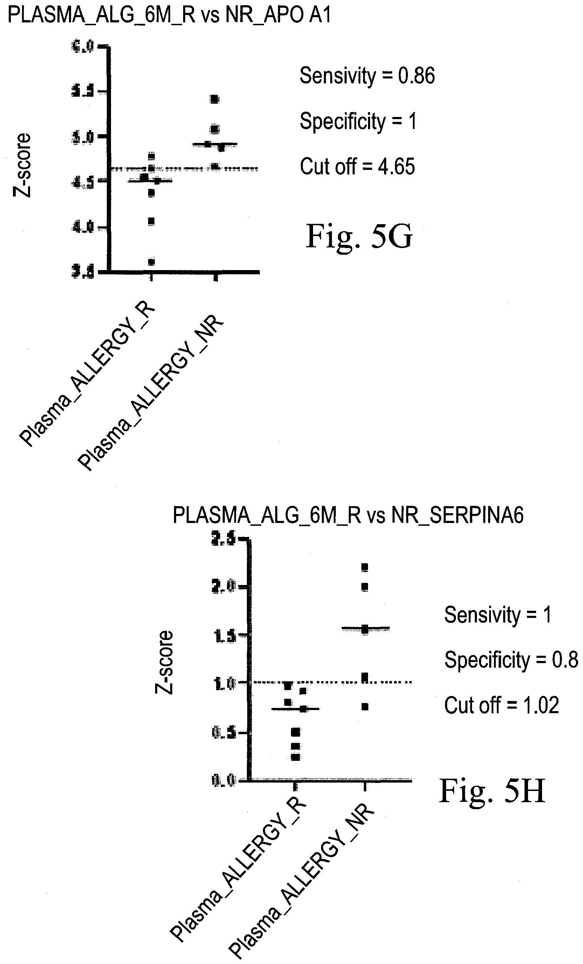

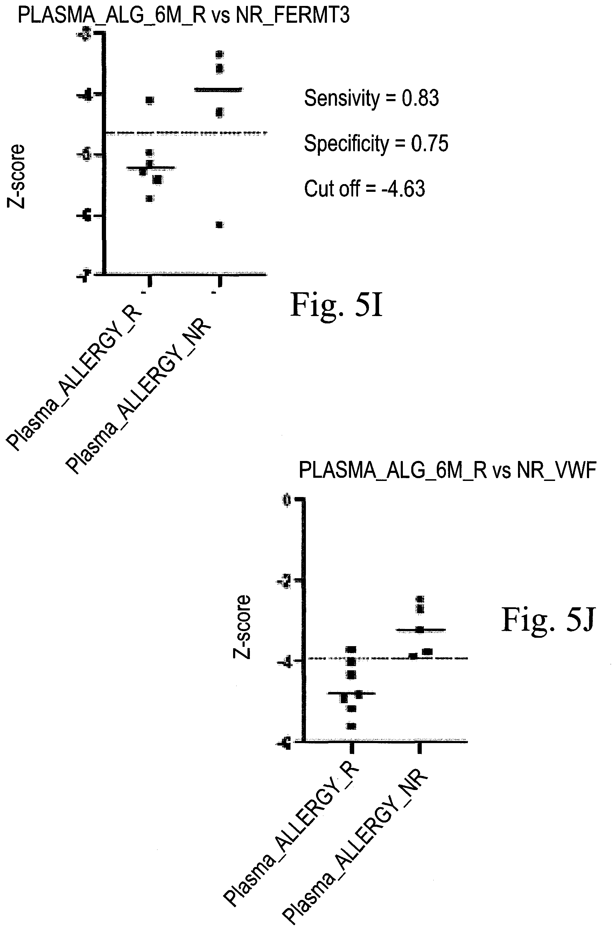

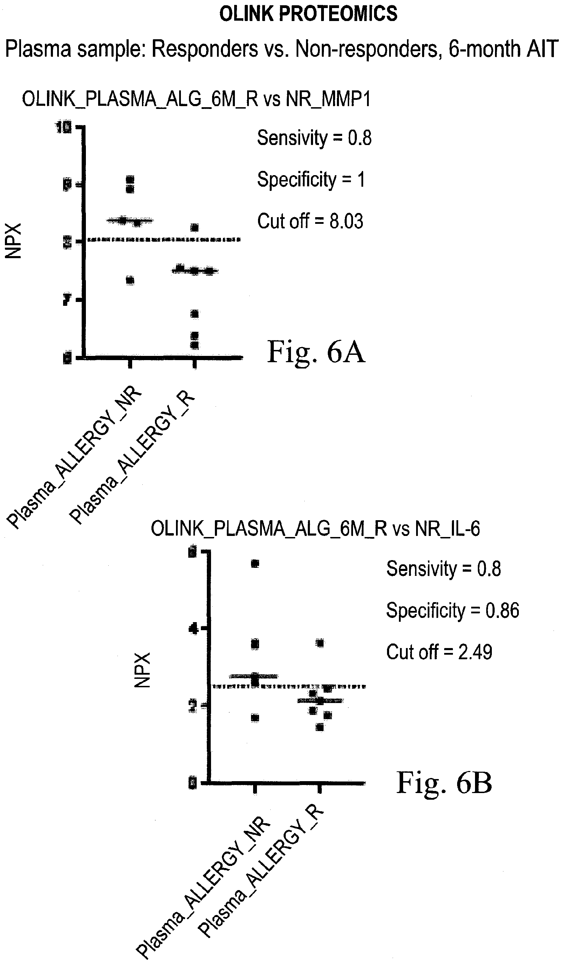

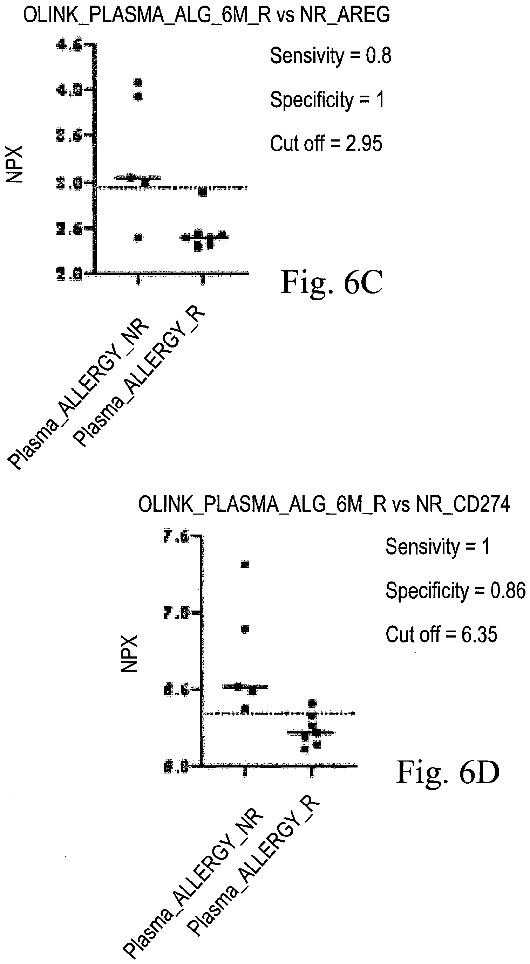

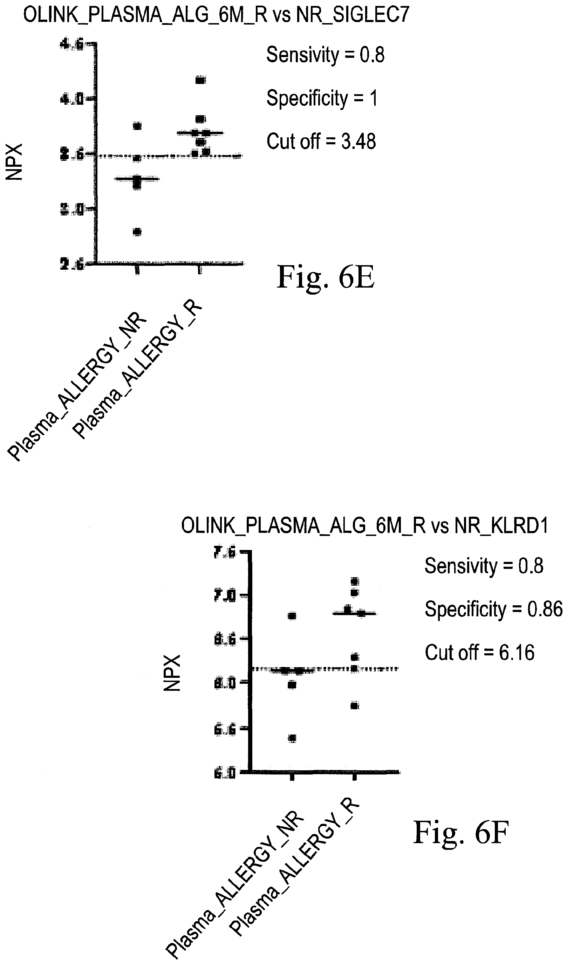

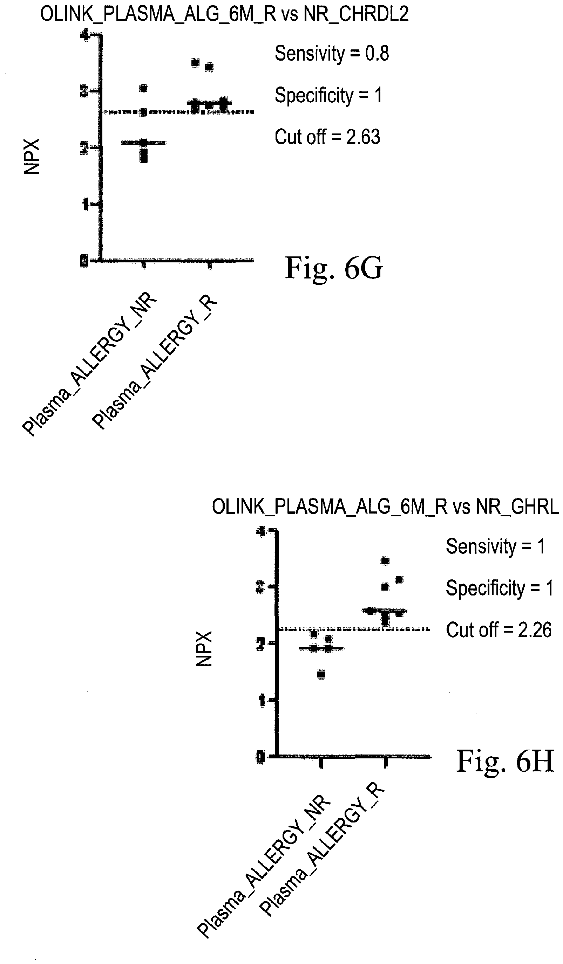

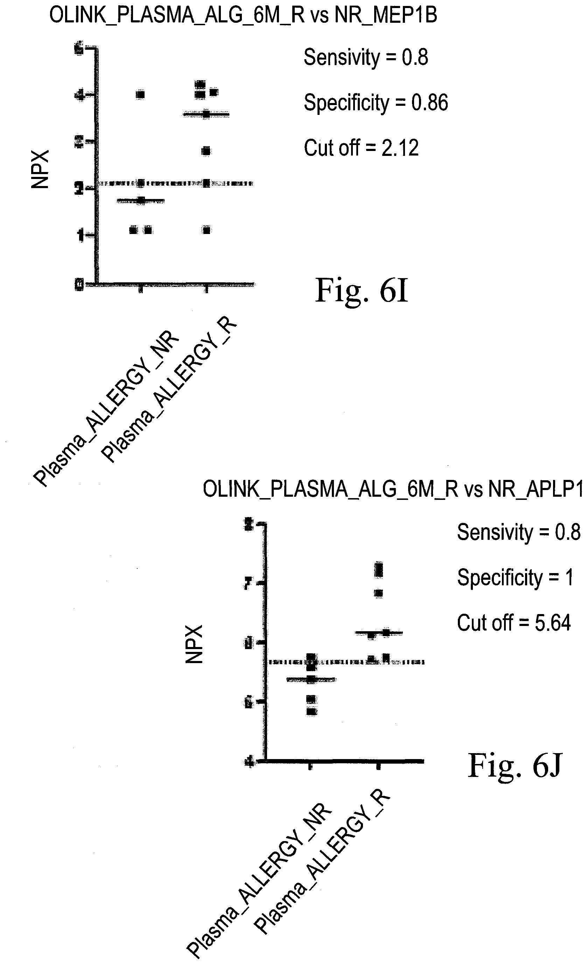

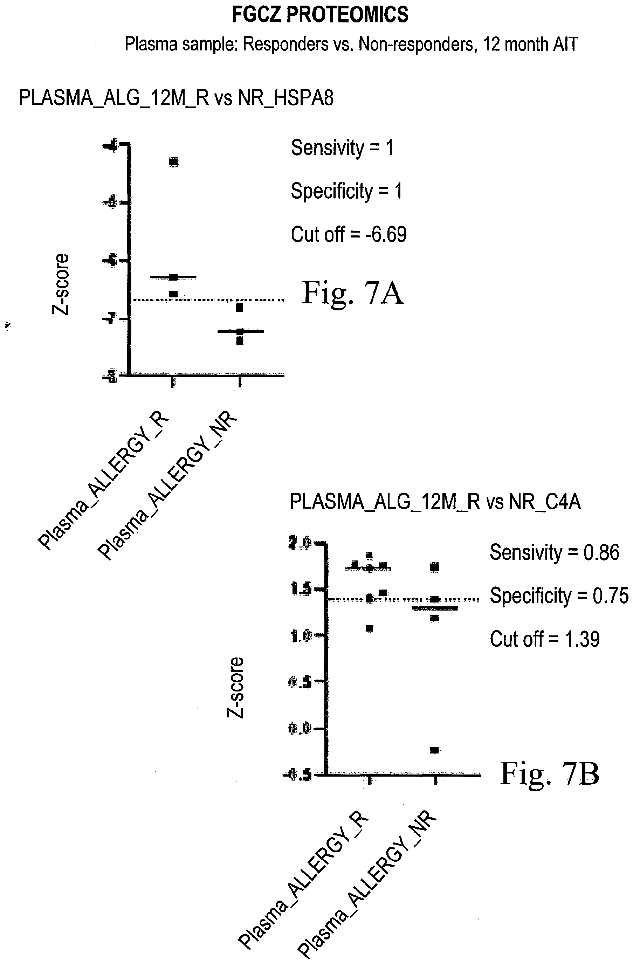

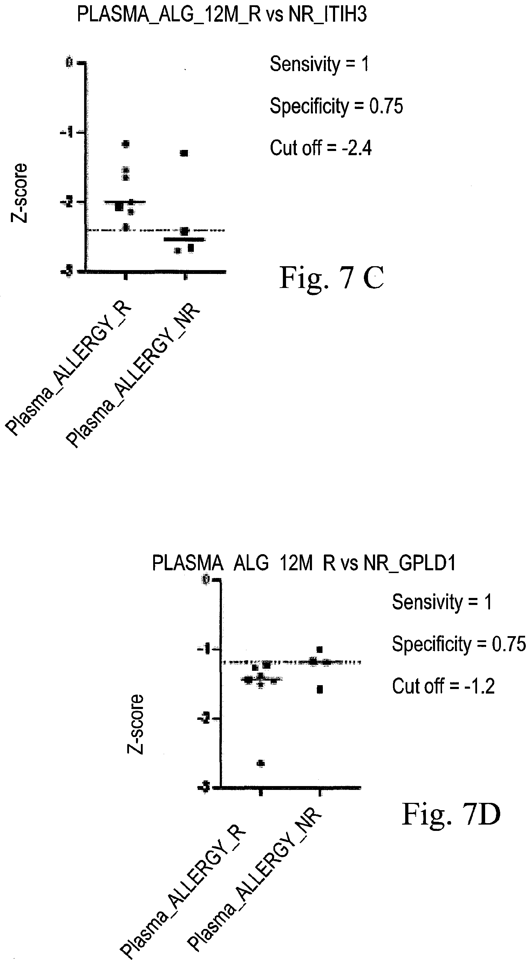

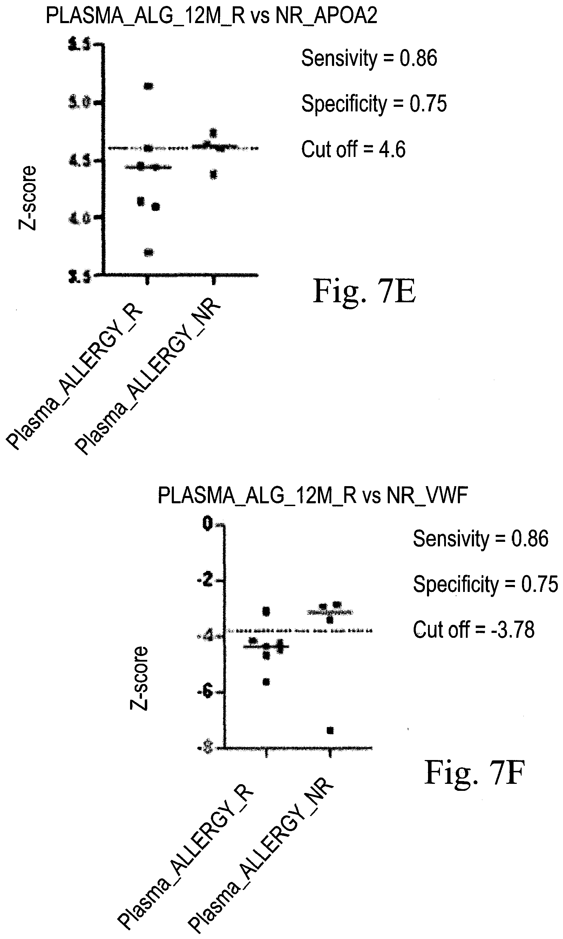

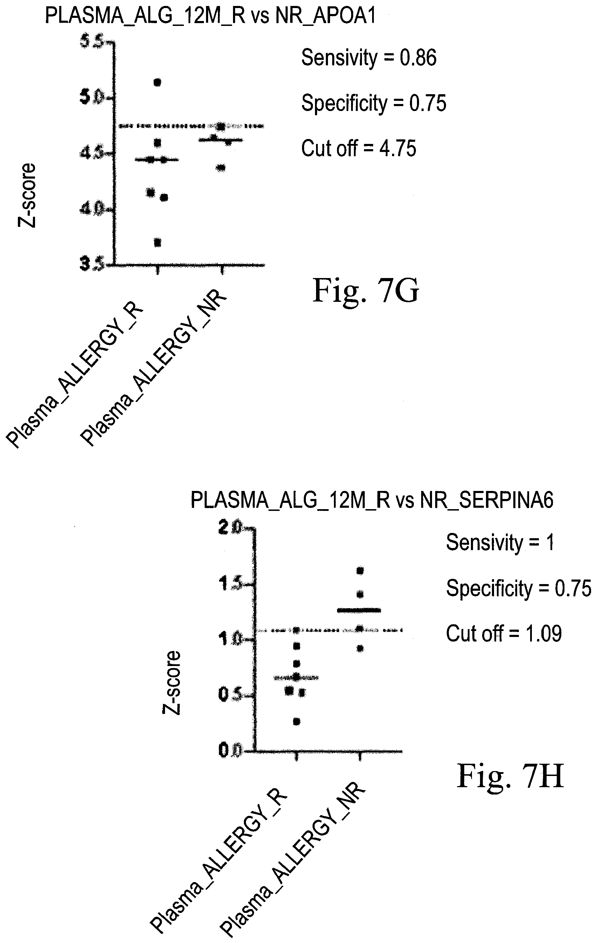

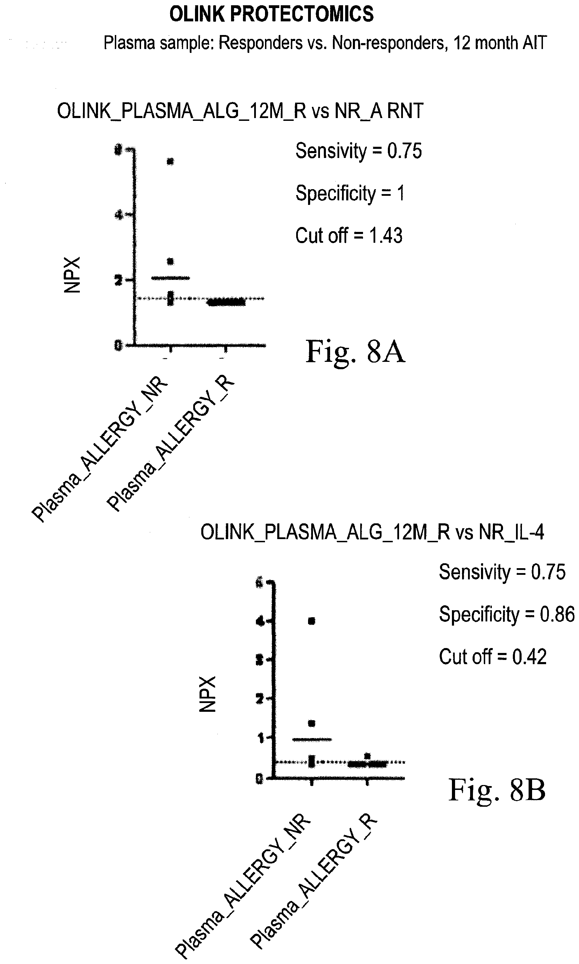

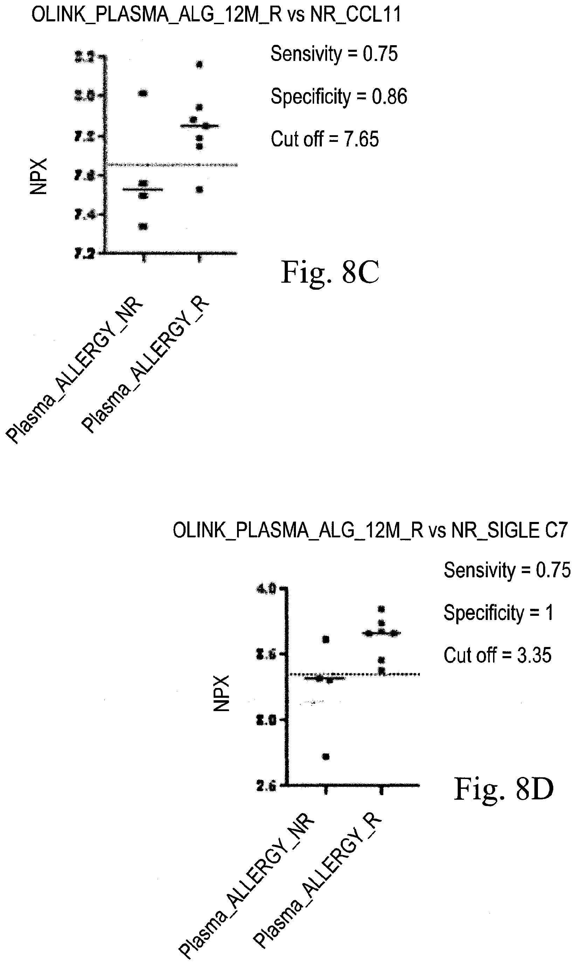

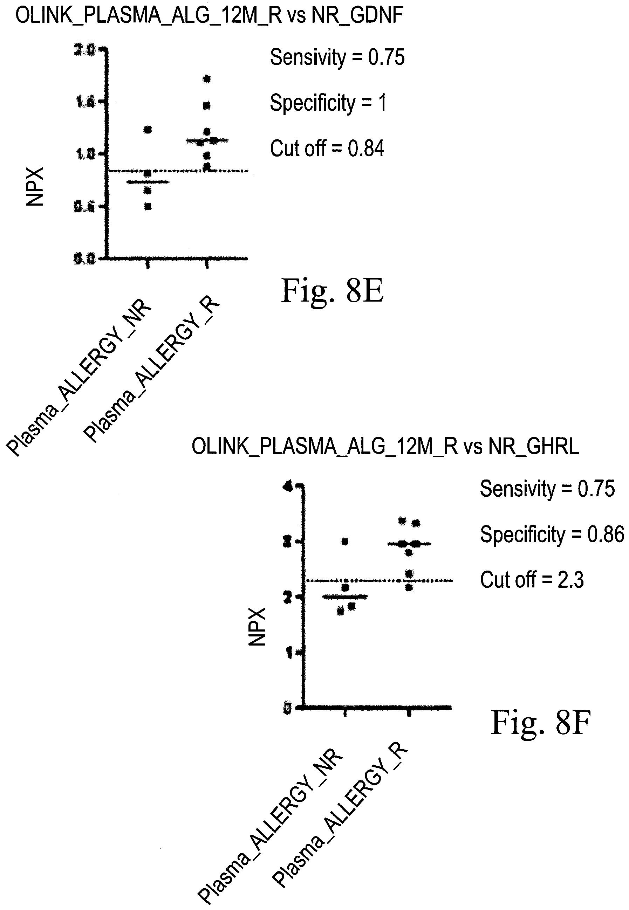

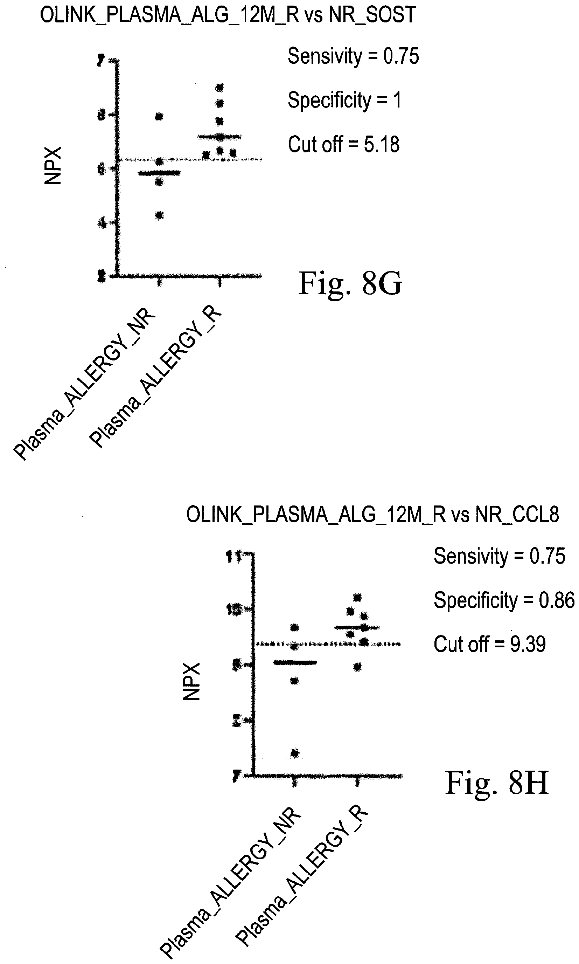

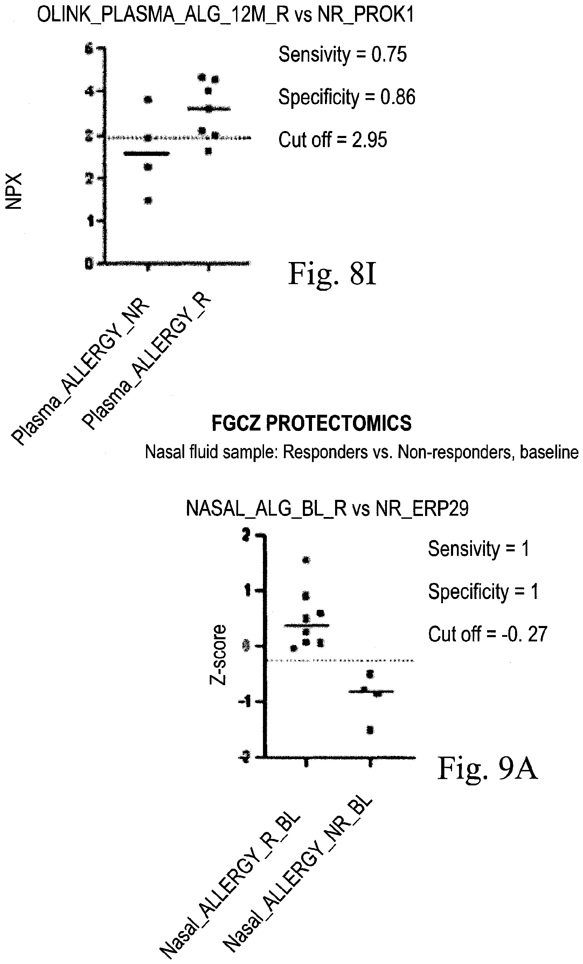

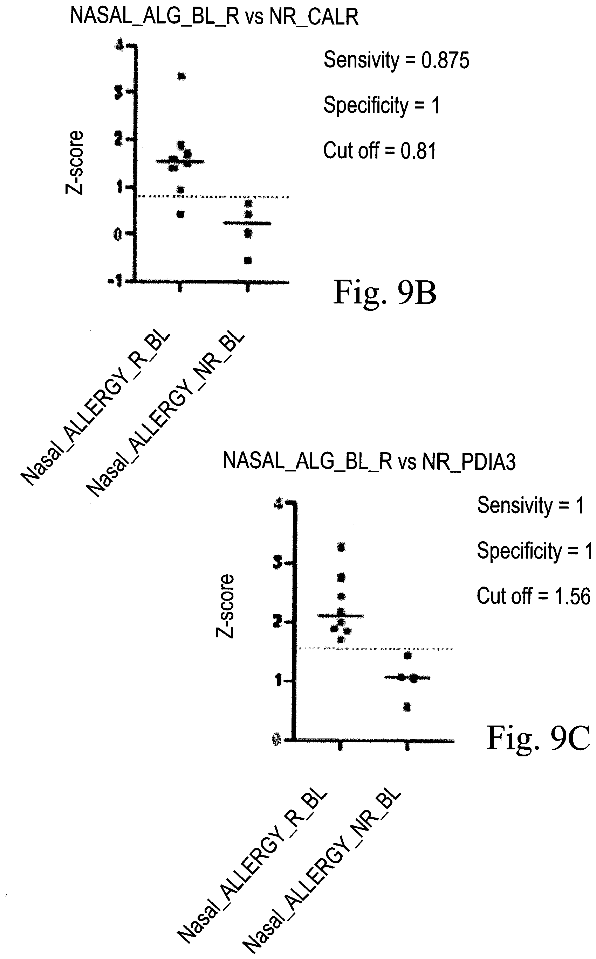

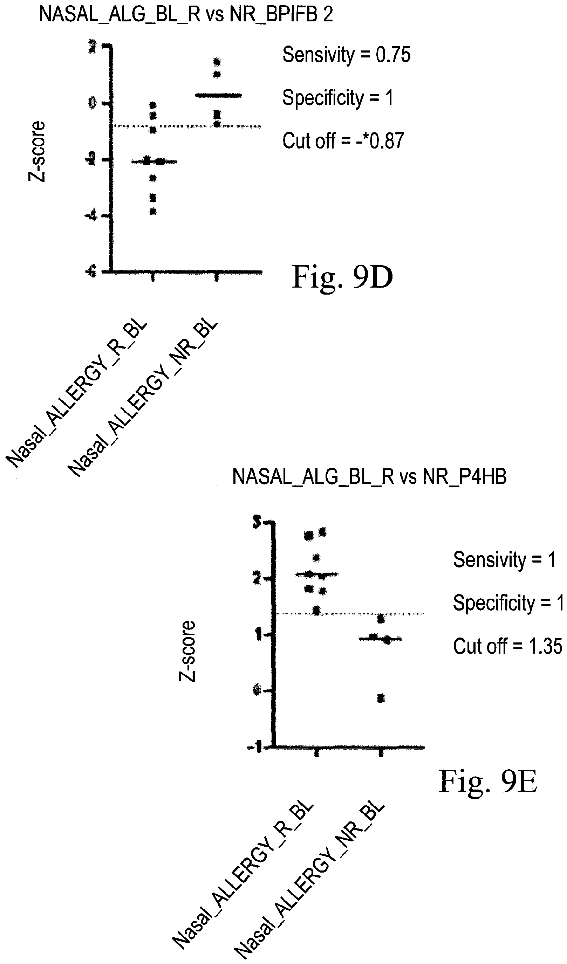

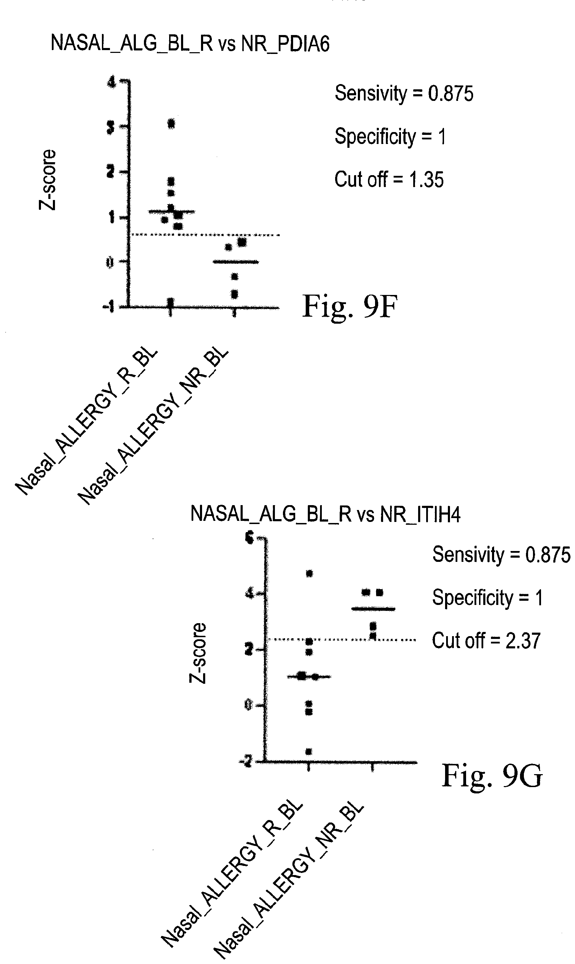

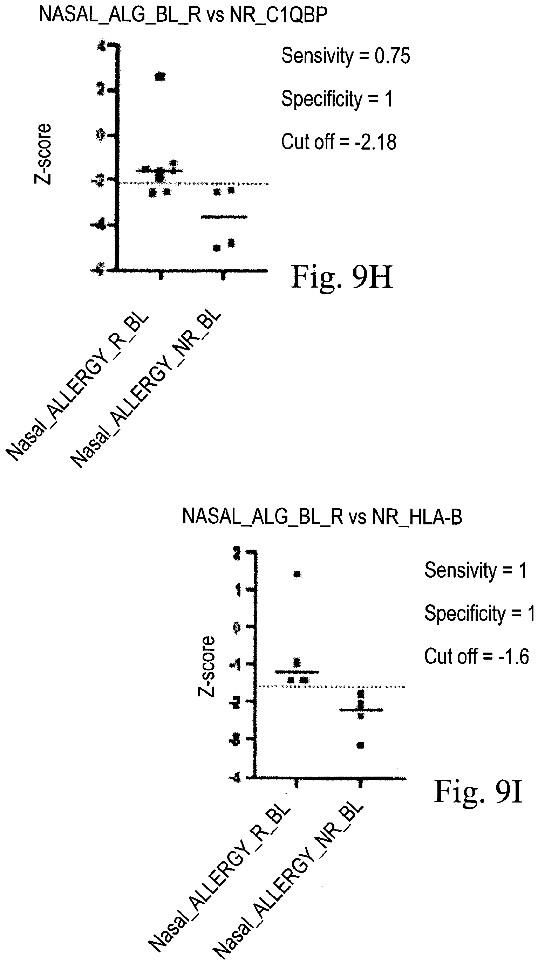

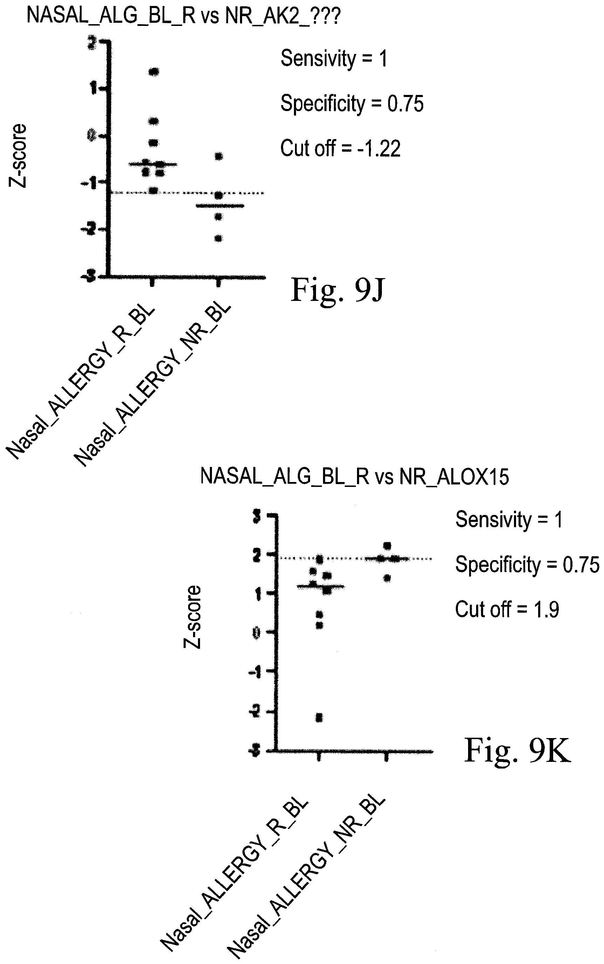

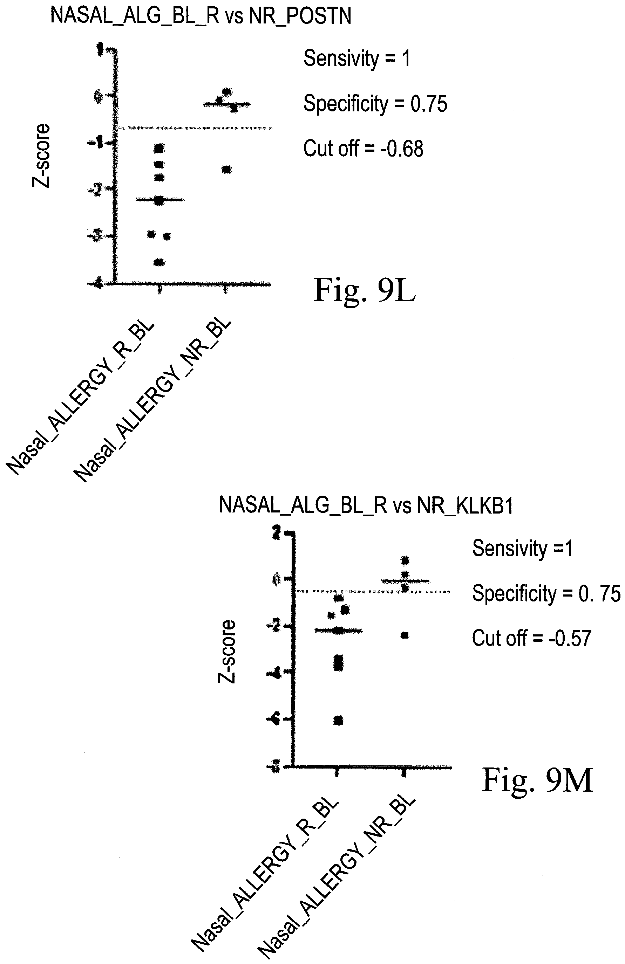

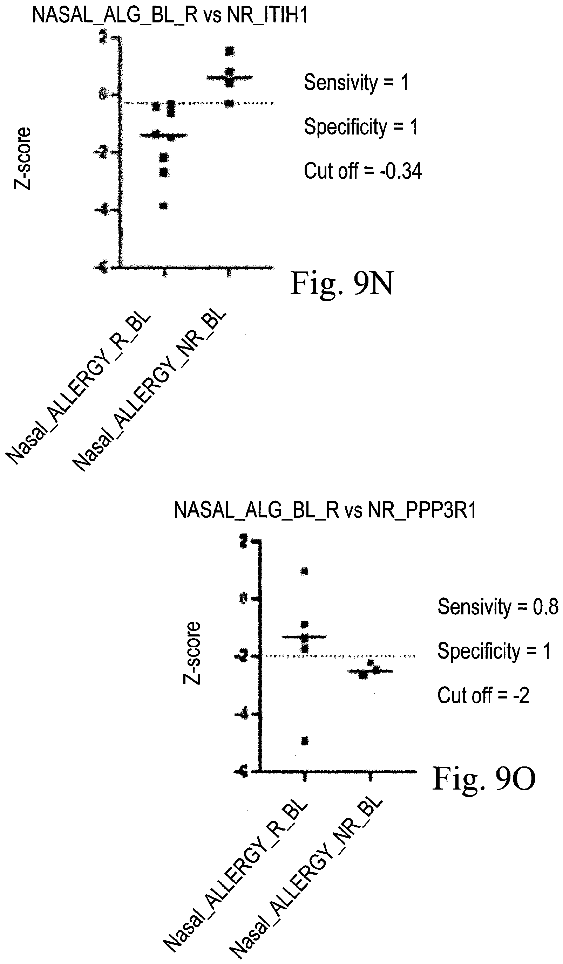

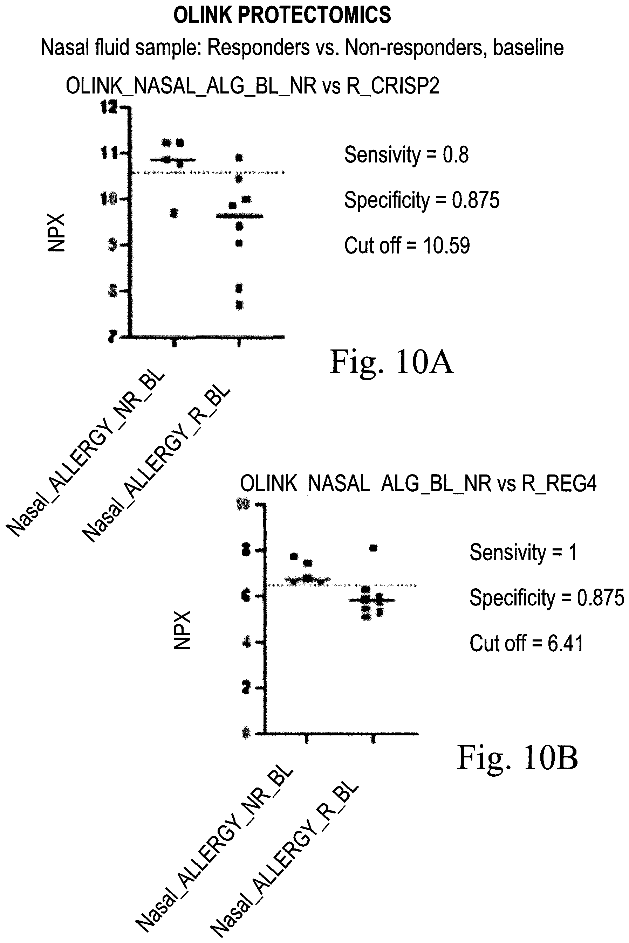

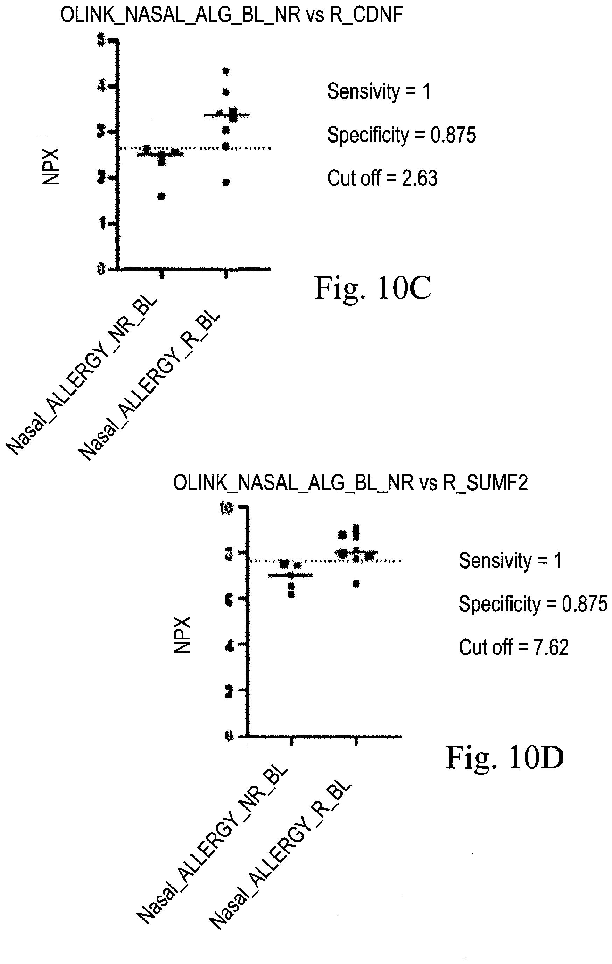

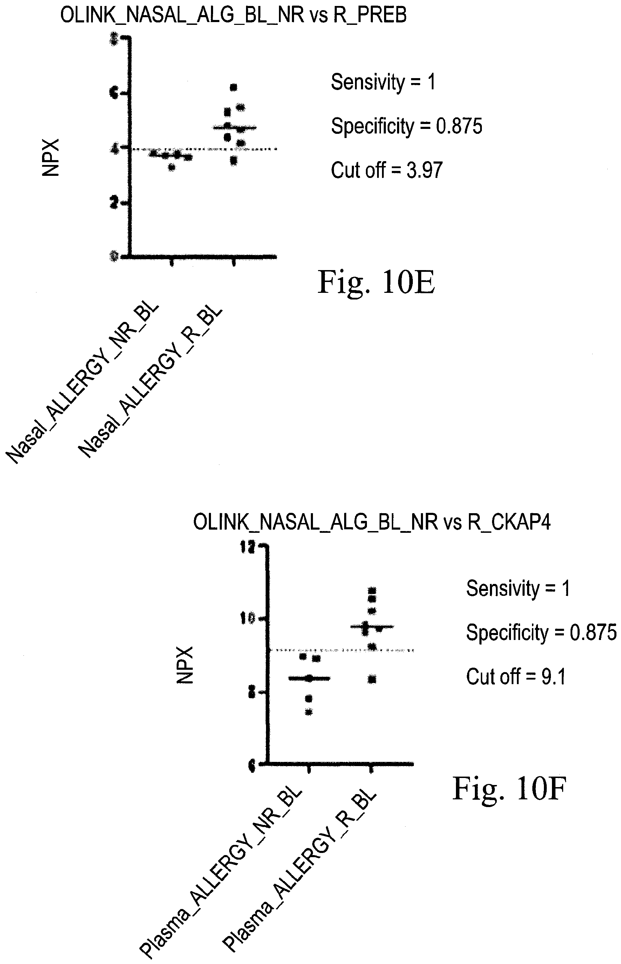

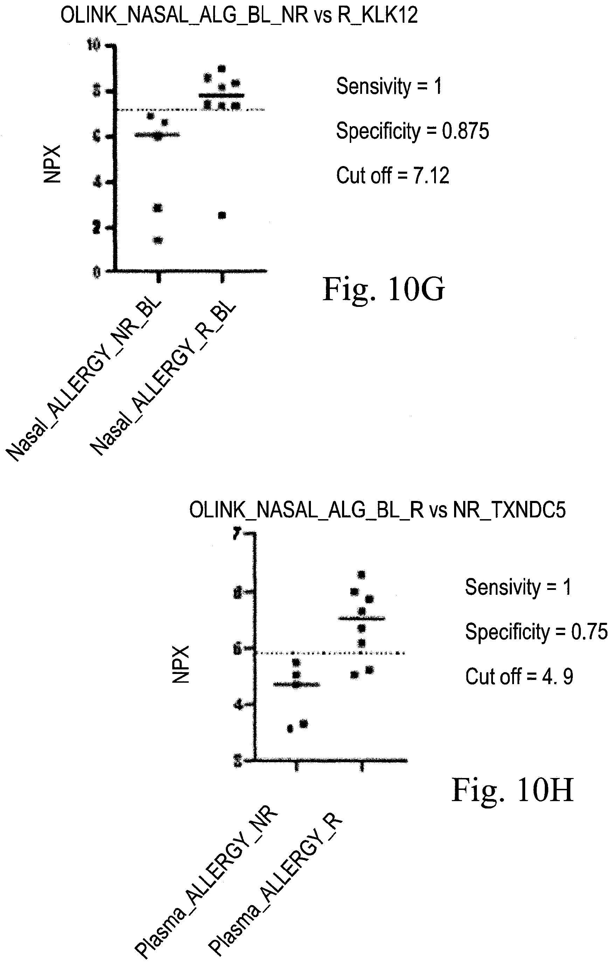

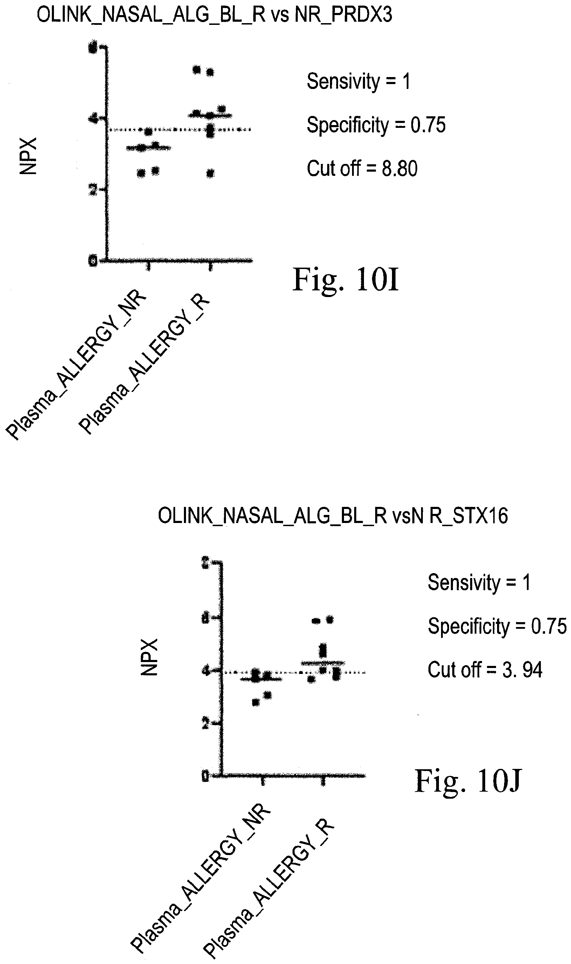

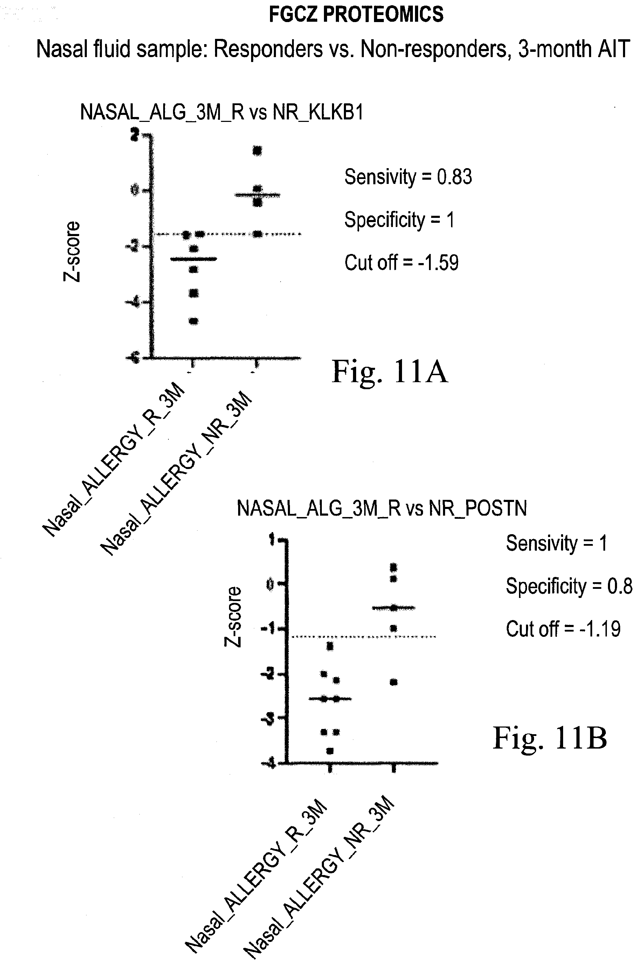

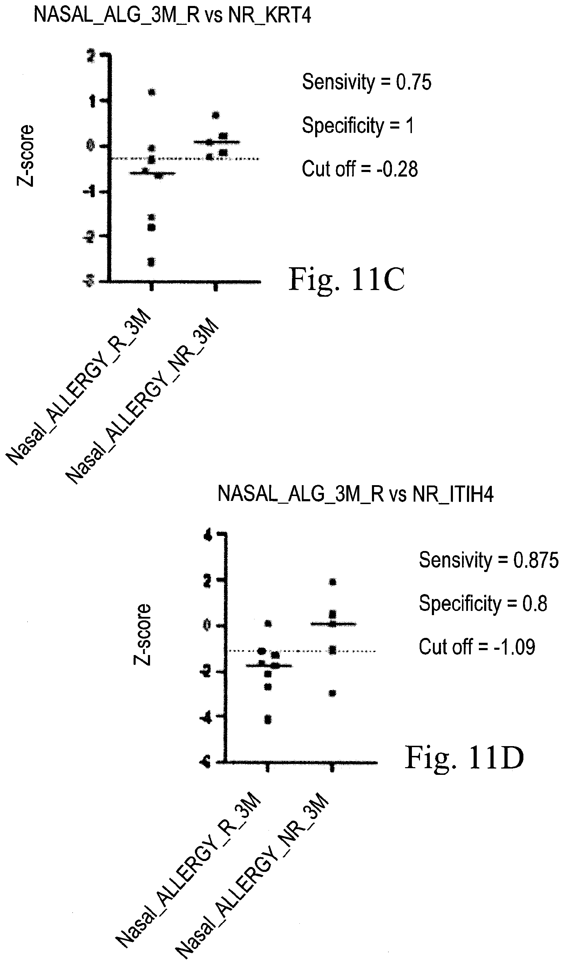

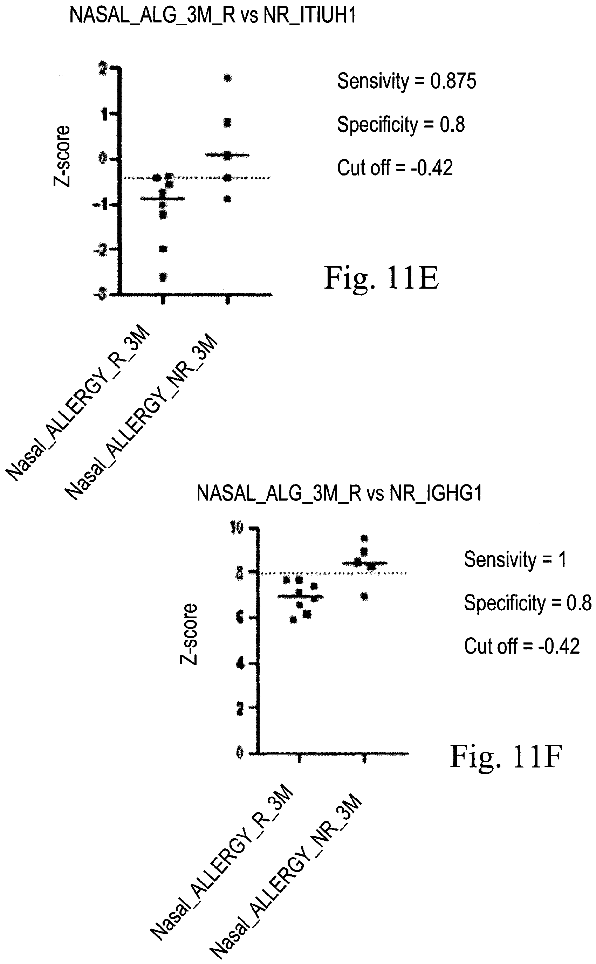

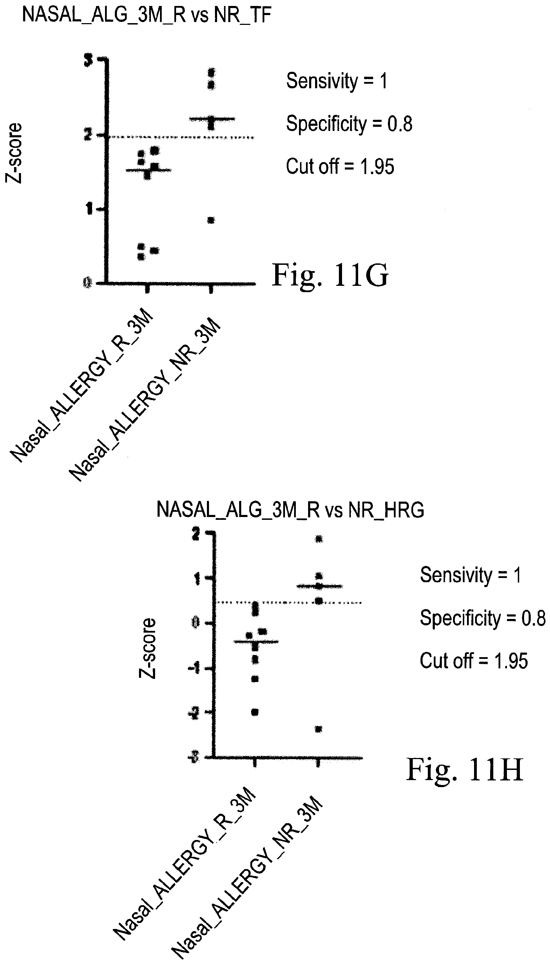

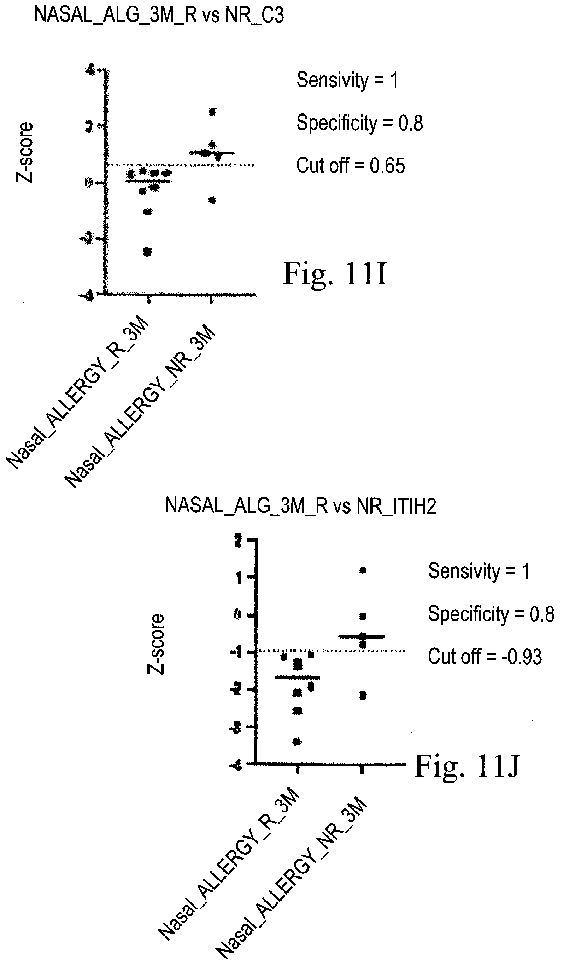

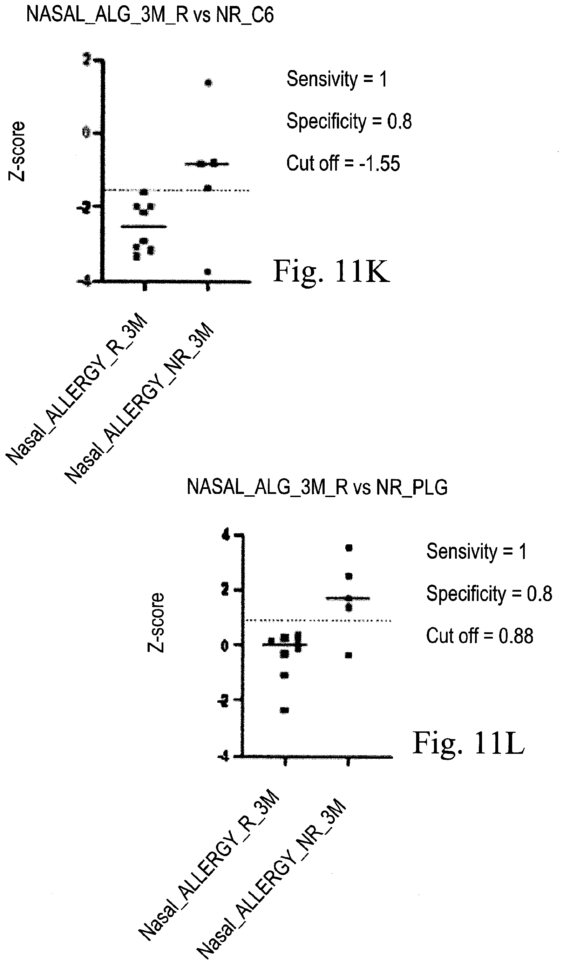

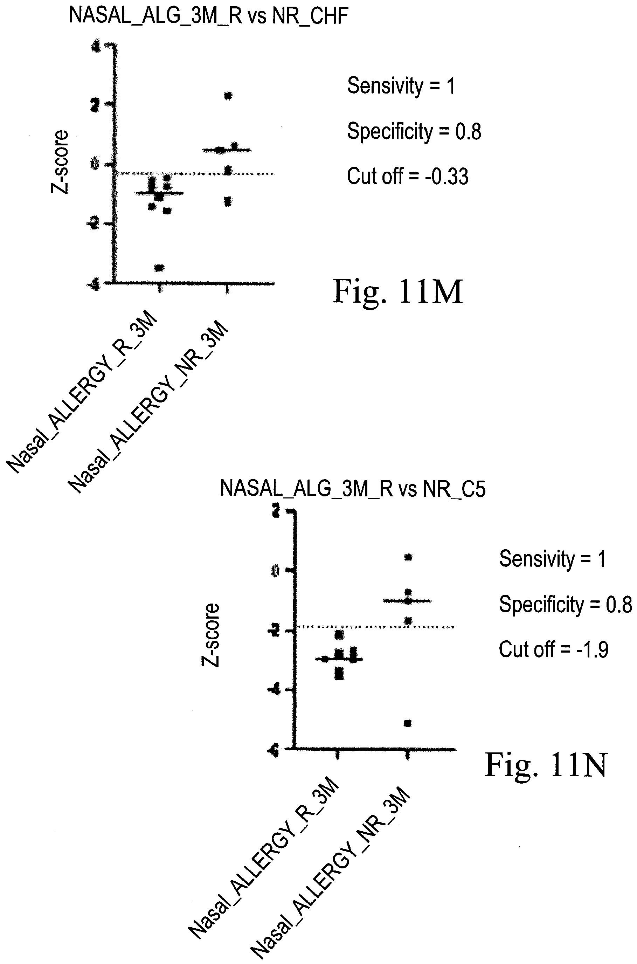

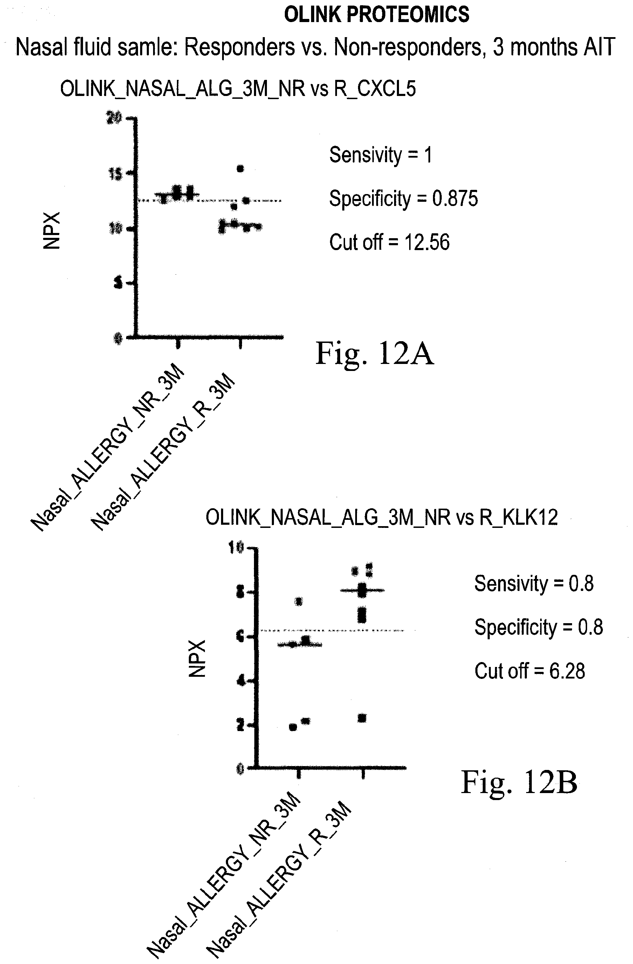

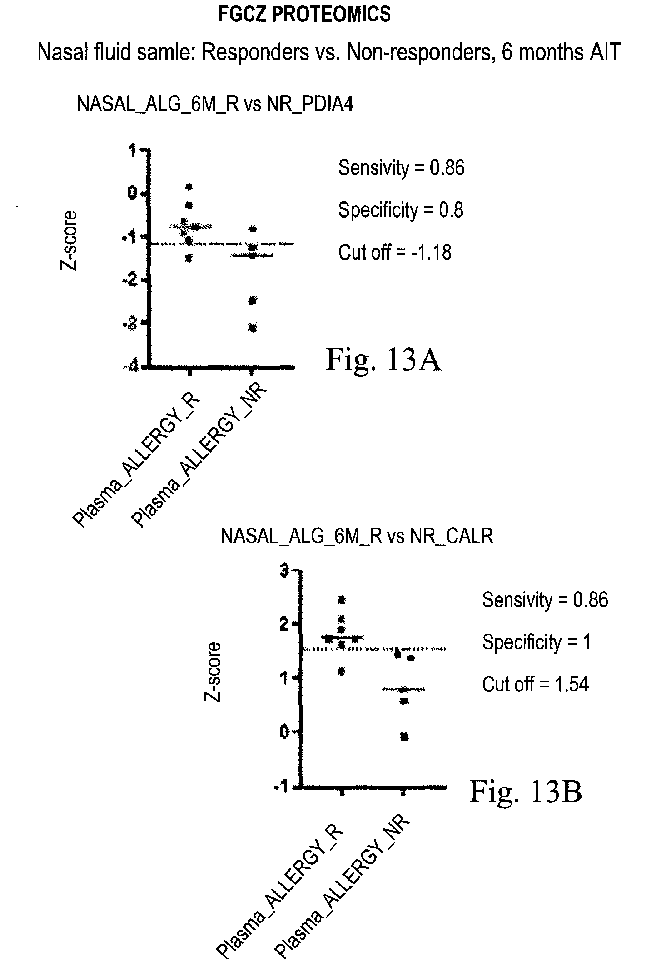

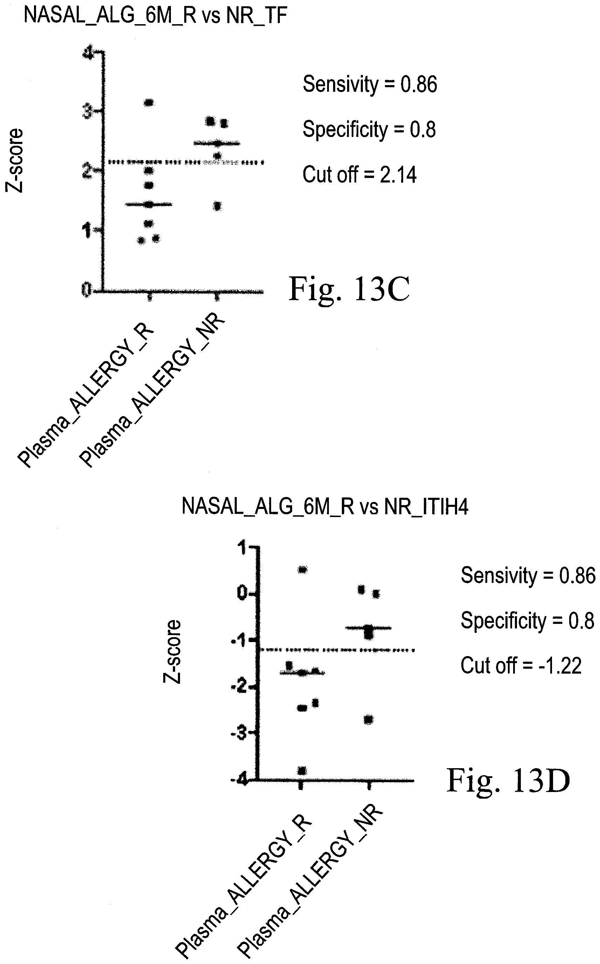

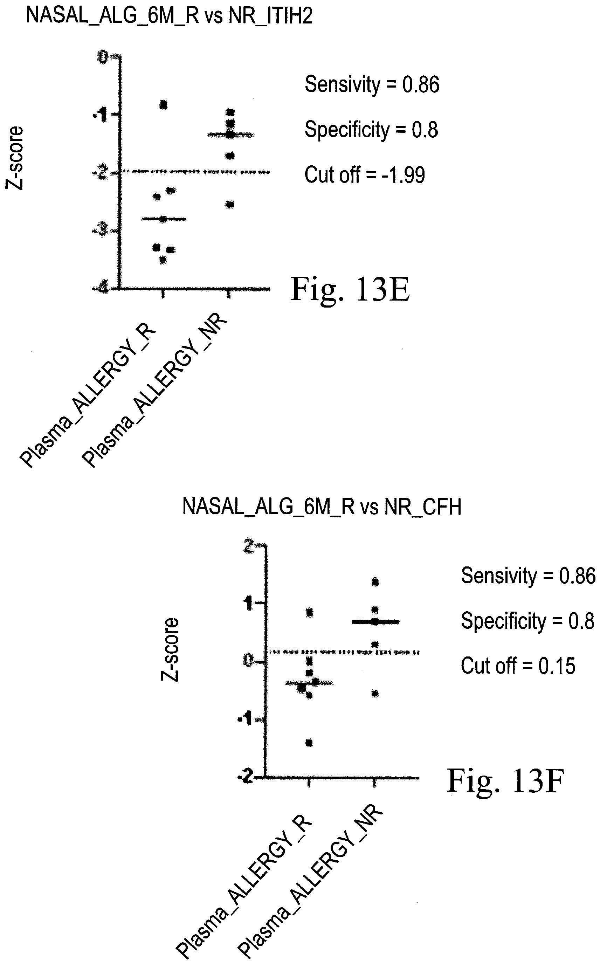

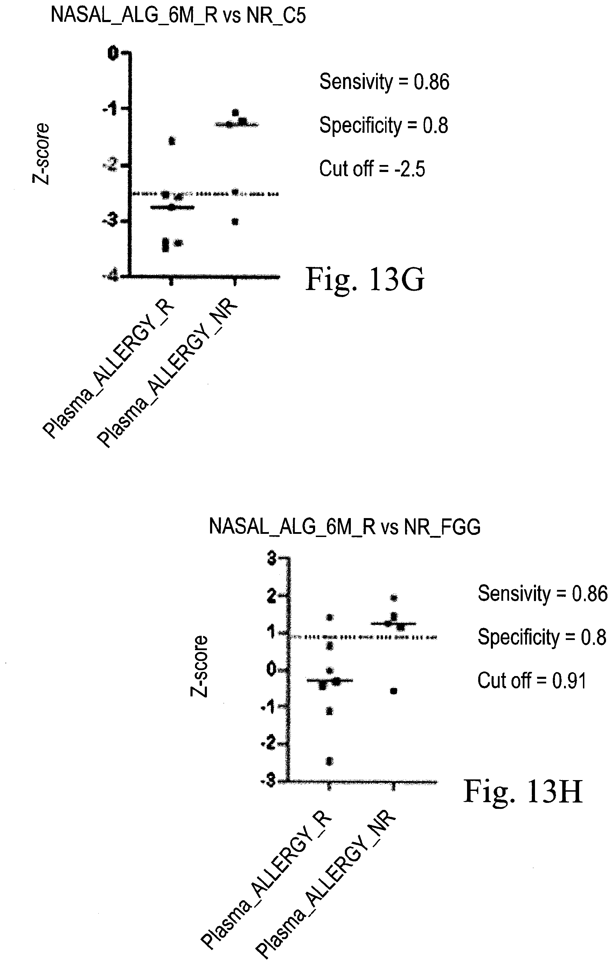

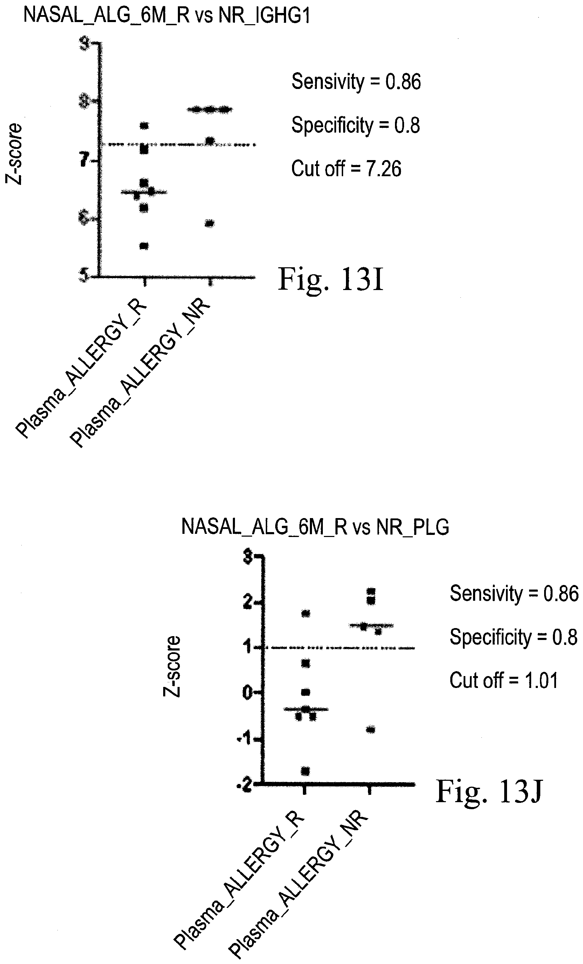

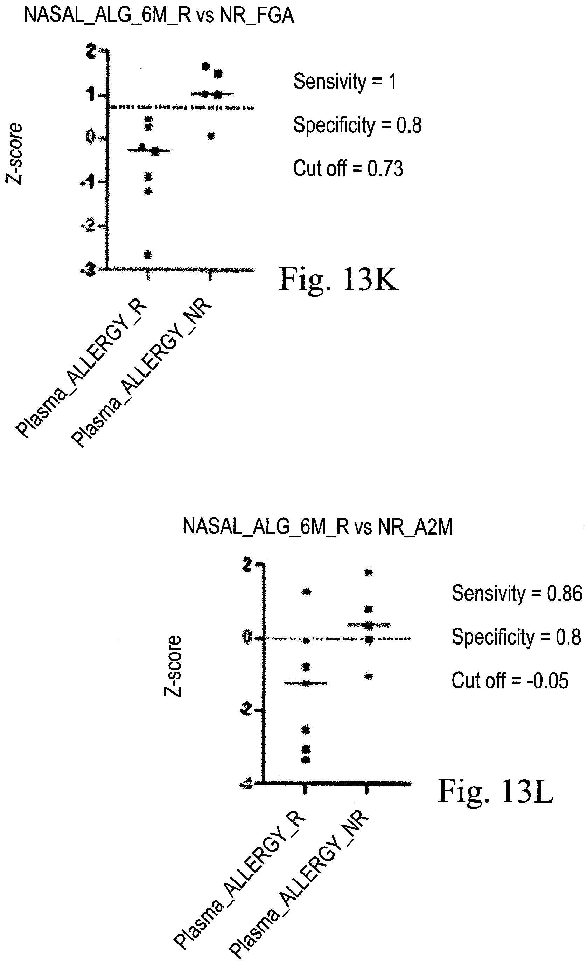

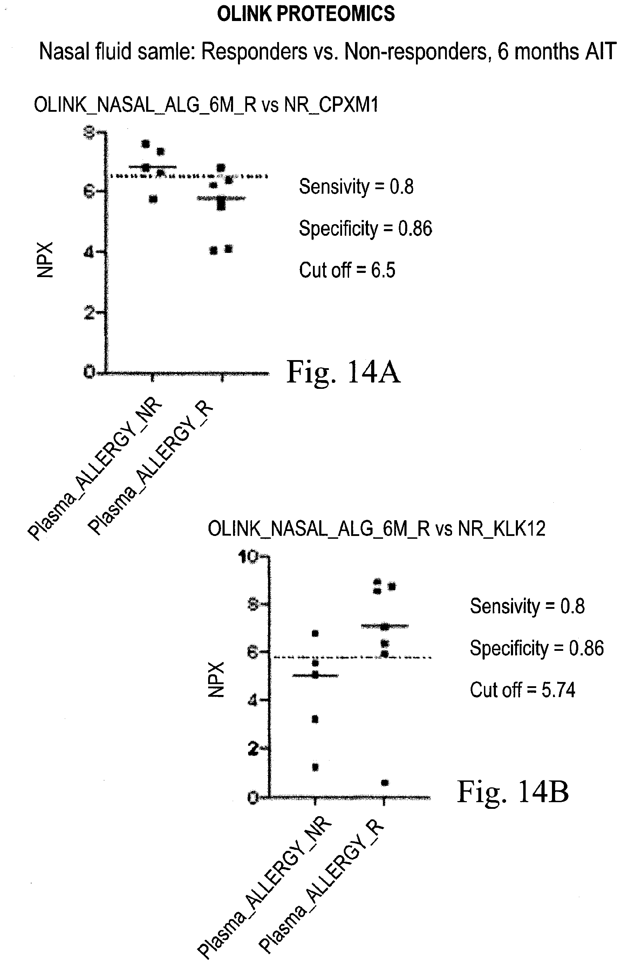

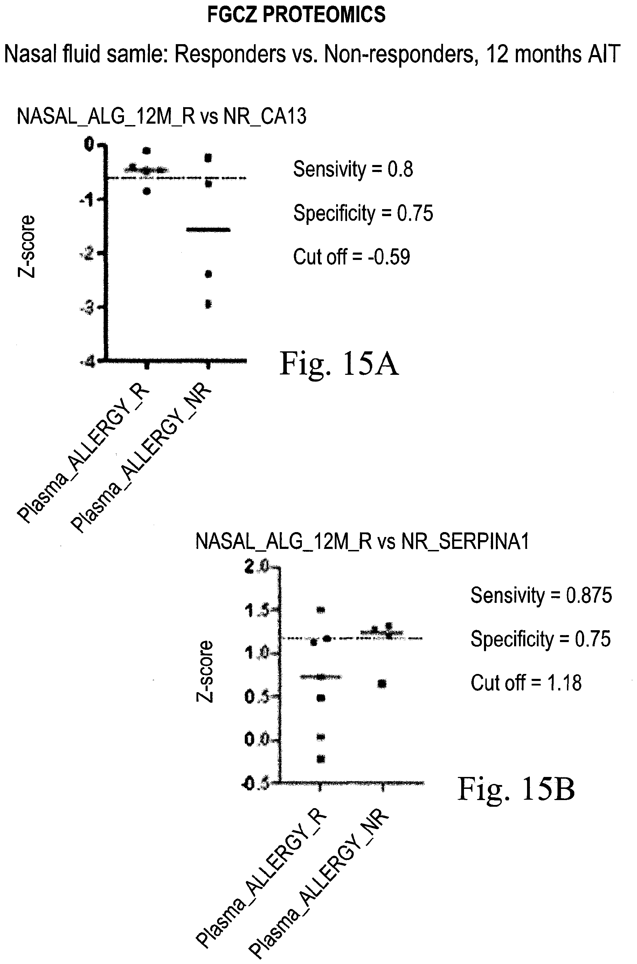

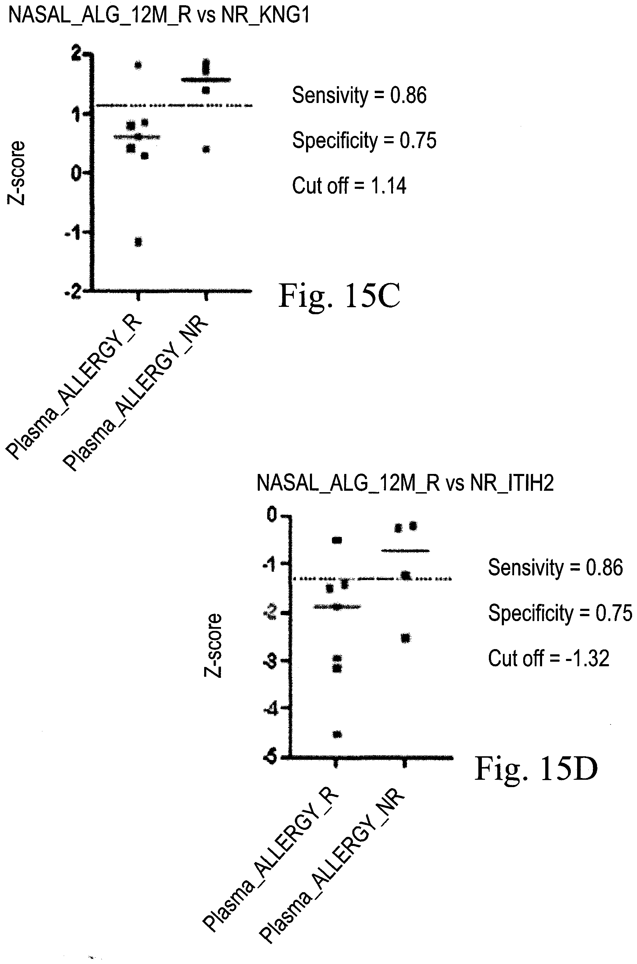

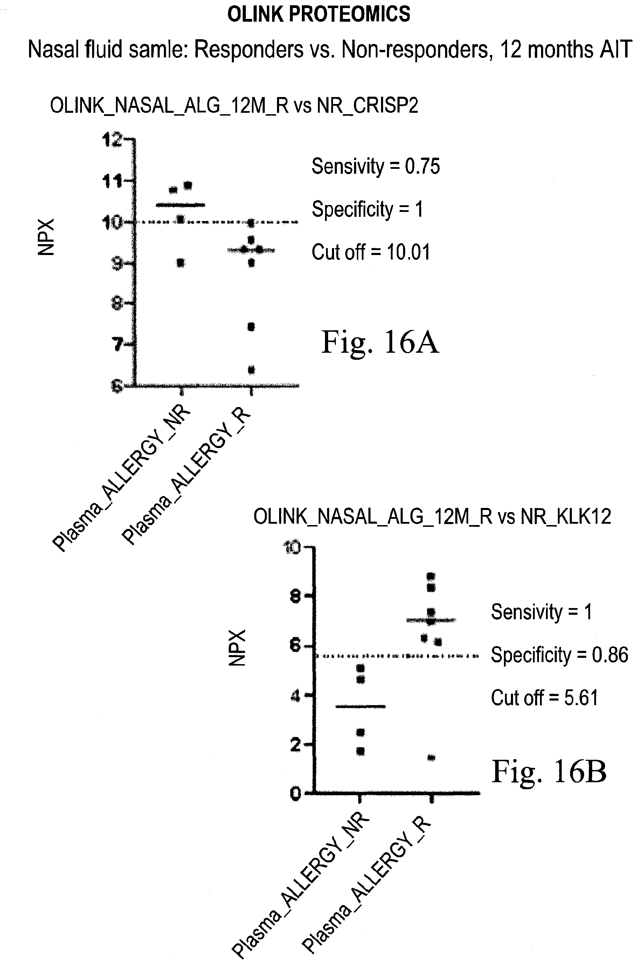

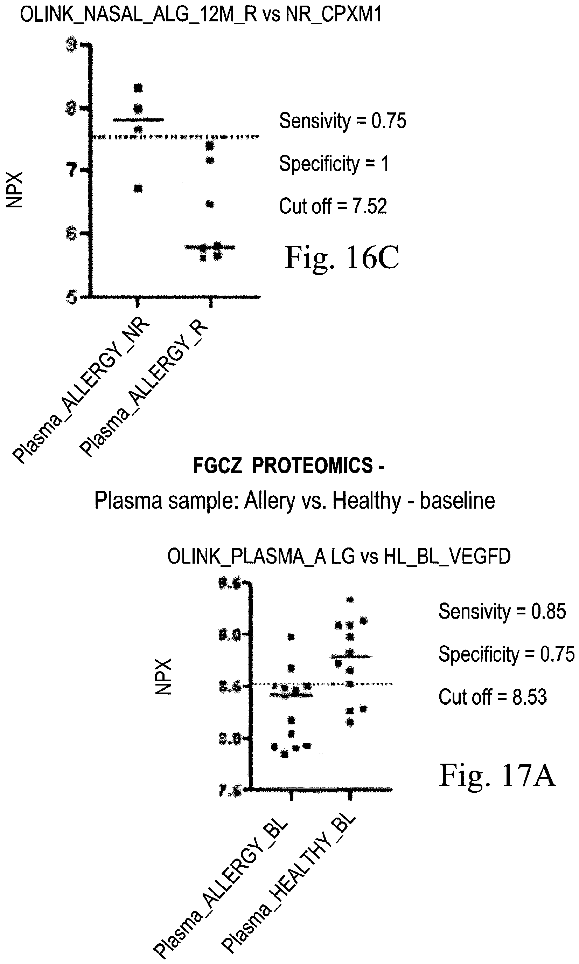

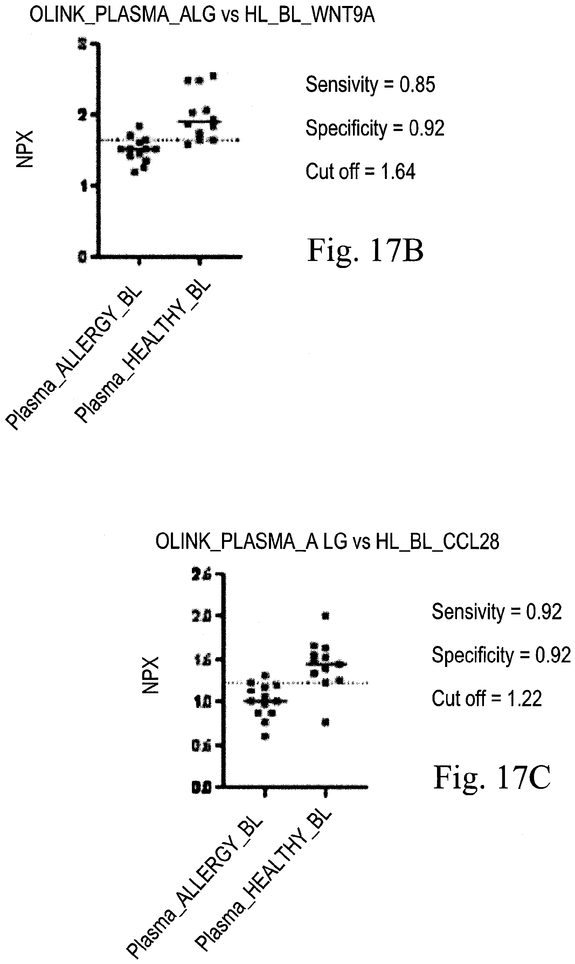

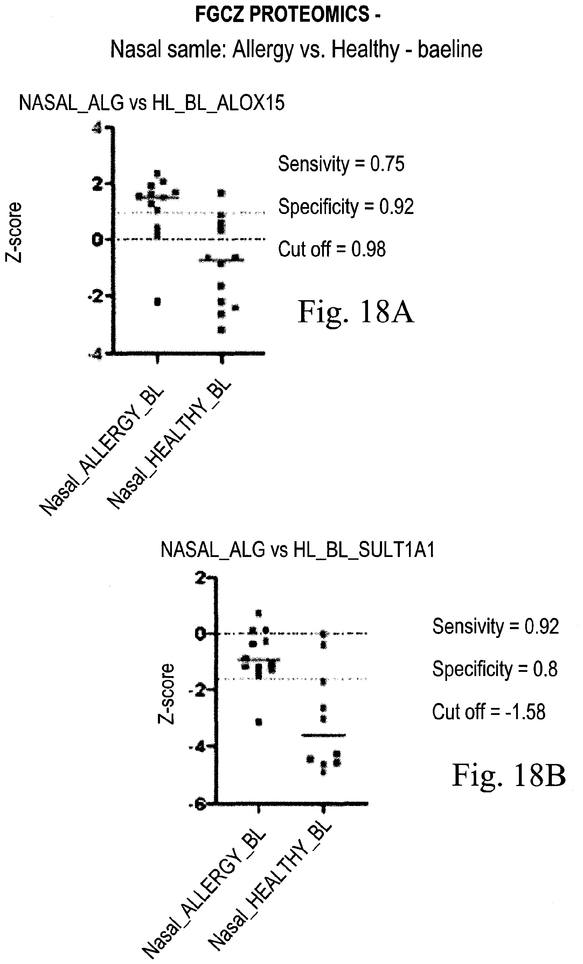

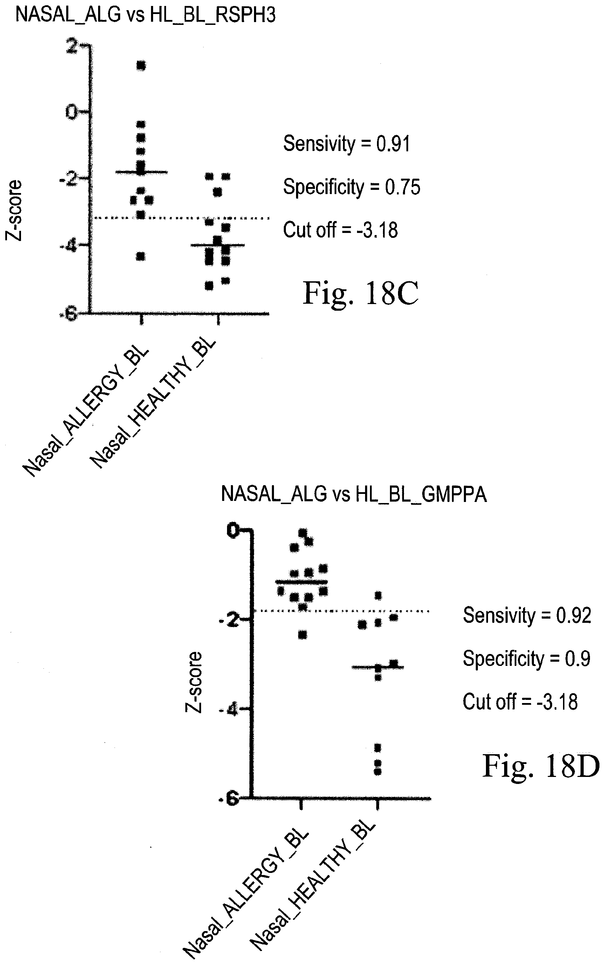

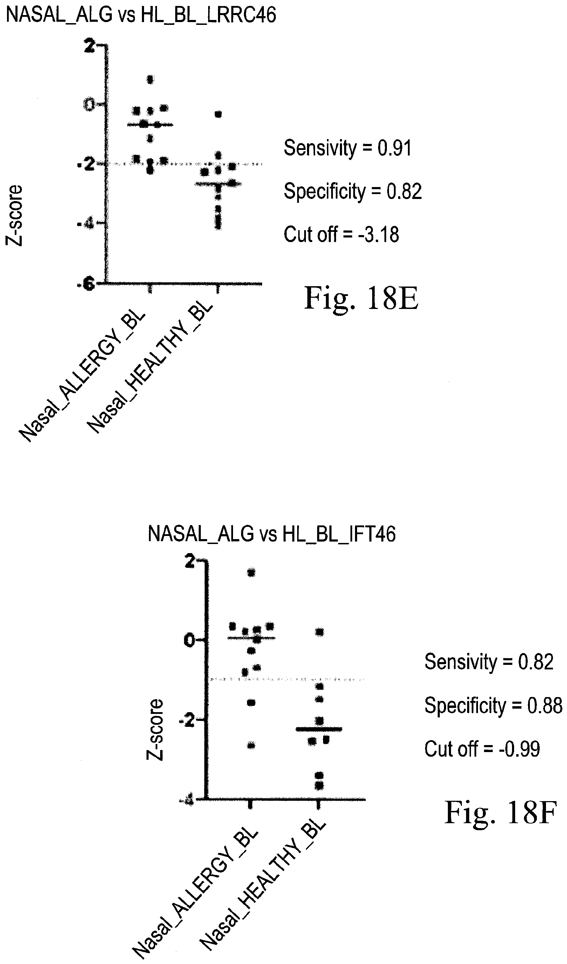

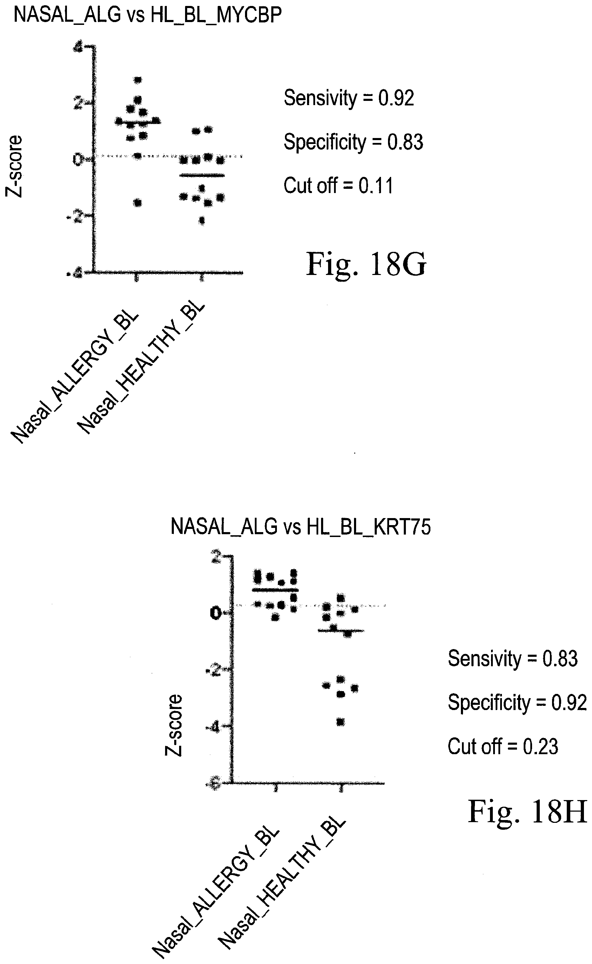

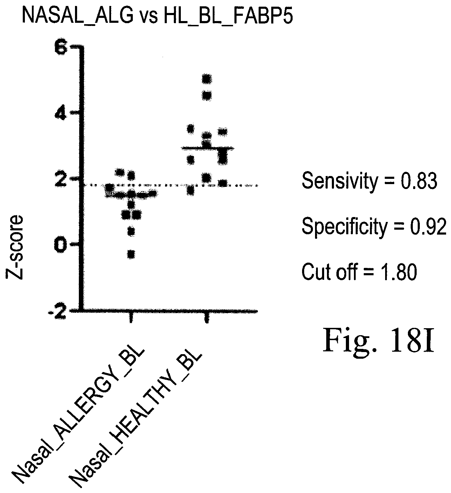

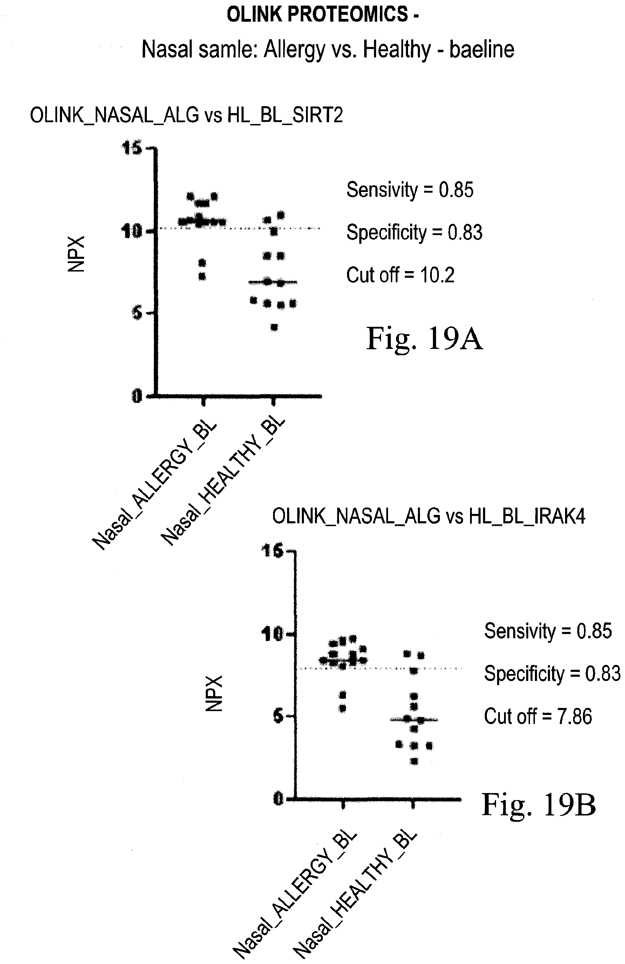

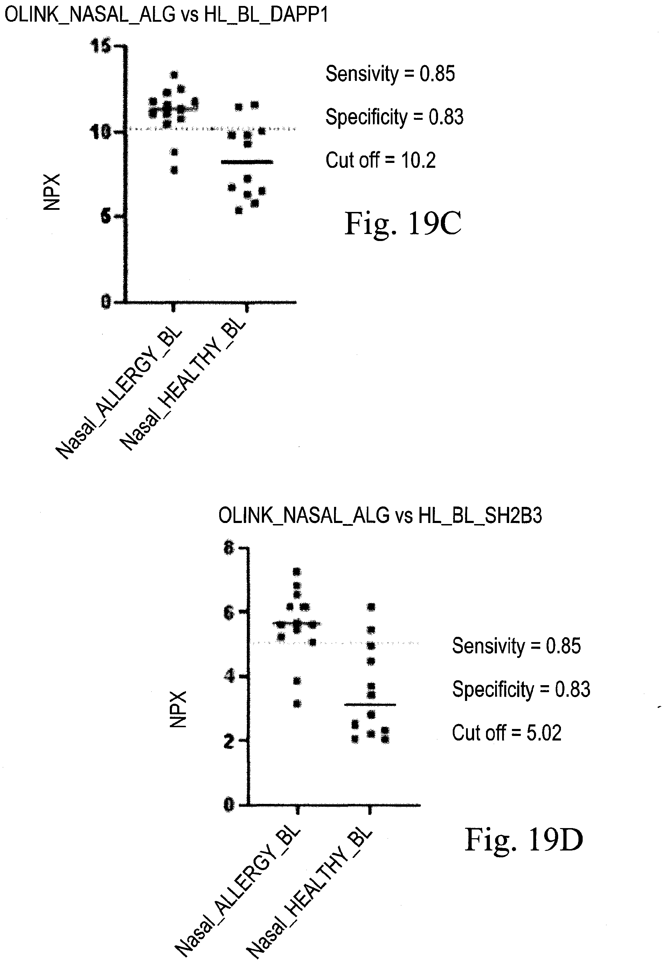

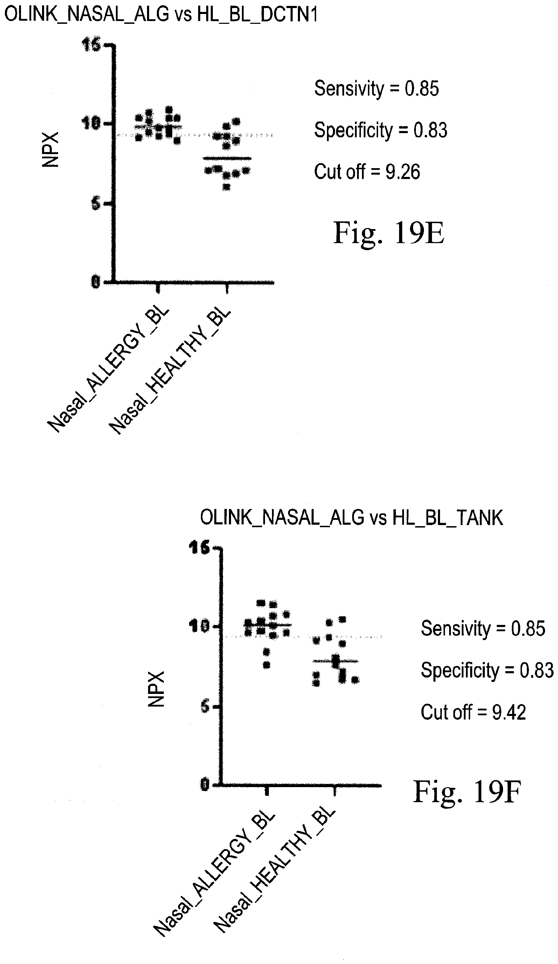

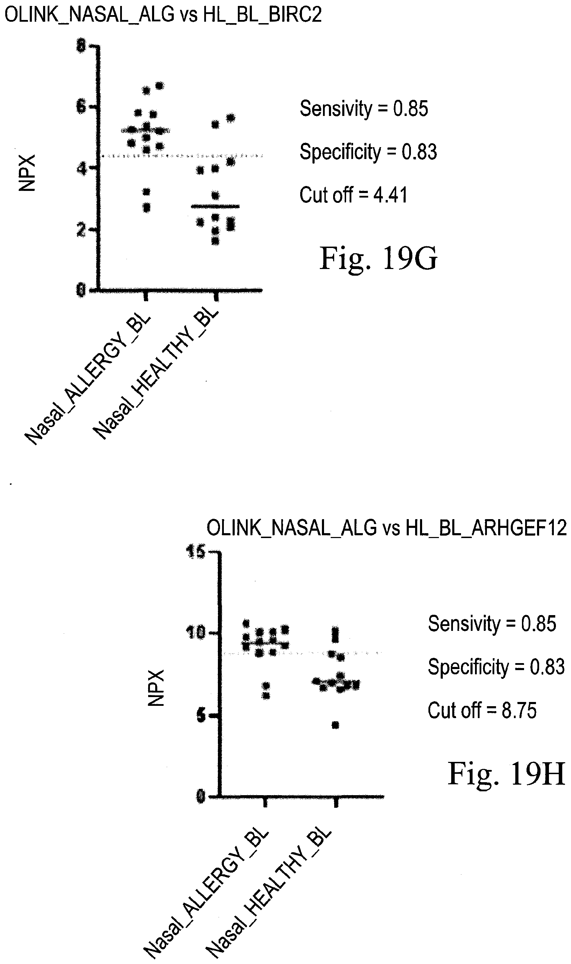

## Claims

1. A method of identifying a patient with grass and/or birch pollen allergy responsive to treatment with allergic immunotherapy (AIT) comprising determining in-vitro in a plasma sample from said allergic patient the level of expression of
the marker protein CCL8 alone or
in combination with one or more marker proteins selected from the group consisting of ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 and METAP1D.

2. A method of determining the clinical efficacy of a treatment with AIT using a method according to claim 1 .

3. Use of the marker protein CCL8 alone or in combination with one or more marker proteins selected from the group consisting of ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 and METAP1D according to claim 1 in the identification of a patient with grass and/or birch pollen allergy responsive to treatment with AIT comprising determining in-vitro in a plasma sample from said patient the level of expression of said marker protein.

4. Use of an assay device in the method according to claim 1 .

5. Use of a kit in the method according to claim 1, which comprises:
a) means for determining the level of expression of one or more marker proteins according to claim 1 ,
b) a standard control curve or control value showing a relationship between the level of expression of the marker proteins in plasma or nasal fluid samples and the responsiveness to AIT of allergic patients, and
c) a control sample or control value indicative of the level of expression of the marker proteins in a plasma or nasal fluid sample from a non-responder to AIT.

## Patentansprüche

1. Verfahren zur Identifizierung eines Patienten mit Gräser- und/oder Birkenpollenallergie, der auf eine Behandlung mit Allergen-Immuntherapie (AIT) anspricht, umfassend die in-vitro Bestimmung in einer Plasmaprobe des allergischen Patienten des Expressionslevels von
dem Markerprotein CCL8 allein oder in Kombination mit
einem oder mehreren Markerproteinen, ausgewählt aus der Gruppe bestehend aus ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 und METAP1D.

2. Verfahren zur Bestimmung der klinischen Wirksamkeit einer Behandlung mit AIT unter Verwendung eines Verfahrens gemäß Anspruch 1.

3. Verwendung des Markerproteins CCL8 allein oder in Kombination mit einem oder mehreren Markerproteinen, ausgewählt aus der Gruppe bestehend aus
ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 und METAP1D gemäß Anspruch 1 bei der Identifizierung eines Patienten mit Gras- und/oder Birkenpollenallergie, der auf die Behandlung mit AIT anspricht, umfassend die in-vitro-Bestimmung des Expressionslevels des Markerproteins in einer Plasmaprobe des Patienten.

4. Verwendung einer Assay-Vorrichtung in dem Verfahren gemäß Anspruch 1.

5. Verwendung eines Kits in dem Verfahren gemäß Anspruch 1, das umfasst:
a) Mittel zur Bestimmung des Expressionslevels eines oder mehrerer Markerproteine gemäß Anspruch 1 ,
b) eine Standard-Kontrollkurve oder einen Kontrollwert, die/der eine Beziehung, zwischen dem Expressionslevel des Markerproteins in Plasma oder Nasensekret-Proben und das Ansprechen auf AIT von allergischen Patienten zeigt, und
c) eine Kontrollprobe oder ein Kontrollwert, bezeichnend des Expressionslevel der Markerproteine in einer Plasma oder Nasensekret-Probe von einem AIT-Non-Responder.

## Revendications

1. Procédé d'identification d'un patient présentant une allergie au pollen de graminées et/ou de bouleau répondant à un traitement par immunothérapie allergénique (ITA) comprenant la détermination in vitro dans un échantillon de plasma dudit patient allergique du niveau d'expression de
la protéine marqueur CCL8 seule ou
en combinaison avec une ou plusieurs protéines marqueurs sélectionnées dans le groupe constitué des ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 et METAP1D.

2. Procédé de détermination de l'efficacité clinique d'un traitement par ITA en utilisant un procédé selon la revendication 1.

3. Utilisation de la protéine marqueur CCL8 seule ou en combinaison avec une ou plusieurs protéines marqueurs sélectionnées dans le groupe constitué des ITGB3, MYL6, FLNA, ACTB, KLKB1, APOA1, ITIH1, C2, SERPINA6, SHBG, GFRA2, GKN1, CCL11, BTN3A2, SIGLEC7, IL18, CHRDL2, REG4, GHRL, CXCL11, MEP1B, GSAP, CXCL5 et METAP1D selon la revendication 1 dans l'identification d'un patient présentant une allergie au pollen de graminées et/ou de boulot répondant à un traitement par ITA comprenant la détermination in vitro dans un échantillon de plasma dudit patient du niveau d'expression de ladite protéine marqueur.

4. Utilisation d'un dispositif de dosage dans le procédé selon la revendication 1.

5. Utilisation d'un kit dans le procédé selon la revendication 1 qui comprend :
a) un moyen de détermination du niveau d'expression d'une ou plusieurs protéines marqueurs selon la revendication 1,
b) une courbe d'étalonnage témoin ou une valeur témoin présentant une relation entre le niveau d'expression des protéines marqueurs dans les échantillons de plasma ou de fluide nasal et la réponse à une ITA de patients allergiques, et
c) un échantillon témoin ou une valeur témoin indiquant le niveau d'expression des protéines marqueurs dans un échantillon de plasma ou de fluide nasal d'un patient ne répondant pas à une ITA.
